(19) Europäisches Patentamt
European Patent Office
Office européen des brevets

(11) EP 2 391 658 B1

(12) **EUROPEAN PATENT SPECIFICATION**

(45) Date of publication and mention of the grant of the patent:
**23.09.2015 Bulletin 2015/39**

(21) Application number: **10735433.4**

(22) Date of filing: **29.01.2010**

(51) Int Cl.:
***C08F 26/06*** *(2006.01)*
***C08F 226/06*** *(2006.01)*
***B01J 20/26*** *(2006.01)*
***B01J 20/30*** *(2006.01)*
***C08F 2/44*** *(2006.01)*
***A61K 31/74*** *(2006.01)*
***C07B 63/00*** *(2006.01)*

(86) International application number:
**PCT/AU2010/000088**

(87) International publication number:
**WO 2010/085851 (05.08.2010 Gazette 2010/31)**

(54) **MOLECULARLY IMPRINTED POLYMERS**
MOLEKULAR GEPRÄGTE POLYMERE
POLYMÈRES À EMPREINTES MOLÉCULAIRES

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **29.01.2009 AU 2009900328**

(43) Date of publication of application:
**07.12.2011 Bulletin 2011/49**

(73) Proprietors:
- **Commonwealth Scientific and Industrial Research Organisation**
  **Campbell, Australian Capital Territory 2612 (AU)**
- **Monash University**
  **Clayton VIC 3800 (AU)**

(72) Inventors:
- **HEARN, Milton, T., W.**
  **Balwyn, Victoria 3103 (AU)**
- **LANGFORD, Steven**
  **Narre Warren North, Victoria 3804 (AU)**
- **TUCK, Kellie Louise**
  **Murrumbeena, Victoria 3163 (AU)**
- **HARRIS, Simon**
  **Bulleen, Victoria 3105 (AU)**
- **BOYSEN, Reinhard Ingemar**
  **Wheelers Hill, Victoria 3150 (AU)**
- **PERCHYONOK, Victoria Tamara**
  **Glenhuntly, Victoria 3163 (AU)**
- **DANYLEC, Basil**
  **Box Hill North, Victoria 3129 (AU)**
- **SCHWARZ, Lachlan**
  **Rowville, Victoria 3178 (AU)**
- **CHOWDHURY, Jamil**
  **Skye, Victoria 3977 (AU)**

(74) Representative: **Campbell, Neil Boyd**
**Dehns**
**St Bride's House**
**10 Salisbury Square**
**London**
**EC4Y 8JD (GB)**

(56) References cited:
**WO-A1-2005/112670**
**WO-A1-2006/001721**
**US-A1- 2002 015 690**
**US-A1- 2002 198 349**
**US-A1- 2006 292 545**

- **H. CAO ET AL: "Evaluation of New Selective Molecularly Imprinted Polymers for the Extraction of Resveratrol from Polygonum Cuspidatum",MACROMOLECULAR RESEARCH, vol. 14, no. 3, 20 March 2006 (2006-03-20) , pages 324-330, XP002687338,**
- **QUAGLIA, M. ET AL.: 'Target Analogue Imprinted Polymers with Affinity for Folic Acid and Related Compounds' J AM CHEM SOC vol. 123, 2001, pages 2146 - 2154**
- **XIE, J. ET AL.: 'Affinitive Separation and On-Line Identification of Antitumor Components from Peganum nigellastrum by Coupling a Chromatographic Column of Target Analogue Imprinted Polymer with Mass Spectrometry' ANAL CHEM vol. 74, 2002, pages 2352 - 2360**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PARMPI, P. ET AL.: 'Biomimetic glucose recognition using molecularly imprinted polymer hydrogels' BIOMATERIALS vol. 25, 2004, pages 1969 - 1973, XP004485112

## Description

### FIELD OF THE INVENTION

The present invention is directed to molecularly imprinted polymers (MIPs) which have applications in extracting bioactive compounds from a range of bioprocessing feedstocks and wastes.

### BACKGROUND OF THE INVENTION

Molecularly imprinted polymers (MIPs) can be designed with variable levels of cross-linking to produce polymers with controlled rigidity or flexibility, dependent upon the functional requirement, which contain cavities (binding sites) that can be tailor made to be specific for any target analyte. Using a pure sample of templates, MIPs can be prepared that are specific for the template compound or selective for other molecules having a similar chemical structure. MIPs are a polymeric network having high selectivity and specificity, which can be likened to that of an antibody for its antigen. MIPs offer the benefits of enhanced resistance to temperature, extremes of pH, solvents, proteases, and degradation or denaturation processes, which makes them reusable materials for the pre-concentration, extraction and separation of potential value-add biomolecules from complex feedstocks.

Strong epidemiological evidence exists for the health benefits of fruit and vegetables on cardiovascular disease, gastrointestinal tract cancers, cataracts and other diseases and ailments. Phytochemicals are candidates for contributing to a major part of this effect. Although knowledge of the mechanisms of uptake, metabolism and how these molecules exert their biological effects *in vivo* is uncertain, the health benefits afforded by these bioactive compounds is widely recognised and accepted.

One class of phytochemicals which are of considerable interest are the polyphenols, of which resveratrol is an example. Other interesting classes are the chalcones, indoles, coumarins, flavanones and flavanols, anthocyanins and the phytosterols and phytostanols.

With regard to resveratrol and its analogues or phytosterols and their analogues, there has been little published literature examining ways to produce MIPs of the required specificity. The literature that does exist predominately emphasises only the analytical mode of separation. Where attempts have been made to use the produced MIP in a preparative separation context, no attempt has been made to optimise the MIP performance or address some of the key challenges for larger scale use. For example, the work of Xiang *et al.*[1] is based on the concept that non-covalent molecular imprinting techniques are unsuitable for templates, such as resveratrol, dissolved in polar solvents, and hence it was necessary for acrylamide to be used as the monomer, whilst in the work of Ma *et al.*[2] no attention was given to control the selectivity of the derived MIP, with the consequence that the resveratrol derivatives, trans-polydatin and emodin, bound more strongly to the MIP despite their glucosidated or modified structures. The work of Cao *et al.*[3] to a large extent simply restates the same conclusions of Xiang *et al.*[1] or Ma *et al.*[2], a situation which is not surprising in view of the use of very similar methods and an identical set of test compounds. There is thus a need for specific MIPs to be developed, which have been tailored and optimised for a particular template or class of templates with the derived molecularly imprinted polymers suitable for industrial-type applications. Specifically, there is a need to make suitable MIPs that are compatible with systems currently employed by the manufacturing industries, permitting the isolation of target compounds of commercial importance within the food and similar industries.

### SUMMARY OF THE INVENTION

The present inventors have designed molecularly imprinted polymers with resveratrol analogue templates, specifically an imine analogue of resveratrol (called "green resveratrol" by the inventors), and an amide analogue. They have demonstrated the application of a resveratrol selective MIP in a solid phase extraction technique, allowing the selective enrichment of resveratrol from grape seed and peanut meal extract, in the latter case resulting in at least a 20 fold increase in resveratrol concentration without further procedural intensification or optimisation. This outcome can be contrasted to the low purification factors obtained with the MIP systems of the prior art, such as those described by Ma *et al.*[2], Xiang *et al.*[1] or Cao *et al.*[3]. In addition, they have demonstrated the ability to scale up the molecularly imprinted solid phase technique, thereby illustrating the scalability of the process.

In a first aspect, the invention provides a MIP imprinted with (*E*)-5-[(4-hydroxy-phenylimino)-methyl]-benzene-1,3-diol or 3,5-dihydroxy-*N*-(4-hydroxyphenyl)benzamide.

### BRIEF DESCRIPTION OF THE FIGURES

**Figure 1.** Structure of reseveratrol (1A) and computer generated model of resveratrol surface using PM3 calculations displaying electronic character and likely interaction sites (1B).

**Figure 2.** Molecular modelling titration results showing estimated interaction energy between resveratrol and 4VP clusters using PM3 geometry optimisation calculations showing a distinct minimum at three eq 4VP.

**Figure 3.** Space filled image of a resveratrol complex with three 4VP units interacting through each of the stilbene hydroxyl and pyridinyl nitrogen head groups. Calculations did not include solvent considerations and should only be used as a guide.

**Figure 4.** Modelling titration data for the estimated net interaction energies ($\Delta E_f$) for the complexes between resveratrol and the functional monomers acrylamide (AAM) (Figure 4A) and 4-vinylbenzoic acid (4VBA) (Figure 4B) using semi-empirical calculations using a PM3 force field. In addition to this the static binding data for 50 mg of polymer in a solution of 0.5 mM resveratrol in acetonitrile is shown (Figures 4C and 4D).

**Figure 5.** Downfield movement of the aromatic -OH shift of resveratrol upon addition of increasing molar equivalents of the functional monomer 4VP. Indicates that a pre-polymerisation complex is forming which is likely through H bonding interactions with the pyridinyl N group.

**Figure 6.** Rebinding data for the polymers MIP 1, MIP 2, MIP 3 and MIP 4 and corresponding NIPs displaying resveratrol affinity from a resveratrol solution (0.05mM) in acetonitrile.

**Figure 7.** Resveratrol binding affinity to polymers MIP 1, MIP 2, MIP 3, MIP 4 NIP 1 and NIP 2 in a binding assay using 0.5 mM resveratrol solution in acetonitrile.

**Figure 8.** Static rebinding data for MIP and their respective NIP control materials prepared with the following T:FM:XL ratios: 1:3:15 (MIP 8), 1:3:17 (MIP 10), 1:3:20 (MIP 9), 1:3:30 (MIP 2) and 1:3:40 (MIP 5).

**Figure 9.** Static Binding data for multiple MIP 8 batches A-J (LS3-37p84-17-4-08a-j), K (LS3-26p62-1-4-08a), L (LS3-26p62-1-4-08b) and M (ls2-6p10-7-2-07) indicating the reproducibility in MIP production and binding performance in acetonitrile. All measurements were conducted in duplicate.

**Figure 10.** Resveratrol binding capacity under static conditions to MIP 8 (30 mg) over a range of resveratrol concentrations in ACN up to 5 mM. Samples were mixed overnight (18 hours) using a suspension mixer at approximately 40 rpm. Samples were then centrifuged, and an aliquot of the supernatant analysed by HPLC to determine the free resveratrol concentration.

**Figure 11.** Cross-reactivity binding data for MIP 8 under static conditions for a range of resveratrol analogues (0.5 mM) in acetonitrile without competition.

**Figure 12.** Static binding data comparing resveratrol affinity between a resveratrol imprinted polymer (MIP 8) and MIPs templated with resveratrol analogues.

**Figure 13.** Results from MISPE binding assay comparing both the effect of cross-linking amount and influence of ethanol in the porogen ($MIP_E$) under dynamic SPE conditions after removal of non-specific binding via extensive washing with acetonitrile.

**Figure 14.** MISPE binding of resveratrol (0.5 mM) under aqueous conditions with resveratrol bound (μmole/g polymer) after washing with: A) loading solution; B) 2 × 20% (v/v) ethanol in water.

**Figure 15.** The effect of ethanol and water content on the retention/binding of resveratrol under aqueous conditions. Binding was assessed using resveratrol solutions (0.5 mM) in 20, 50 and 80% ethanol (v/v), respectively, to MIP 8. The data shown refers to percentage of the initial resveratrol solution bound after A) loading and B) clean up washes comprising 1 mL loading solution *(i.e* 20, 50 and 80% ethanol) and three consecutive washes of 50% aqueous ethanol (v/v).

**Figure 16.** Demonstrates that MIP 8 is capable of concentrating resveratrol from a complex feedstock containing multiple potential competitors for available binding sites. Diagrams A) shows the HPLC trace of spiked grape seed

extract with large amounts of unknown species masking resveratrol; B) shows the resveratrol (RT 2.735 min) eluted after applying a resveratrol standard solution (0.5 mM); C) shows resveratrol (RT 2.735 min) eluted from MISPE column after application of spiked grape seed extract and D) shows the results after application of spiked grape seed to the respective NISPE column resulting in no observable peak at 2.735 min, indicating that no resveratrol was retained.

**Figure 17.** Chromatograms of (A) untreated peanut meal, (B) MISPE treated peanut meal and (C) peanut meal extract after SPE treatment using a non-imprinted control polymer (NIP) as stationary phase.

**Figure 18.** Chromatograms of A) untreated peanut meal with resveratrol concentration of approximately 0.98 μg/mL of peanut meal extract, B) MISPE treated peanut meal resulting in resveratrol concentration of approximately 19.5 μg/mL and C) peanut meal extract after SPE treatment using a non-imprinted control polymer (NIP) as stationary phase.

**Figure 19.** Single analyte selectophore binding of alkene 1, amide 2, and imine 3, with $MIP_{RES}$.

**Figure 20.** Recognition of (*E*)-Resveratrol by $MIP_{RES}$, $MIP_{AMIDE}$ and $MIP_{IMINE}$.

**Figure 21. (A)** Static binding isotherms for the binding of (*E*)-resveratrol to $MIP_{AMIDE}$ (A) and $NIP_{AMIDE}$ (▲); **(B)** comparison of the selective affinity of both $MIP_{AMIDE}$ (A) and $MIP_{RES}$ (♦) for (*E*)-resveratrol, expressed as the selectivity (MIP - NIP).

**Figure 22.** Cross-reactivity studies on $MIP_{RES}$, $MIP_{AMIDE}$ and their respective NIP control polymers for (*E*)-resveratrol **1,** 3,5-dihydroxy-*N*-(4-hydroxyphenyl)benzamide **2,** catechin **3** and (*E*)-piceid **4.**

**Figure 23.** RP-HPLC chromatograms using $EtOH/H_2O$ (8:2 v/v) as mobile phase solvent B with UV-Vis detection at 321nm for **(A)** peanut meal extract and eluates from **(B)** $MIP_{RES}$, **(C)** $MIP_{AMIDE}$ and **(D)** $NIP_{RES}$ control columns. (*E*)-resveratrol elutes at $R_t$ = 12.2 min. The identity of (*E*)-resveratrol was confirmed by LC-ESI-MS.

**Figure 24.** Schematic showing the concept for sequential MISPE treatment of extracts obtained from complex food sources, whereby multiple bioactive components may be separated from a single source.

**Figure 25.** RP-HPLC chromatograms of **(A)** the peanut meal extract (10 g/500 mL) in $EtOH/H_2O$ (1:1 v/v) and acid elution of molecules captured by **(B)** $MIP_{RES}$ then **(C)** $MIP_{AMIDE}$. Chromatograms were obtained by UV-Vis detection at $\lambda$ = 321 nm. *(E)*-resveratrol elutes at $R_t$ = 17 minutes.

**Figure 26.** RP-HPLC chromatograms of **(A)** the resveratrol-depleted peanut meal extract resulting from the first round of MISPE processing and acid elution of molecules captured by **(B)** $MIP_{RES}$ then **(C)** $MIP_{AMIDE}$. Chromatograms were obtained by UV-Vis detection at $\lambda$ = 321 nm. A-type procyanidin elutes at $R_t$ = 15 minutes.

**Figure 27.** Schematic depiction of a sequential SPE format MIP column configuration for the isolation of multiple target compounds from a feed extract. All undesirable species pass through each MIP column leaving multiple target species bound to their respective MISPE columns, which can then be eluted to yield each of the respective target compounds in high purity.

## DETAILED DESCRIPTION OF THE INVENTION

The formation of a MIP typically involves the following steps: a template is mixed together with a monomer (typically in excess) to form pre-polymerisation complexes comprising a single template associated with a number of monomer molecules; these pre-polymerisation complexes are then cross-linked into a polymer by polymerisation of the monomers with a cross-linking agent; and the template is subsequently removed from the cross-linked complexes. The cross-linked monomers of the complexes comprise surfaces and cavities which are complementary to the shape of the template. The cross-linking reaction is generally carried out in the presence of porogen which ensures that the MIP has an open structure comprising a number of pores. These pores allow the molecules of a solution to move through the MIP so that the molecules are able to interact with the surfaces and cavities. An analyte which has the same or similar shape to the template will more strongly interact with the MIP than other molecules in the solution, so that the MIP can be used to capture, concentrate and/or separate the analyte.

Various different types of manufacture can be employed, e.g. (a) monolith type preparation, (b) particles made

by grinding and sizing, (c) precipitation polymerization procedures.

As would be clear to those skilled in the art, the techniques used are applicable to molecules other than resveratrol.

The monomers used in the MIPs of the present invention include those that promote or facilitate hydrogen-bonding interactions and/or π-π bonding interactions and include:

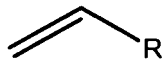

where R is selected from the group consisting of $C_{1-4}$alkyl, amide, nitrile, carboxylic acid, primary or secondary amine, $CO_2C_{1-4}$alkyl, $C_{1-4}OH$, hydroxyalkyl acrylate, benzene, benzyl amine, naphthalene, anthracene, pyridine, pyrimidine, purine, N-imidazole; and

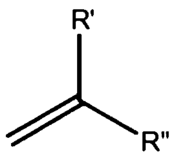

where R' is selected from the group consisting of H and $CH_3$;
and R" is selected from the group consisting of $C_{1-4}$alkyl, amide, nitrile, carboxylic acid, primary or secondary amine, $CO_2C_{1-4}$alkyl, $C_{1-4}OH$, and hydroxyalkyl methacrylate.

In a preferred form, the monomer is selected from the group consisting of:

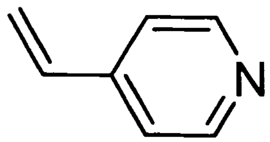

4-vinylpyridine

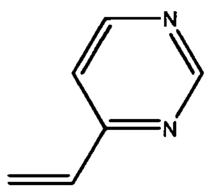

2-vinylpyrimidine

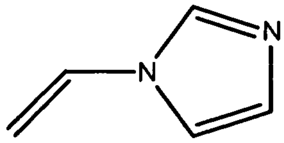

*N*-vinylimidazole

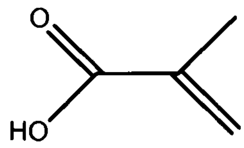

Methacrylic acid

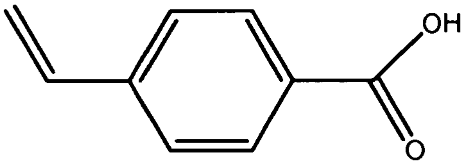

*p*-vinylbenzoic acid

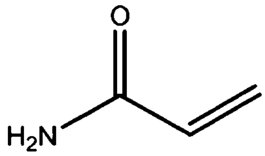

acrylamide

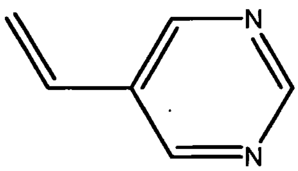

5-vinylpyrimidine

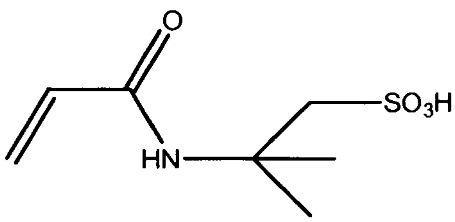

2-acryloylamino-2-methylpropane sulfonic acid

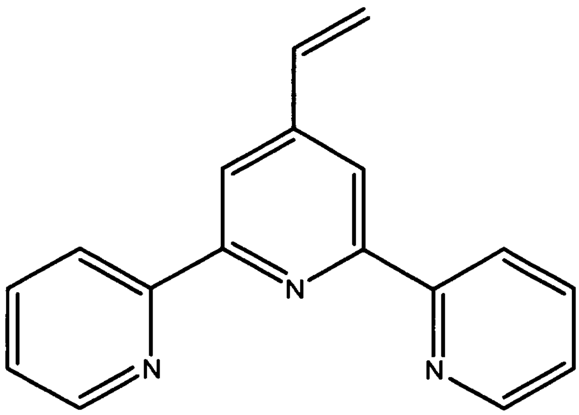

Vinylterpyridine

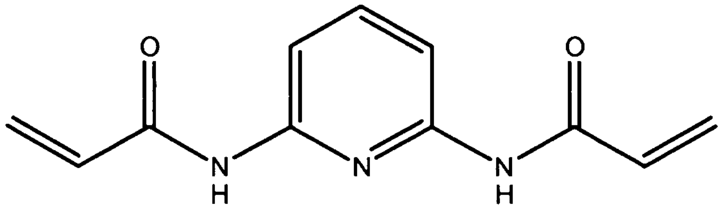

*N,N'*-(pyridine-2,6-diyl)-acrylamide

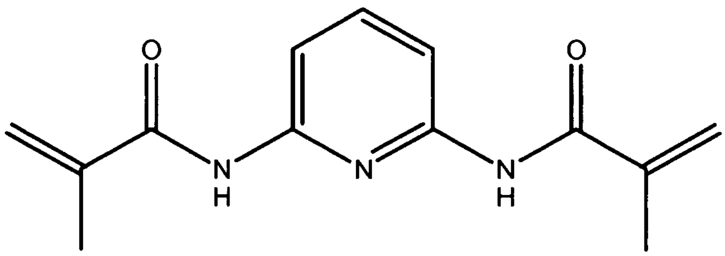

*N,N'*-(pyridine-2,6-diyl)-bis(2-methacrylamide)

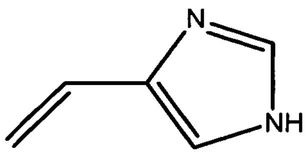

4-vinyl-1*H*-imidazole

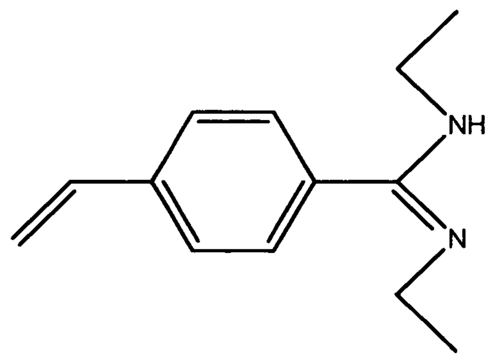

(*E*)-*N,N'*-diethyl-4-vinylbenzimidamide, and

1,2,3,4,5-pentachloro-6-(4-vinylphenoxy)benzene

The person skilled in the art would be aware of a wide range of cross-linking agents that would be suitable for use in the MIPs of the present invention. In a preferred embodiment, the cross-linking agent is selected from the group consisting of:

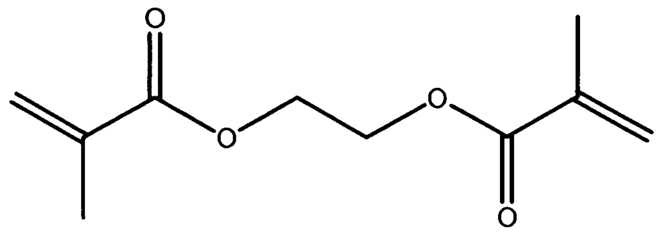

Ethyleneglycol dimethacrylate (EGDMA, EDMA)

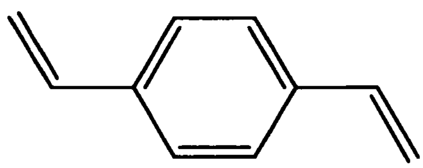

Divinylbenzene

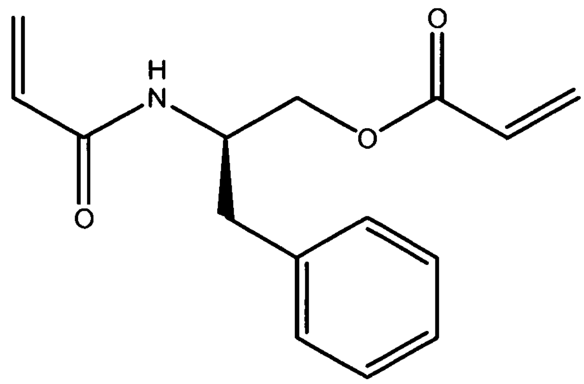

*N,O*-diacrylphenylalaninol

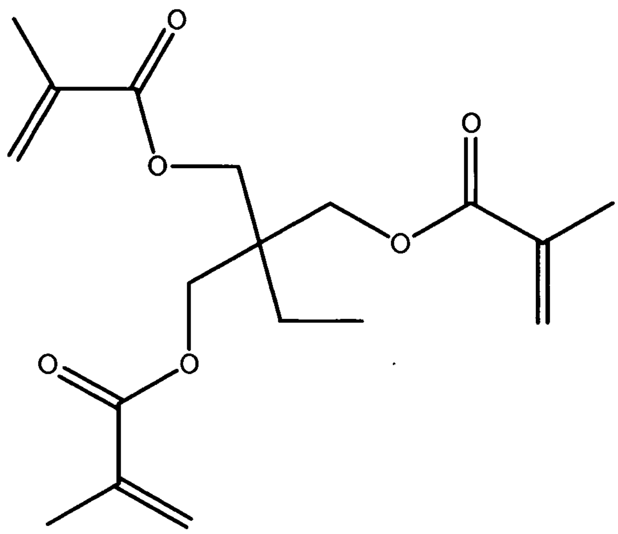

Trimethacrylolpropane trimethacrylate

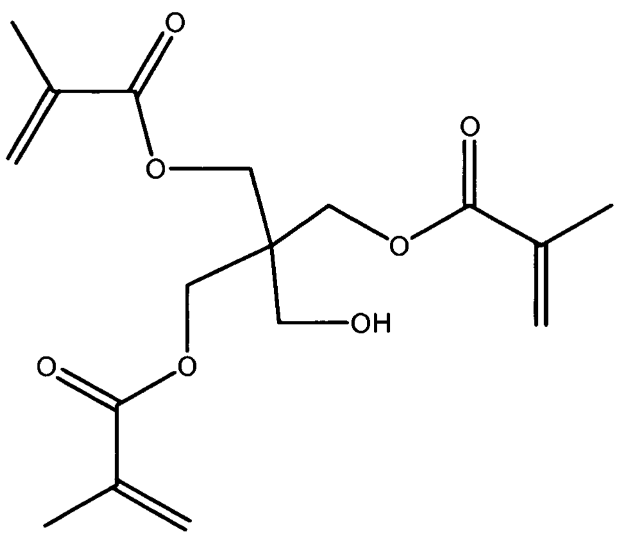

Pentaerythritol triacrylate

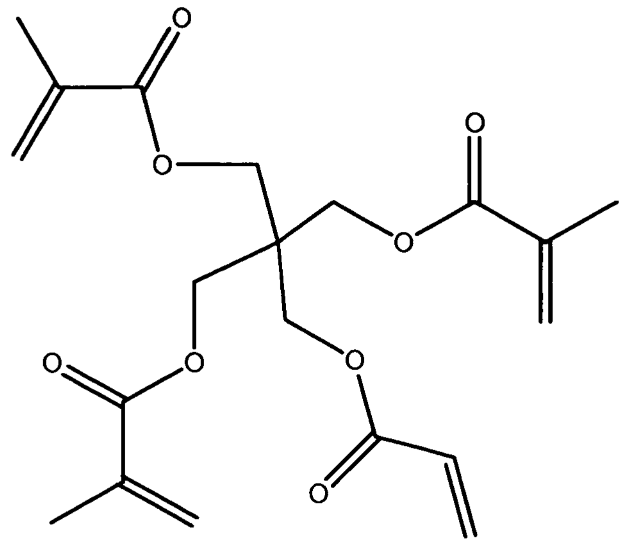

Pentaerythritol tetraacrylate

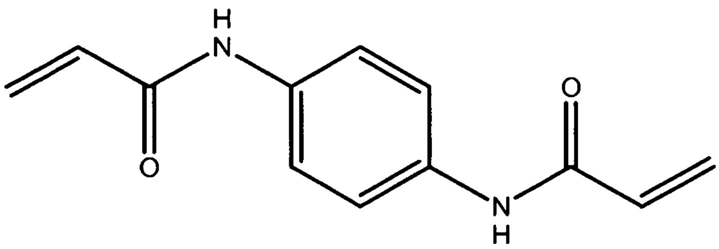

*N,N'*-diacryloyl-1,4-diaminobenzene

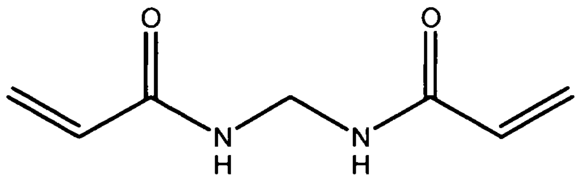

*N,N'*-methylenediacrylamide

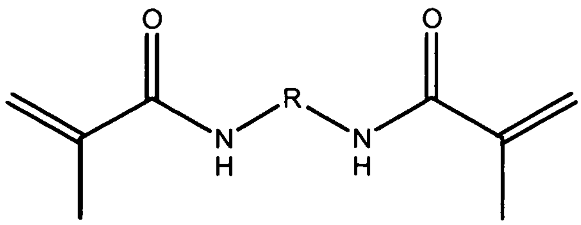

dimethacrylamides, where R is an alkyl chain, preferably 1 to 4 carbons in length

In certain embodiments, the monomer and the cross-linking agent may be the same compound. Porogens

suitable for use in the methods and MIPs of the present invention include those that promote or facilitate hydrogen-bonding interactions and those that promote or facilitate hydrophobic interactions or a combination of both.

Porogens that facilitate hydrogen bond formation include acetonitrile, acetone, ethyl acetate and dimethyl formamide (DMF) or a combination thereof, in addition to mixtures of the above with suitable quantities of ethanol, methanol or dimethyl sulfoxide (DMSO).

Porogens that facilitate hydrophobic interactions include aqueous mixtures of acetonitrile, acetone, ethyl acetate, DMF, ethanol, methanol, DMSO or a combination thereof.

The present invention provides a molecularly imprinted polymer (MIP) imprinted with an analogue of resveratrol where the *trans*-ethylene linker is replaced with an imine or amide, i.e. the imine (*E*)-5-[(4-hydroxy-phenylimino)-methyl] -benzene-1,3-diol or the amide 3,5-dihydroxy-*N*-(4-hydroxyphenyl)benzamide.

The imine and amide anlogues of resveratrol analogues are simpler and cleaner to synthesise than resvertrol itself. MIPs prepared using these templates have been shown to have a comparable binding affinity for resveratrol as MIPs prepared using resveratrol as a template.

In a preferred from, the MIP is for use in isolating resveratrol.

Preferably, when resveratrol is used as a template, the ratio of resveratrol to 4-vinylpyridine is 1:3. This ratio has been shown by modelling studies and by empirical results to be the optimum ratio for a resveratrol binding MIP. Similarly, the ratio of the imine (*E*)-5-[(4-hydroxy-phenylimino)-methyl]-benzene-1,3-diol and the amide 3,5-dihydroxy-N-(4-hydroxyphenyl)benzamide to 4-vinylpyridine is preferably 1:3.

Conditions have been established and optimized for the synthesis of a small library of polyfunctionalized (*E*)-stilbene (resveratrol) analogues using a convergent methodology. The synthetic procedures have introduced various functional groups into the core scaffolds, which has resulted in the production of a library of low molecular weight compounds having unique compositions of matter. A summary of the molecules produced to date is shown in Table 1.

**Table 1.** Structures of resveratrol analogues that have been synthesised for interaction with monomer during MIP formation.

| | | |
|---|---|---|
| 3-Point | 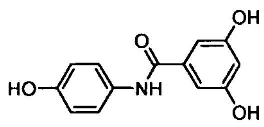 17 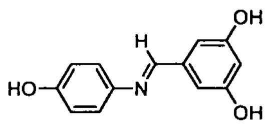 18 | **17.3,5-dihydroxy-*N*-(4-hydroxyphenyl)benzamide** **18. (*E*)-5[(4-hydroxy-phenylimino)-methyl]-benzene-1,3-diol** **19. (*E*)-3,5-dinitro-4'-hydroxystilbene** |
| | | |

Suitable cross-linking agents are described above. Preferably, the cross-linking agent is EDGA.

In another preferred form, the ratio of polymerised 4-vinylpyridine to polymerised cross-linking agent is three to fifteen.

An expanded repertoire of templates used in MIP preparation could confer the potential to form a chemical class-selective MIP rather than a molecule-selective MIP.

The temperature of the polymerisation and the period of time over which it occurs may be important. The present inventors have found that the preferred temperature range for preparing MIPs of the present invention is between 50-55°C, whereas the imprinted polymers prepared in the cited literature were at a single temperature of 45°C (Xiang *et al.* [1]) for excessive periods of time, such as 24 hours, or at a single temperature of 60°C (Ma *et al.*[2] and Cao *et al.*[3]). In a preferred form, the porogen is selected from porogens that promote or facilitate hydrogen-bonding interactions.

More preferably, the porogen comprises one or more solvents selected from the group consisting of acetonitrile, acetone, ethyl acetate, and dimethylformamide. Even more preferably, the porogen further comprises one or more solvents selected from the group consisting of ethanol, methanol and dimethylsulfoxide.

In a preferred form, the porogen comprises a mixture of acetonitrile and ethanol. In an even more preferred form, the ratio of acetonitrile to ethanol is about 5 to 1.

In the MIPs of the present invention, the template may be incorporated in the polymer by non-covalent means, eg by hydrogen-bonding, π- π interactions, donor-acceptor interactions and van der Waals interactions or by covalent means such as by covalent attachment to monomers of the MIP.

Removal of the template from a covalent MIP can be achieved by any means known to be suitable to those in the art. The means include, but are not limited to, acid hydrolysis, base hydrolysis, reduction (using $NaBH_4$ or $LiAlH_4$), washing with a weak acid (to remove metal co-ordination bonds) and thermal cleavage to remove a reversible urethane bond. The latter procedure follows an adaptation of a method to cleave urethane bonds at elevated temperatures, such

as 60°C in DMSO, although the very harsh conditions of 180°C in DMSO as describe by Ki and coworkers[4] for the preparation of molecularly imprinted silica spheres, would not be suitable for the types of fully organic based monomers as detailed above. Additionally, enzymatic cleavage can be considered to be possible using *e.g.* esterases.

The present inventors have further shown that MIPs can be used to extract components from samples by using MIPs encased in "teabags" ie a permeable mesh that allows the MIP to be readily inserted or "dipped"into the sample and then removed. Suitable meshes include cotton-based materials, Gilson® 63μm sieve mesh and Sigma Aldrich dialysis tubing cellulose membrane (12 kDa MWCO).

invention provides a MIP imprinted with (*E*)-5-[(4-hydroxy-phenylimino)-methyl]-benzene-1,3-diol or 3,5-dihydroxy-*N*-(4-hydroxyphenyl)benzamide, wherein said MIP comprises a polymerised monomer.

In one embodiment, the MIP comprises a cross-linking agent.

Preferably, the MIP is for use in the extraction of resveratrol.

The monomer, cross-linking agent and porogen used in preparation of the MIP may be selected from those described previously.

Preferably, the monomer is 4-vinylpyridine and the cross-linking agent is EDGA. More preferably, the ratio of template to 4-vimylpyrdine used in preparation of the MIP is 1:3.

Preferably, the MIP comprises one or more features of one or more of the other aspects of the invention.

The invention also provides a method of extracting resveratrol from a sample, said method comprising exposing the sample to a MIP as defined above.

Preferably, the resveratrol is subsequently washed or elute from the MIP.

It would be understood by those skilled in the art that the aspects of the present invention are closely interrelated and that therefore the featurs of one aspect of the present invention may also be relevant to another aspect of the present invention.

In order that the nature of the present invention may be more clearly understood preferred forms thereof will now be described by reference to the following nonlimiting Examples.

## Examples

### A Synthesis of resveratrol and resveratrol analogues.

A number of polyfunctionalized (*E*)-stilbene analogues **1** have been synthesised as templates around which new molecularly imprinted polymers (MIPs) can be generated. These compounds, along with their "saturated" diphenethyl analogues 2, were also used as probes to investigate the characteristics of these MIPs. These compounds were synthesized using a convergent methodology where the key step was the palladium catalysed coupling of a functionalized benzoyl chloride **3** with a functionalized styrene **4.** Best results were obtained when the acid chlorides were freshly generated from the parent benzoic acids **5,** which were then immediately on-reacted. This synthetic methodology is described in Scheme 1.

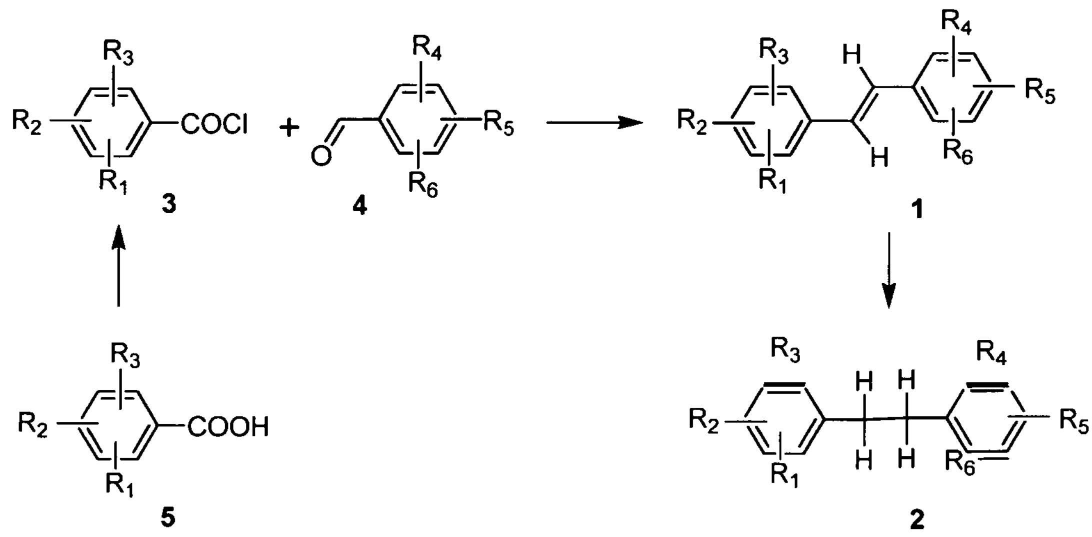

**Scheme 1:** Synthetic Methodology for Production of Stilbene and Phenethyl based Compounds

The key coupling step for generating the nitrogen isosteres is shown below in Scheme 2.

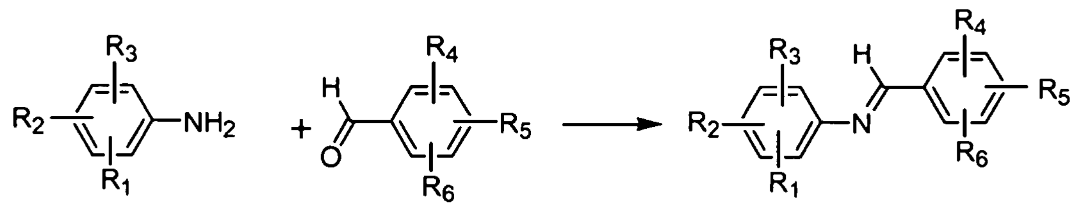

**Scheme 2.** Coupling step for the production of nitrogen isosteres of resveratrol.

Most AR solvents were used as purchased from the manufacturer except for dimethylformamide (DMF), which was dried over 4Å molecular sieves and toluene which was dried over sodium wire. Milli-Q distilled water was used for aqueous manipulations. Saturated aqueous solutions of reagents were written, for example, as sat. $NaHCO_3$. Solvent extracts of aqueous solutions were dried over anhydrous sodium sulfate, filtered and then rotary evaporated to dryness at low pressure (10-400 mbar) and 30 - 35°C in a temperature-controlled water bath.

Analytical thin layer chromatography (TLC) was performed using aluminium sheets (Merck) coated with silica gel 60 $F_{254}$. The components were visualised by (i) fluorescence at 254 nm and (ii) exposure to iodine vapour or dipping into an ethanolic phosphomolybdic acid solution and heating until charred.

Column chromatography was conducted using silica gel 60 (Merck), 0.040-0.063 mm (230-400 mesh): eluent mixtures are expressed as volume/volumes.

Melting points were determined using a Büchi B-545 melting point apparatus.

Proton nuclear magnetic resonance ($^1H$ NMR) spectra were recorded at (i) 200 MHz on a Bruker AC-200 spectrometer, (ii) 300 MHz with a Bruker DPX-300 spectrometer, or (iii) 400 MHz with a Bruker DRX-400 spectrometer. The $^1H$ NMR spectra refer to solutions in deuterated solvents as indicated. The residual solvent peaks have been used as an internal reference. Resonances were assigned according to the following convention: chemical shift (δ) measured in parts per million (ppm) relative to the residual solvent peak, multiplicity, number of protons, coupling constants (J Hz), and assignment. Multiplicities are denoted as (s) singlet, (d) doublet, (dd) doublet of doublets, (ddd) doublet of doublet of doublets, (t) triplet, (dt) doublet of triplets, (td) triplet of doublets, (q) quartet, or (m) multiplet and prefixed (b) broad where appropriate.

Carbon nuclear magnetic resonance ($^{13}C$ NMR) spectra were recorded at (i) 50 MHz on a Bruker AC-200 spectrometer, (ii) 75 MHz on a Bruker DPX-300 spectrometer, or (iii) 100 MHz on a Bruker DRX-400 spectrometer with the spectra referring to deuterated solutions in solvents indicated.

Low resolution electrospray ionisation mass spectra (ESI) were recorded on a Micromass Platform II API QMS Electrospray mass spectrometer. Analyses were conducted in both positive (ESI+) and negative (ESI-) polarity. Principle ion peaks (*m*/*z*) are reported with their intensities expressed as percentages of the base peak in brackets. High-resolution electrospray mass spectra (HRMS) were recorded on a Brucker BioApex 47e Fourier Transform mass spectrometer.

The following examples serve to illustrate these synthetic methodologies.

**Compounds Synthesised**

**Example 1.**

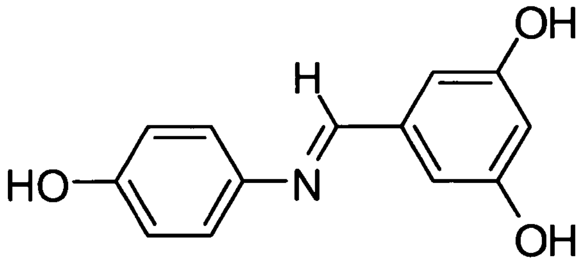

**(E)-5-[(4-Hydroxy-phenylimino)-methyl]-benzene-1,3-diol** (1). A mixture of 3,5-dihydroxybenzaldehyde, 4-aminophenol and anhydrous sodium sulphate in dichloromethane was vigorously stirred at room temperature for 3 hours. Further anhydrous sodium sulphate was added and stirring continued for 1 hour. Dichloromethane was removed by rotary evaporation. The remaining white powder was resuspended in boiling ethanol, then removed by vacuum filtration. The recovered solid was rinsed with further boiling ethanol. The clear filtrates were combined and rotary evaporated to dryness to give (*E*)-5-[(4-hydroxy-phenylimino)-methyl]-benzene-1,3-diol as a pale pink solid. lit (WO 2006/108864 A2) 162°C (dec); $^1H$ NMR ($d_6$-DMSO): δ 6.36(pseudo t, 1H, *J* = 2.2Hz, H-4), 6.80-6.85(m, 4H, H-2, H-6, H-3', H-5'), 7.16-7.22(m, 2H, $J_{ortho}$ = 8.8Hz, H-2', H-6'), 8.43 (s, 1H, imine-H), 9.47 (bs, 3H, 3 x phenolic-OH); $^{13}C$ JMOD NMR ($d_6$-DMSO): δ 106.27(4), 107.41(2,6), 116.70(3',5'), 123.42(2',6'), 139.35(1), 143.61(1'), 157.16(4'), 158.38(imine-C), 159.62(3,5); LRESI positive ion mass spectrum; m/z 230($MH^+$, 100%), 231(13%); HRESI positive ion mass spectrum;$C_{13}H_{11}NO_3Na^+$; calc. 252.0637, measured 252.0633.

A further "three point" exemplar has been generated with the synthesis of 3,5-dihydroxy-*N*-(4-hydroxyphenyl)benzamide (2). The procedure used was that described by S.J. Kim *et al.,* (Amorepacific Corporation; Applicant), "Hydroxybenzamide derivatives, the method for preparing thereof and the cosmetic composition containing the same", WO2007/021067 A1. The below scheme summarizes this preparation, starting from commercially available 3,5-dihydroxybenzoic acid and 4-aminophenol.

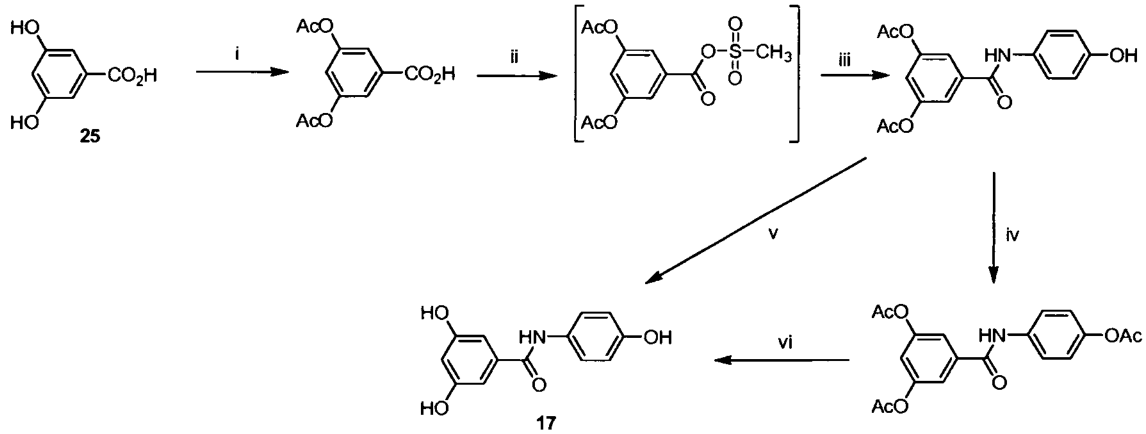


**Scheme 3** Scheme for the synthesis of 3,5-dihydroxy-*N*-(4-hydroxyphenyl)benzamide (2)

Reagents and conditions: (i) $Ac_2O$, $Et_3N$, EtOAc, reflux, 4 hours, 34% yield; (ii) $Et_3N$, THF, $CH_3SO_2Cl$, 30 minutes; (iii) 4-aminophenol, 0°C, 4 hours, 82% yield; (iv) $Ac_2O$, pyridine, DMAP, EtOAc, reflux, 2 hours, 76% yield; (v) (a) $KOH_{(aq)}$, reflux, 75 minutes. (b) $HCl_{(aq)}$, 55% yield; (vi) (a) $KOH_{(aq)}$, reflux, 2 hours. (b) $HCl_{(aq)}$, 60% yield

**Example 2.**

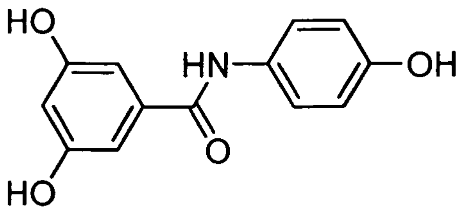

**3,5-Dihydroxy-*N*-(4-hydroxyphenyl)benzamide** (2).

**METHOD 1; Hydrolysis of the diacetate.** The reaction was conducted under a positive pressure of Argon gas. An aqueous solution of potassium hydroxide was added to "crude" 5-(4-hydroxyphenylcarbamoyl)-1,3-phenylene diacetate. The reaction was heated to reflux for 75 minutes, returned to room temperature and 1M HCl added until a precipitate formed (pH approx. 3). This was vacuum filtered off and the solid repeatedly washed in the funnel with water, and then dried under vacuum to return 3,5-dihydroxy-*N*-(4-hydroxyphenyl)benzamide as fluffy small white needles. $R_f$ 0.39 (4:1 EtOAc/hexane); mp 266.0-266.5°C; $^1H$ NMR ($CD_3OD$): $\delta$ 6.47(pseudo t, 1H, $J_{meta}$ = 2.2Hz, H-4), 6.77-6.82(m, 4H, H-3', H-5', H-2, H-6), 7.43-7.46(m, 2H, $J_{ortho}$ = 8.9Hz, H-2', H-6'); $^1H$ NMR ($d_6$-DMSO): $\delta$ 6.42(pseudo t, 1H, $J_{meta}$ = 2.2Hz, H-4), 6.72-6.78(m, 4H, H-3', H-5', H-2, H-6), 7.51-7.56(m, 2H, $J_{ortho}$ = 8.9Hz, H-2', H-6'), 9.20(s, 1H), 9.51(s, 2H), 9.83(s, 1H) ; $^{13}C$ JMOD NMR ($CD_3OD$): $\delta$ 104.41(4), 104.67(2,6), 113.92(3',5'), 122.13(2',6'), 129.10(1'), 136.08(1), 153.26(4'), 157.41(3,5), 166.70(C=O); LRESI negative ion mass spectrum; *m/z* 244 ($[M-H]^-$, 100%), 489($[2M-H]^-$, 29%); HRESI positive ion mass spectrum; *m/z* for $C_{13}H_{11N}O_4$, calculated $[MH]^+$ 246.0766, measured 246.0764.

**METHOD 2; Hydrolysis of the triacetate.** The reaction was conducted under a positive pressure of Argon gas. A solution of potassium hydroxide in water was added to "crude" 5-(4-acetoxyphenylcarbamoyl)-1,3-phenylene diacetate. The reaction was heated to reflux for 2 hours and on return to room temperature, 1M HCl was then added until a precipitate formed (pH approx. 3). This was vacuum filtered off and repeatedly washed with water. The white solid then dried under vacuum to return 3,5-dihydroxy-*N*-(4-hydroxyphenyl)benzamide as fluffy small white needles. Melting point and spectroscopic data were identical to the previously prepared material.

**B Molecularly Imprinted Polymers: Resveratrol**

**Design of Molecularly Imprinted Polymers**

The design of molecularly imprinted polymers (MIPs) requires the selection of a monomer species that will interact favourably with the intended template species, such that a pre-polymerisation complex is formed between template and monomer. The use of tools such as molecular modelling and NMR spectroscopy assist in the selection of appropriate monomers by performing a 'virtual screen', which reduces the number of polymer preparations required in MIP development.

**Molecular Modelling**

Modelling calculations were performed using PM3 geometry optimisation without solvent considerations to yield theoretical energy of formation values, $\Delta H_f$. The geometry optimisation and surface electron potential of resveratrol was generated (Figure 1), with electronegative regions shown in red and electropositive in blue, giving some insight into how resveratrol may interact with potential functional monomers.

Monomer clusters were modelled, with clusters sizes ranging from 1 to 6 monomer units. These calculations yielded predictive $\Delta H_f$ values for the interaction of the monomer with itself at these cluster sizes (Table 2). Introduction of the theoretical geometry optimisation and surface electron parameters for resveratrol parameters permit estimation of the average energy of formation for the complex (Figure 2) determined using the following equation:

$$\Delta E_i = \left(\Delta H_{f_Complex} - \left(\Delta H_{f_Template} + \Delta H_{f_Monomer}\right)\right).$$

The presence of cross-linking monomers were not included in these modelling calculations.

**4-Vinylpyridine based Complexes**

4VP is expected to interact through both hydrogen bonding and aromatic π-π interactions (Figure 3).

**Table 2.** PM3 calculations of $\Delta E_i$ and $\Delta H_f$ for Resveratrol - 4VP modelling titration.

| 4VP Monomer eq | Av. $\Delta H_\phi$ Kcal/mol monomer cluster | Av. $\Delta H_\phi$ Kcal/mol Resveratrol | Av. Kcal/mol ΔE complex | Av. $\Delta E_i$ interaction Kcal/mol |
|---|---|---|---|---|
| 1 | 46.1118 | -74.5499 | -30.5636 | -2.1255 |
| 2 | 87.7339 | -74.5499 | 9.0896 | -4.0944 |
| 3 | 132.8645 | -74.5499 | 51.4990 | -6.8156 |
| 4 | 175.7882 | -74.5499 | 94.5293 | -6.7089 |
| 6 | 261.6890 | -74.5499 | 179.6830 | -7.4561 |

Table 2 shows that the optimum ratio of resveratrol to 4VP in the formation of a MIP should theoretically be 1:3. Similar studies for the imine (*E*)-5-[(4-hydroxy-phenylimino)-methyl]-benzene-1,3-diol or the amide 3,5-dihydroxy-*N*-(4-hydroxyphenyl)benzamide also show 1:3 ratios.

Both Ma et al and Zhuang et al (J. Chromatographic Sci. (2008), 46(8), pp 739-742, have reported the preparation of MIPs from reseveratrol and 4-vinylpyridine. However, they did not identify the optimal 1:3 binding ratio either by empirical or modelling techniques.

**Acrylamide and 4-vinylbenzoic acid complexes**

Modelling titration experiments were conducted using various other functional monomers, including acrylamide (AAM) and 4-vinylbenzoic acid (4VBA) for which the data are shown below, as well as with 4-vinylpyridine (4VPyr). Because AAM has been employed by *Xiang et al.*[1] in a unsystematic experiment based on a resveratrol:AAM:EGDMA ratio of 1:6:30, we replicated this preparation for comparison purposes, despite the fact that our modelling studies indicating the requirement for only 3 equivalents AAM were needed for optimal MIP construction. Static binding evaluation data for the AAM-based MIP derived with the 1:6:30 ratio confirmed our conclusion from the modelling studies, with the results indicating that although some low affinity for resveratrol was observed, the selective performance was significantly poorer than for MIPs employing 4-vinylpyridine. A molecularly imprinted polymer was also prepared using 4VBA as functional monomer based on the preparation conditions used to make MIP 8 (the most successful 4VP-based MIP). In this case, the modelling data failed to show any reliable minimum for the monomer ratio and in most cases predicted unfavourable interactions (for 2, 3 and 4 equivalents 4VBA). The static binding evaluation data supported this prediction with no recognition of resveratrol and negligible affinity observed (Figure 4). This result may be explained by the lack of suitable donor-acceptor interactions between the electron deficient aromatic groups of resveratrol and 4VBA, thus preventing any favourable interactions from taking place.

| Polymer Code | Initial Reference Code | Resveratrol Template (T) | Functional Monomer (FM) | Cross-linker (XL) | Porogen |
|---|---|---|---|---|---|
| AAM MIP | LS1-9 | 1mmol | AAM, 6 mmol | EGDMA, 30 mmol | ACN |
| AAM NIP 1 | LS1-9b | - | AAM, 6 mmol | EGDMA, 30 mmol | ACN |
| 4VBAMIP | LS2-7p12 | 1 mmol | 4VBA, 3 mmol | EGDMA, 15 mmol | ACN/EtOH (5/1) |

(continued)

| Polymer Code | Initial Reference Code | Resveratrol Template (T) | Functional Monomer (FM) | Cross-linker (XL) | Porogen |
|---|---|---|---|---|---|
| 4VBA NIP | LS2-7p12b | - | 4VBA, 3 mmol | EGDMA, 15 mmol | ACN/EtOH (5/1) |

### $^1$H NMR Spectroscopy Titrations

Resveratrol was dissolved in $CD_3CN$, and was titrated with increasing molar equivalents of 4VP. Upon each addition, $^1$H NMR spectra were recorded, and the change in aromatic -OH shifts followed. The presence of H bonding interactions was evidenced by the consistent downfield movement of the aromatic -OH shift (Table 3 and Figure 5). After 8 equivalents, the OH peak became unobservable.

**Table 3** $^1$H NMR titration for Resveratrol against increasing amounts of 4VP.

| Resveratrol (mmol) | 4VP molar eq | 4VP vol. (μL) additions | 4VP total vol. (μL) | Δ (ppm) |
|---|---|---|---|---|
| 0.1 | 0 | 0 | 0 | 6.95 |
| 0.1 | 0.5 | 5.1 | 5.1 | 6.99 |
| 0.1 | 1.0 | 5.1 | 10.2 | 7.05 |
| 0.1 | 2.0 | 5.1 | 20.4 | 7.16 |
| 0.1 | 3.0 | 10.2 | 30.6 | 7.29 |
| 0.1 | 4.0 | 10.2 | 40.8 | 7.43 |
| 0.1 | 6.0 | 20.4 | 61.2 | 7.65 |
| 0.1 | 8.0 | 20.4 | 81.6 | 7.75 |

Resveratrol imprinted monoliths were prepared using 4VP and EGDMA as functional and cross-linking monomers respectively in various porogenic mixtures (Table 4) After polymerisation monoliths were ground, sieved to a particle size of 60-90 μm, followed by repeated washing in methanol containing acetic acid (10% v/v) to remove the template.

### Molecularly Imprinted Polymers (MIPs) Templated with Resveratrol

First and Second generation molecularly imprinted polymers (MIPs) were prepared as described in Table 4, using the following procedure. In a glass test tube fitted with a suba seal, the template (resveratrol) and functional monomer (4VP) were sonicated for 10 minutes prior to the addition of cross-linker (ethylene glycol dimethacrylate, EGDMA) and free radical initiator (AIBN) followed by $N_{2(g)}$ purge for 10 minutes. Thermal polymerization for 1$^{st}$ generation MIPs was carried out at 45°C for 12 hours, then at 50°C for 24 hours, and for 2$^{nd}$ generation MIPs thermal polymerization was performed at 50°C for 16 hours, then 55°C for 5 hours and 60°C for 4 hours, resulting in bulk monolith polymers. Non-imprinted control polymers (NIPs) were prepared in exactly the same manner, but in the absence of the template. Monolith polymers were removed from reaction tubes and ground using a Reutsh 200 ball mill, then sieved to size (60-90 μm). Fines were removed by repeated suspension of polymer particles in acetone and filtering through a 20 μm glass frit. The resveratrol template was removed by repeated washing in methanol/acetic acid (10%) until no template was present as observed by UV-VIS spectrophotometry at $v_{max}$ of 235 nm, 307 nm and 321 nm. UV-Vis analysis of the washing extracts showed that > 99% of the template had been removed.

**Table 4**

| Polymer Code | Resveratrol Template (T) | Functional Monomer (FM) | Cross-linker (XL) | Porogen |
|---|---|---|---|---|
| MIP 1 | 1mmol | 4VP, 1 mmol | EGDMA, 10 mmol | ACN, 15 mL |
| NIP 1 | - | 4VP, 1mmol | EGDMA, 10 mmol | ACN, 15 mL |
| MIP 2 | 0.33 mmol | 4VP, 1 mmol | EGDMA, 10 mmol | ACN, 15 mL |
| MIP 3 | 0.25 mmol | 4VP, 1 mmol | EGDMA, 10 mmol | ACN, 15 mL |
| MIP 4 | 0.17 mmol | 4VP, 1 mmol | EGDMA, 10 mmol | ACN, 15 mL |
| NIP 2-4 | - | 4VP, 1 mmol | EGDMA, 10 mmol | ACN, 15 mL |

(continued)

| Polymer Code | Resveratrol Template (T) | Functional Monomer (FM) | Cross-linker (XL) | Porogen |
|---|---|---|---|---|
| MIP 5 | 1.32 mmol | 4VP, 4 mmol | EGDMA, 55 mmol | ACN, 20 mL |
| NIP 5 | - | 4VP, 4 mmol | EGDMA, 55 mmol | ACN, 20 mL |
| MIP 6 | 0.33 mmol | 4VP, 1 mmol | EGDMA, 10 mmol | EtOH/$H_2O$ (4:1 v/v), 5 mL |
| NIP 6 | - | 4VP, 1mmol | EGDMA, 10 mmol | EtOH/$H_2O$ (4:1 v/v), 5 mL |
| MIP 7 | 1 mmol | 4VP, 3 mmol | EGDMA, 15 mmol | ACN, 15 mL |
| NIP 7 | - | 4VP, 3mmol | EGDMA, 15 mmol | ACN, 15 mL |
| MIP 8 | 1 mmol | 4VP, 3 mmol | EGDMA, 15 mmol | ACN/EtOH (5 :1 v/v), 6 mL |
| NIP 8 | - | 4VP, 3 mmol | EGDMA, 15 mmol | ACN/EtOH (5 :1 v/v), 6 mL |
| MIP 9 | 1 mmol | 4VP 3 mmol | EGDMA, 20 mmol | ACN/EtOH (5:1 v/v), 6 mL |
| NIP 9 | - | 4VP 3 mmol | EGDMA, 20 mmol | ACN/EtOH (5:1 v/v), 6 mL |
| MIP 10 | 1 mmol | 4VP 3 mmol | EGDMA, 17 mmol | ACN/EtOH (5:1 v/v), 6 mL |
| NIP 10 | - | 4VP 3 mmol | EGDMA, 17 mmol | ACN/EtOH (5:1 v/v), 6 mL |
| MIP 11 | 1 mmol | 4VP 3 mmol | TRIM, 3mmol | ACN/EtOH (5:1 v/v), 6 mL |
| NIP 11 | - | 4VP 3 mmol | TRIM, 3mmol | ACN/EtOH (5:1 v/v), 6 mL |
| MIP 12 | 1 mmol | *N*VIM 3 mmol | EGDMA 15mmol | ACN/EtOH (5:1 v/v) |
| NIP 12 | - | *N*VIM 3 mmol | EGDMA 15mmol | ACN/EtOH (5:1 v/v) |
| MIP 13 | 1 mmol | *N*VIM 6 mmol | EGDMA 15mmol | ACN/EtOH (5:1 v/v) |
| NIP 13 | - | *N*VIM 6 mmol | EGDMA 15mmol | ACN/EtOH (5:1 v/v) |
| MIP 14 | 1 mmol | *N*VIM 6 mmol | TRIM 6mmol | ACN/EtOH (5:1 v/v) |
| NIP 14 | - | *N*VIM 6 mmol | TRIM 6mmol | ACN/EtOH (5:1 v/v) |

An example of a Typical MIP preparation is described below:

**Preparation of MIP 8 (LS3-54p118-1-7-08)**

228.2 mg of resveratrol (1 mmol) and 321 μL of 4VP (3 mmol) were dissolved in 1 mL of EtOH and 5 mL of ACN in a 15 mL test tube and sealed with a suba seal. The solution was purged with $N_{2(g)}$ for 2 minutes and then sonicated for 20 minutes. To the solution was added 2.83 mL of EGDMA (15 mmol) and 51 mg of AIBN (0.31 mmol) radical initiator. The reaction mixture was then purged with $N_{2(g)}$ for a further 2 minutes prior to being submersed into a 50°C water bath for 18 hours. The water temperature was then increased to 60°C for 24 hours. As the polymerisation initiation liberates $N_{2(g)}$, the test tube was fitted with a needle and syringe barrel with plunger to act as a pressure valve. This was repeated for the NIP control polymer without the addition of the resveratrol template.

The bulk polymer monolith was then removed from the test tube, crushed and ground to a particle size of 60 -90 μm using a Restch 200 ball mill. The resulting polymer particles were the washed repeatedly in MeOH/AcOH (10%) (v/v) to remove the template and any unreacted monomer species. The MIP and NIP materials were then washed with MeOH to remove any traces of AcOH and dried under reduced pressure.

**MIP Binding Evaluation**

Initial MIP binding studies were performed using 4.5mL fritted polypropylene reaction tubes fitted to a Mettler Toledo MiniBlock™ system. Using this apparatus, first generation MIPs were rapidly evaluated for resveratrol affinity after a short incubation period, followed by vacuum assisted filtration into fraction collection tubes. These evaluations were designed to identify the most suitable template to functional monomer ratio. The rebinding studies were performed as follows:

(1) 50 mg of MIP (or respective NIP control) was measured into a reaction tube fitted with a 25 μm polypropylene porous frit;

(2) a rebinding solution of resveratrol (0.05 mM) in acetonitrile was added and the reaction mixture was incubated for 30 min at room temperature with continuous shaking;

(3) the filtrate was collected, diluted five-fold with acetonitrile - a 3 mL aliquot was analysed by UV-VIS spectroscopy at $v_{max}$ 235 nm, 307 nm and 321 nm using a quartz cuvette with a 1 cm path length and the concentration of free resveratrol calculated from an experimentally determined a five point calibration curve. The concentration of resveratrol bound to the MIP (or respective NIP) was then determined as the difference between the initial resveratrol concentration and the final free resveratrol concentration. This was then expressed as the amount bound, B, in μmoles/g polymer.

(4) MIPs were washed sequentially with acetonitrile methanol and methanol containing acetic acid (10% v/v) and again with methanol to remove bound resveratrol.

MIP negative control experiments for resveratrol binding were performed identically to the above procedure, with the exception that the rebinding solution did not contain resveratrol.

Figure 6 illustrates the binding of resveratrol to MIPs prepared with the template resveratrol and using 4VP as the functional monomer. Multiple fast screening assays were performed using 1 mL of resveratrol solution (0.05 mM) in acetonitrile to demonstrate the affinity of the MIP for the template resveratrol and the reproducibility of binding. Evidence of an imprinting effect is clearly apparent for each of the resveratrol imprinted polymers prepared and tested.

To further ascertain the most appropriate template to functional monomer ratio, an assay employing a 10 fold increase in resveratrol concentration (0.5 mM) was employed. The results are set out in Figure 7.

The relative imprinting factor (IF), relating the performance of the MIP with respect to the NIP control, was determined for each MIP, from

$$\mathrm{I}F = B_{\mathrm{MIP}}/B_{\mathrm{NIP}} ,$$

where $B_{MIP}$ and $B_{NIP}$ is the amount of resveratrol bound to the MIP and NIP respectively.

**Table 5.** Relative binding performance with corresponding imprinting factors (I*F*) for assays at both resveratrol concentrations of 0.05 and 0.5 mM.

| | 0.05 mM Resveratrol Assay | | | 0.5 mM Resveratrol Assay | | |
|---|---|---|---|---|---|---|
| Polymer | $B_{MIP}$ μmoles/g | $B_{NIP}$ μmoles/g | I*F* | $B_{MIP}$ μmoles/g | $B_{NIP}$ μmoles/g | I*F* |
| MIP 1 | 0.904 | 0.376 | 2.40 | 5.706 | 3.411 | 1.67 |
| MIP 2 | 0.946 | 0.448 | 2.11 | 8.031 | 3.481 | 2.31 |
| MIP 3 | 0.746 | 0.448 | 1.70 | 6.080 | 3.481 | 1.75 |
| MIP 4 | 0.909 | 0.448 | 2.03 | 4.147 | 3.481 | 1.19 |

Thus, in accordance with the modeling data, the most suitable template:monomer ratio was identified to be 1:3 for the preparation of a resveratrol imprinted polymer using 4VP as the functional monomer (Table 5). MIPs prepared in this manner demonstrated maintained high performance levels (highest capacity and IF values) after consecutive repeated binding studies at both low and intermediate resveratrol concentrations (0.05 and 0.5 mM respectively).

**Effect of Porogen and Cross-linking Amount**

MIPs based on this formulation were subsequently prepared with varying amounts of EGDMA to assess the effect of cross-linking on binding affinity. Example MIPs are MIP 2, MIP 5, MIP 7, MIP 8, MIP 9 and MIP 10).

However it was observed that MIP 7 resulted in a pseudo precipitation polymerisation that produced a talc-like polymer which was physically soft or weak and was reduced to fines or beads of a particle size < 20 μm. This was due to the low amount of cross-linking and large porogen volume which was required to dissolve the resveratrol template. Although such sizes are useful for analytical techniques, this polymer was deemed unsuitable for future large scale or industry scale separation techniques, as the small particle size would result in unacceptably high back-pressures regardless of binding performance (data not shown).

Therefore a small amount of EtOH was included in the ACN porogen (MIP 8) which increased the solubility of the template resveratrol thus affording a smaller porogenic volume and the preparation of a monolithic polymer which could be ground and sieved to a suitable particle size.

An example preparation for a polymer containing an ACN/EtOH (5:1 v/v) porogenic mixture is described below for MIP 8 (LS2-6p10-7-2-07):

Static binding assays were employed to evaluate the effect of cross-linking. An example of an assay is detailed below:

Into a 1.7 mL eppendorf tube was measured 30 mg of MIP, 1.5 mL of resveratrol (0.5 mM) in acetonitrile and the resulting sample mixture was mixed for 18 hours using a rotary suspension mixer at 28°C. Each sample was prepared in duplicate and repeated with NIP control samples. In addition to this blank control samples were prepared using 30 mg of the respective polymer with 1.5 mL of blank acetonitrile solution. The samples was then centrifuged at 13000 rpm for 15 minutes and a 200 μL aliquot removed for reverse phase HPLC analysis under isocratic conditions with UV-Vis detection at a wavelength of $\gamma$ = 321 nm. The area under the curve was determined and the concentration of free resveratrol determined using an experimental five point calibration curve. The concentration of resveratrol bound to the MIP was calculated by subtracting the free resveratrol concentration from the concentration of the binding solution. The results are set out in Figure 8.

An important aspect of this invention is the ability to replicate the MIP preparation and subsequent binding affinity. As an example of this MIP 8 was prepared in multiple batches (Figure 9), and the binding performance observed via static binding studies as described above. The results clearly show good reproducibility with consistent resveratrol binding performance of approximately 10-11 μmoles/g of polymer.

Further studies into binding characteristics of MIPs discussed within this invention include the static capacity and static cross-reactivity studies for which examples using MIP 8 are discussed below. Static Capacity Binding Assay. Into 1.7 mL eppendorf tubes was weighed 30 mg of polymer (both MIP and NIP) to which was added increasing concentrations of resveratrol solution at constant volume (1.5 mL) in acetonitrile. The resulting mixture was incubated while mixing at approximately 40 rpm for 18 hours (unless stated otherwise), after which the mixture was centrifuged at 13000 rpm for 15 minutes. A 200 μL aliquot of the supernatant was removed and analysed by reverse phase HPLC analysis with UV detection, and the concentration of resveratrol in the supernatant determined via a 5 point calibration curve. This was subtracted from the initial binding solution to give the amount of resveratrol bound. Binding (*B*) was expressed as analyte *bound, B* in μmol/g polymer.

Preliminary data shows no clear capacity (as would be evidenced by a plateau in the curve) while the non-specific binding on the NIP control polymer was observed to be quite variable over repeat binding experiments (Figure 10) and surprisingly high.

**Static Cross-Reactivity Binding Assay**

For static cross-reactivity studies the binding procedure was the same as above with the exception that the concentration of analyte binding solutions was kept at 0.5 mM.

Cross-reactivity studies on $MIP_E$ (LS 2-6p10-7-1-07) were continued with a range of zero, one, two, three and four point compounds. Compounds included in the study were: Resveratrol **(1),** 3,5-dihydroxy-N-(4-hydroxyphenyl)benzamide (BDp105-21-8-07) **(2),** 'Green' Resveratrol (BDp55-11-12-2006) **(3),** (E)-5-(4-hydroxystyryl)benzene-1,2,3-triol (BDp75-23-7-2007) **(4),** (E)-4-(3,5-dinitrostyryl)phenol (BDp15-2-11-06) **(5),** (E)-4-(3,5-dimethylstyryl)phenol (BDp127-22-9-06) **(6),** (E)-5-styrylbenzene-1,3-diol (BDp123-8-3-07) (7), Caffeic acid **(8),** (E)-3,4'-(ethene-1,2-diyl)diphenol (BDp35-23-5-07) **(9),** (E)-4-styrylphenol (BDp81-5-10-2007) **(10),** (E)-3-styrylphenol (BDp47-15-6-07) **(11),** (E)-Stilbene **(12),** 5-(4-hydroxyphenethyl)benzene-1,3-diol (BDp55-12-7-2006) **(13),** 5-(4-hydroxyphenethyl)benzene-1,2,3-triol (BDp67-16-7-2007) **(14),** 3-(4-hydroxyphenethyl)phenol (BDp35-23-5-07) **(15),** Bisphenol A **(16),** 3-phenethylphenol (BDp55-27-6-07) **(17),** 4-phenethylphenol (BDp89-9-8-06) **(18).**

The numbering scheme used in this section corresponds to the numbering scheme in Figure 11.

Cross-reactivity binding data (Figure 11) highlights some of the structural requirements related to binding affinity onto MIP 8. It is clear that binding affinity is strongly influenced by the combined presence of multiple hydrogen bonding units and C=C, C=N or NH-C=O linker groups. The affinity and selectivity of the amide and imine analogues **(2** and **3** respectively) is similar to that of resveratrol with approximately 55 % of the applied analyte retained on MIP 8. Interestingly the removal of the C=C double bond **(14)** while retaining the extra -OH group, resulted in a decrease in sorption to both MIP 8 and NIP 8 by approximately 65 %. This same trend was observed for all species, although to a lesser degree, indicating that either the increased electron density or rigidity afforded by the C=C linkage results in greater affinity and in many cases selectivity of MIP 8.

Other notable observations were the loss of affinity upon removal of two or more -OH units such as with the

nitro **(5),** dimethyl **(6)** and stilbene **(12)** analogues for which minimal affinity was observed.

**MIPs Employing Resveratrol Analogues as Templates**

Some of the resveratrol analogues that showed affinity towards MIP 8, such as the amide and the imine analogues (Figure 11) were also incorporated into MIP materials as templates in order to generate a cavity capable of binding resveratrol and or other polyphenols, thus not requiring resveratrol as the template. These MIP materials were prepared as summarized in Table 6 using the procedure described above for MIP 8.

**Table 6** Summary of some examples of MIP preparations employing resveratrol analogues as the template species.

| Polymer Code | Template (T) | Functional Monomer (FM) | Cross-linker (XL) | T:FM:XL Ratio | Porogen |
|---|---|---|---|---|---|
| MIP 15 | (*E*)-5-((4-hydroxyphenyli mino)methyl)ben zene-1,3-diol (BDp55-11-12-2006), 0.33 mmol | 4VP, 1 mmol | EGDMA, 5 mmol | 1:3:15 | ACN/EtOH (5:1), 2 mL |
| MIP 16 | 3,5-dihydroxy-N-(4-hydroxyphenyl)b enzamide (BDp105-21-8-07), 0.5 mmol | 4VP, 1.5 mmol | EGDMA, 7.5 mmol | 1:3:15 | Acetone 3 mL |
| NIP 16 | - | 4VP, 1.5 mmol | EGDMA, 7.5 mmol | 0:3:15 | Acetone 3 mL |
| MIP 17 | (E)-4-(3,5-dinitrostyryl)phe nol (BDp15-2-11-06), 0.25 mmole | 4VP, 0.75 mmole | EGDMA, 3.75 mmole | 1:3:15 | ACN/EtOH (5:1), 1.5 mL |
| MIP 18 | (*E*)-5-(4-hydroxystyryl)be nzene-1,2,3-triol (BDp75-23-7-2007), 0.25 mmole | 4VP, 1mmol | EGDMA, 5 mmol | 1:4:20 | Acetone, 2 mL |
| NIP 18 | - | 4VP, 1mmol | EGDMA, 5 mmol | 1:4:20 | Acetone, 2 mL |

Examples of molecularly imprinted polymers templated with resveratrol analogues are shown in Figure 12, which display their respective ability to bind resveratrol from an acetonitrile solution under static conditions. Polymers imprinted with the amide and the imine analogues exhibited good resveratrol affinity, with the amide imprinted polymers binding performance approaching that of MIP 8.

**Molecularly Imprinted Solid Phase Extraction (MISPE) Studies**

In order to successfully apply MIP technology to the separation and enrichment of bioactives such as polyphenols at the industry scale, a format affording good separation and high flow is required. As such it was anticipated that an example of an appropriate format for this technology would be a scaleable liquid chromatography technique such as solid phase extraction.

**An Example of MISPE Column preparation**

Initial MISPE and non-imprinted solid phase extraction (NISPE) columns were prepared in 3.5 mL syringe barrels capped with 20 μm glass frits. 100 mg of dry MIP were slurry packed into the syringe barrels, allowed to settle overnight. A second glass frit cap was placed on the top of the wet packed column, which was then gently compressed using a plunger, with care taken to keep the column wet, thus preventing the formation of voids and cracks in the column packing. This was repeated for the respective NIP control materials. Columns were successively washed with methanol, methanol containing acetic acid (10% v/v), methanol and acetonitrile.

**General MISPE Assay Protocol**

The MIP columns were pre-conditioned with, successively, methanol containing acetic acid (10% v/v), methanol and acetonitrile). After loading of 1 mL resveratrol (or analyte) solution (0.5 mM) onto the MISPE columns, the eluate and solvent from subsequent washing steps were collected in a single fraction tube. Elution of the bound molecules was achieved by several washes with methanol containing acetic acid (10% v/v). Samples were clarified by centrifugation and an aliquot of the supernatant was analysed by reversed-phase HPLC. Samples comprising solvents other than acetonitrile or acetonitrile/$H_2O$ were evaporated under vacuum at 30°C then reconstituted in acetonitrile prior to analysis.

All samples were chromatographed *via* isocratic elution in acetonitrile/$H_2O$ (7:3) as mobile phase at a flow rate of 0.5 mL/min with UV-VIS detection at 321 nm for detection and quantification of resveratrol.

The preparation of MISPE materials have been further improved using a modified procedure and apparatus, which has resulted in MIPs that have demonstrated enhanced adsorption selectivity for resveratrol in multiple, reproducible MISPE assays. This procedure has been applied to several different MIPs prepared using various template:functional monomer:cross-linker (T:FM:XL) ratios and alternative porogens. The retention of resveratrol on these MISPE columns is shown in Figure 13, which demonstrates that MIPs prepared using acetonitrile/ethanol (5:1 v/v) with a 1:3:15 T:FM:XL ratio clearly outperforms other MIP preparations with the imprinting factor approaching 10:1 after extensive washing with acetonitrile. The presence of ethanol in the porogenic solvent has a beneficial influence, which may result from the increased solubility of the template, greater structural rigidity due to a reduced porogenic volume or by influencing intermolecular interactions, such as $\pi$-$\pi$ stacking, during the pre-polymerisation stage of preparation.

The performance of MIP 8 was subsequently examined under semi-aqueous conditions (ethanol (20% v/v in water) in MISPE format with a clean up step comprising two washes with 20% (v/v) ethanol in water, followed by successive washes with 1% (v/v) acetonitrile in methanol, and acetonitrile. Figure 14 displays high adsorption onto MIP 8 with approximately 80 % of the initial resveratrol solution retained and an imprinting factor approximately 2 after clean up with an aqueous ethanol rinse. However, selectivity between MIP and NIP was greatly increased while maintaining good levels of adsorption after washing with acetonitrile was applied to remove non-specific binding.

The effect of ethanol and water content on resveratrol affinity to MIP 8 was examined by increasing the ethanol content under aqueous binding conditions. The amount of resveratrol bound expressed as a percentage of the initial loading solution for ease of comparison (Figure 15). In addition to this, Figure 15 illustrates the application of aqueous ethanol as an environmentally friendly alternative to acetonitrile as a clean up solvent to remove non-specific binding. Based on this data it appears that an EtOH content of between 20 - 50 % results in preferential resveratrol recognition when under aqueous conditions.

### Application of a complex sample from agricultural feedstocks

In order to assess the ability of MIP 8 to concentrate resveratrol from a complex feedstock such as grape press waste and thus be an effective value adding process to the Australian food industry, a grape seed sample was applied to a 100 mg MISPE column packed with MIP 8.

Raw grape seed was provided by CSIRO, Food Science Australia Werribee (Batch No. 02VIN03). 2 × 10 g samples were measured out, one of which was spiked with 200□g of resveratrol, and continuously extracted in acetone *via* soxhlet based on procedure reported by *Romero-Perez et al.*[6] yielding control BDp119-14-9-2006 and spiked BDp119-14-9-2006 extracts. 1 g of each extract were separately suspended in 80% (v/v) ethanol, then sonnicated for 30 minutes and centrifuged at 3000 rpm for 30 minutes. 1 mL aliquots of the respective soluble extracts were applied to off-line MISPE columns packed with 100 mg of MIP 8, the washed with 80 % (v/v) ethanol followed by successive washes with acetonitrile and final elution with 10% (v/v) AcOH in methanol. A resveratrol standard solution in 80% (v/v) ethanol in water was applied to a separate MISPE column packed with MIP 8 and treated identically to the grape seed extracts. Non-imprinted control columns (NISPE) were treated exactly the same as their respective MIP counterparts. Aliquots of recovered bound material from MIPSE columns were analyzed by HPLC (see Figure 16).

Figure 16 B) and Figure 16 C) demonstrate that MISPE columns packed with MIP 8 were able to concentrate resveratrol from a complex feedstock such as grape seed extract, as evidenced by the peak consistent with resveratrol observed at 2.735 minutes after elution from the MISPE column, whereas no such peak was observed for the NISPE column.

### Demonstration of Molecularly Imprinted Solid Phase Extraction (MISPE) of Peanut Meal Extract on a 1 g of MIP Material

10g of peanut meal extract (BDp63-9-7-07) made up in 500 mL EtOH/$H_20$ (50/50 v/v). Mixture was centrifuged and filtered after which 100 mL aliquot was treated by molecularly imprinted solid phase extraction (MISPE) using 1 g of MIP 8 material (1s3-26p62-1-4-08). The MIP column was then washed with 4 column volumes (4 × 10 mL) of aqueous ethanol (EtOH/$H_2O$ 50/50 v/v), followed by a selective clean up wash using 3 column volumes of aqueous acetone (acetone/$H_2O$ 50/50 v/v). The remaining bound analytes were eluted via 5 × 5mL 10% AcOH in Me0H and 2 × 5 mL acetone. This procedure was repeated using a column packed with 1g of non-imprinted control polymer (NIP). The elution fractions were combined, evaporated to dryness and made up to 1 mL in 50 % aqueous EtOH of which 200 $\mu$L aliquots were analysed by reversed-phase HPLC employing isocratic elution at a flow rate of 0.5 mL/min (Table 7).

**Table 7.** Gradient profile employed for RP-HPLC analysis of untreated and MISPE treated peanut meal extract. Solvent A = $H_2O$ with 0.1 % AcOH, Solvent B = EtOH/$H_2O$ (80:20 v/v) with 0.1 % AcOH.

| Time (min) | % Solvent B |
|---|---|
| 0 | 25% |
| 2 | 25% |
| 6 | 37.5% |
| 9 | 37.5% |
| 12 | 62.5% |
| 15 | 62.5% |
| 18 | 100.0% |
| 22 | 100.0% |
| 25 | 25.0% |
| 29 | 25.0% |

**Results Summary**

Reversed-phase HPLC generated chromatograms of untreated and MISPE treated peanut meal extract (Figure 17) demonstrates a selective enrichment of resveratrol (approximately 20 fold increase in concentration) and the retention (no enrichment observed) of an unidentified analyte (RT = 9.6 min). It should be noted that the MISPE treatment procedure has yet to be optimised, and as such the enrichment of resveratrol and other analytes may yet be improved.

**Demonstration of Molecularly Imprinted Solid Phase Extraction (MISPE) of Peanut Meal Extract on a 1 g of MIP Material (40-fold enrichment)**

**Experimental Summary**

Peanut meal extract (10g) was made up in 500 mL EtOH/$H_2O$ (50/50 v/v). The mixture was centrifuged and filtered after which 100 mL aliquot was treated by molecularly imprinted solid phase extraction (MISPE) using 1g of molecularly imprinted polymer (MIP) material. The MIP column was then washed with 4 column volumes (4 × 10 mL) of aqueous ethanol (EtOH/$H_2O$ 50/50 v/v), followed by a selective clean up wash using 3 column volumes of aqueous acetone (acetone/$H_2O$ 50/50 v/v). The remaining bound analytes were eluted via 5 × 5mL 10% AcOH in MeOH and 2 × 5 mL acetone. This procedure was repeated using a column packed with 1g of non-imprinted control polymer (NIP). The elution fractions were combined, evapourated to dryness and made up to 1 mL in 50 % aqueous EtOH of which a 200 μL aliquots were analysed by reverse phase HPLC employing gradient elution at a flow rate of 0.5 mL/min (Table 8).

**Table 8. Gradient profile employed for RP-HPLC analysis of untreated and MISPE treated peanut meal extract. Solvent A = $H_2O$ with 0.1 % AcOH, Solvent B = EtOH/$H_2O$ (80:20 v/v) with 0.1 % AcOH**.

| Time (min) | % Solvent B |
|---|---|
| 0 | 25.0 |
| 2 | 25.0 |
| 6 | 37.5 |
| 9 | 37.5 |
| 12 | 62.5 |
| 15 | 62.5 |
| 18 | 100.0 |
| 22 | 100.0 |
| 25 | 25.0 |
| 29 | 25.0 |

**Results Summary**

Reversed phase HPLC generated chromatograms of untreated and MISPE treated peanut meal extract (Figure 18) demonstrates a selective enrichment of resveratrol with an imprinting factor (*I*) of 66. It can also be noted that an unidentified analyte (RT = 9.6 min) with a positive m/z of 577 amu was also captured but no significant enrichment was observed. It should be noted that this MISPE treatment procedure has yet to be fully optimised, and as such the enrichment of resveratrol and other analytes may be improved further.

**C. Polyphenolic template selectophores for molecularly imprinted polymers**

Reported here is the synthesis of the imine and amide selectophores of (*E*)-resveratrol. These selectophores are more easily and efficiently prepared than (*E*)-resveratrol, as both can be synthesized by a "single-pot" preparation. The performances of the new polyphenolic selectophore-imprinted polymers relative to (*E*)-resveratrol-imprinted polymer is also reported.

**Results and discussion**

**Selectophore Preparations**

(*E*)-Resveratrol **1** can be visualized as a polyphenolic compound with an alkene constraint. Analogues with a variation at this constraint were then selected for synthesis, namely the imine, (*E*)-5-[(4-hydroxy-phenylimino)-methyl]-benzene-1,3-diol **2** and the amide, 3,5-dihydroxy-*N*-(4-hydroxyphenyl)benzamide **3.** All three compounds have two resorcinol hydroxyls that are able to occupy the same 3-dimensional spaces, with the third phenolic hydroxyl then likely to adopt very similar but not identical positions.

Earlier studies in this laboratory used (*E*)-resveratrol as the original template for the preparation of MIPs. There are numerous reported methods for the synthesis of (E)-resveratrol, but most of these synthetic methods contravene both the Principles of Green Chemistry and modern industrial practicality. All of these methods invariably require multistep syntheses with resultant significant losses of materials, along with consequential purification processes, multiple handlings of noxious reagents, protection and deprotection processes and energy to drive the chemical reactions.

The process used for the synthesis of (E)-resveratrol is summarized in Scheme 4 and was used to transform 3,5-dihydroxybenzoic acid 4 to (*E*)-resveratrol 1.

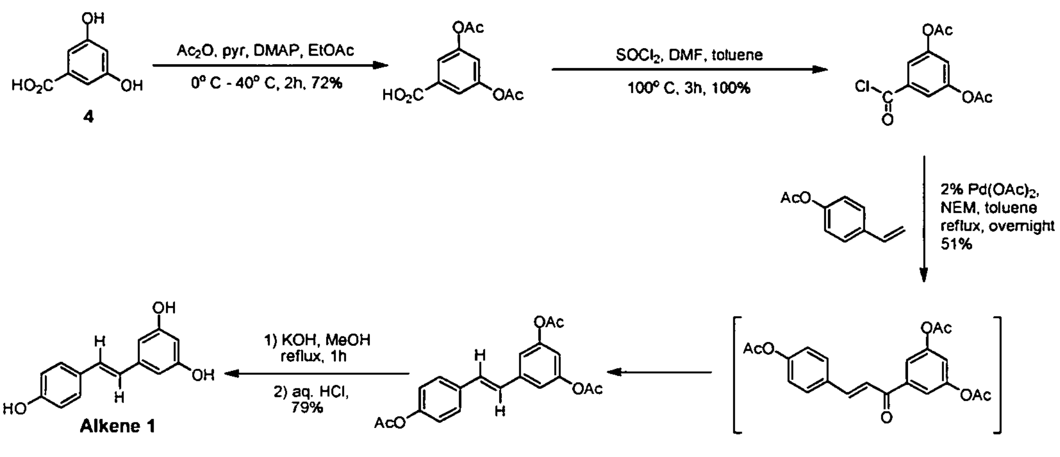


**Scheme 4** Preparation of the alkene (*E*)-resveratrol **1**.

This multistep process returned the desired product in a 29% overall yield, but typically required > 5 days to complete, and necessitated the generation and handling of corrosive and noxious materials, as well as chromatographic purifications.

The imine selectophore (*E*)-5-[(4-hydroxy-phenylimino)-methyl]-benzene-1,3-diol 2 was prepared from commercially available 4 aminophenol 5 and 3,5-dihydroxybenaldehyde 6 as shown in Scheme 5.

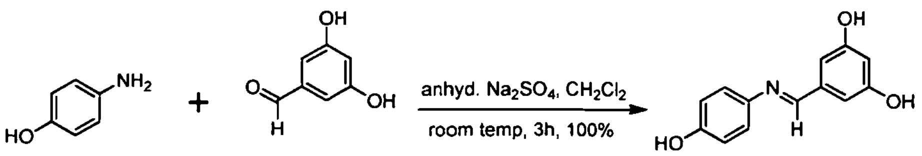


5 6 Imine 2

**Scheme 5** Preparation of the imine (*E*)-5-[(4-hydroxy-phenylimino)-methyl]-benzene-1,3-diol **2**.

We have found this method to be highly advantageous in that it rapidly gave quantitative yields of clean product after a simple filtration workup. The stability of this imine product was confirmed by $^1$H NMR studies in $d_6$-DMSO and $CD_3CN$, which showed that this compound remained unchanged in these solutions over a 6 day period. This imine selectophore also remained intact when used as the template molecule under the conditions employed for MIP preparation, and when used as an analyte to evaluate the binding properties of these MIPs.

The third selectophore example was the amide 3,5-dihydroxy-*N*-(4-hydroxyphenyl)benzamide **3.** An authentic sample was initially prepared by the multistep procedure shown in Scheme **6.**

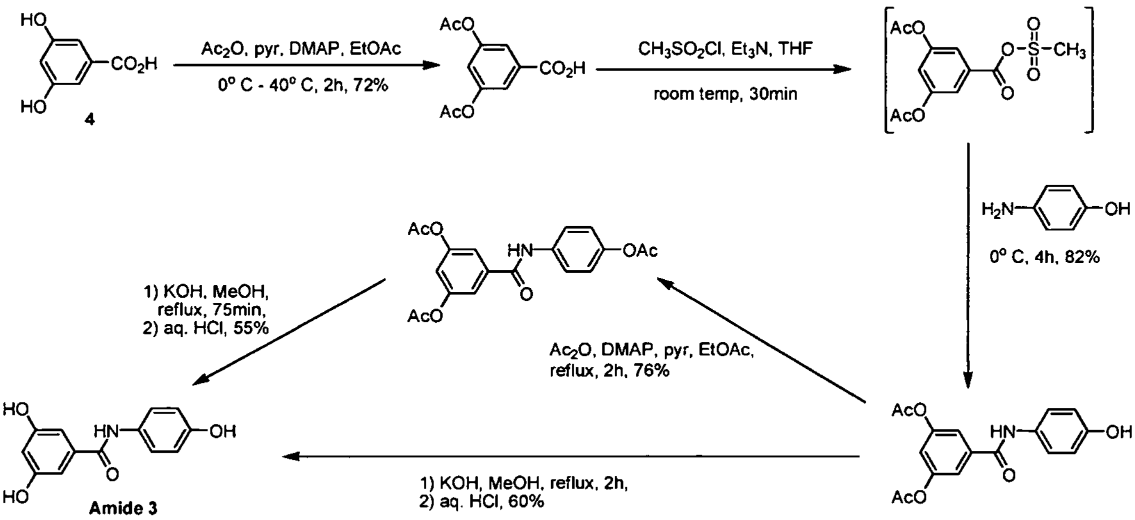


4 Amide 3

**Scheme 6** Preparation of the amide 3,5-dihydroxy-*N*-(4-hydroxyphenyl)benzamide **3**.

Full experimental details for this multistep preparation have been included in the attached electronic supplementary information. This multistep preparation of this amide **3** clearly suffered from the same "non-green" issues that were encountered in the synthesis of (*E*)-resveratrol **1.** Our ongoing requirement for larger amounts of this amide **3** encouraged us to develop an alternative improved process, which is summarized in Scheme 7.

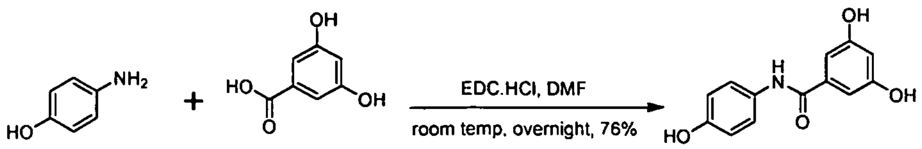


5 4 Amide 3

**Scheme 7.** Alternative preparation of amide 3,5-dihydroxy-*N*-(4-hydroxyphenyl)benzamide **3**.

Workup by removal of the solvent followed by trituration and washing with water rapidly returned the pure amide **3** in 76% yield. The 'green' advantage of this carbodiimide activated coupling is that the same starting reagents were used as for the reported multistep synthesis, but now the amide was produced in an improved overall yield, using a single-pot procedure, and did not require added energy, functional group protection and deprotection, or chromatographic purification.

**Molecularly Imprinted Polymers (MIPs)**

The three selectophores synthesized were used as templates for the preparation of MIPs, and as analytes to interrogate the binding characteristics of the new polymers . The preparation of the molecularly imprinted polymers (MIPs) briefly involved the thermally initiated free radical polymerization of a mixture of the selectophore template, the functional monomer and the crosslinker in a porogen. The resultant solid material was then ground and appropriate sized particles sieved off and washed with acid to desorb the template. Non-imprinted polymers (NIPs) were identically prepared in the absence of template molecule. Single analyte binding studies where performed using the (*E*)-resveratrol imprinted polymer ($MIP_{RES}$) and the corresponding NIP. in these studies, the polymers were incubated overnight with a freshly prepared solution of analyte, and the supernatant was then removed and its concentration determined by RP-HPLC. Subtraction from the original concentration gave the amount of analyte bound (*B*) by the polymer, and these results are summarized in Figure 19.

Within this experimental design, $MIP_{RES}$ displayed both similar binding capacity and comparable selective affinities ($B_{MIP}$ - $B_{NIP}$) for the alkene, the amide and the imine molecules. The similarity of these binding parameters suggests that $MIP_{RES}$ displays generic cross-reactivity towards each of these polyphenolic selectophores. This result is consistent with similar spatial conformations that all of these molecules adopt in solution. These selectophores are also likely to possess similar electronic characteristics as inductive variations associated with these three different linkages are likely to be moderated by both distance from the aromatic hydroxyl groups and the aromatic $\pi$ clouds. Consequently, these selectophores are similarly bound at the recognition surfaces within these MIP cavities.

The suitability of the amide and imine selectophores as (*E*)-resveratrol template mimics was then assessed. Each of these compounds was used as templates for the preparation of new MIPs *(i.e.* $MIP_{AMIDE}$ and $MIP_{IMINE}$ respectively) using a similar protocol to that employed for the preparation of $MIP_{RES}$. Static binding assays were conducted as before, and the ability of these MIPs to recognise (*E*)-resveratrol are reported in Figure 20.

The selectophore-imprinted $MIP_{AMIDE}$ and $MIP_{IMINE}$ both displayed selective affinity for (*E*)-resveratrol, but with reduced capacity compared to $MIP_{RES}$. Despite the fact that these molecules may have similar conformations in solution, the stereotopological spaces occupied by the pyridyl nitrogens of 4-vinylpyridine, which are the complementary functional binding groups during imprinting, must differ for all three MIPs. The decreased binding capacity and lower selective affinity observed for both $MIP_{AMIDE}$ and $MIP_{IMINE}$ may also reflect that these imprinted polymers have a diminished ability to bind and maintain a favoured conformation of the alkene (E)-resveratrol, a finding that may be a consequence of the increased relative structural rigidity of the alkene constraint.

**Conclusion**

Methodolgies to synthesize structural analogues with spatially defined functionalities, or selectophores, of the polyphenol (*E*)-resveratrol have been developed. These compounds were more easily and conveniently prepared using "greener" single-pot procedures. The selectophores were then used as template mimics for the preparation of MIPs, and these new polymers were found to display comparable, but not identical binding, towards this representative polyphenolic compound.

The approach is a technique that can be used to explore and characterize new MIPs, and our findings suggest that appropriate selectophores may be suitable templates to enable the more convenient preparation of generic MIPs which are capable of extracting structurally similar compounds.

**Experimental**

AR solvents were used as purchased from the manufacturer except for dimethylformamide (DMF) which was dried over 4Å molecular sieves, toluene over sodium wire, and pyridine and triethylamine over KOH pellets. Milli-Q distilled water was used for aqueous manipulations. Solvent mixtures are expressed as volume/volumes. Solvent extracts were dried over anhydrous sodium sulfate, filtered and then rotary evaporated to dryness at low pressures ($\geq$ 10 mbar) at 30 - 35°C. Analytical thin layer chromatography (TLC) was conducted on silica gel using Merck® 1.05554.001 plates. The components were visualised by (i) fluorescence at 254 nm and (ii) ethanolic phosphomolybdic acid solution dip and char. Silica gel column chromatography was conducted using Merck® 1.09385.1000. Melting points were determined by open glass capillary method and are uncorrected. NMR spectra were recorded on a Bruker DPX-300; $^{1}H$ at 300 MHz

and $^{13}C$ JMOD at 75 MHz. Deuterated solvents were used as indicated and the residual solvent peaks used for internal reference. *J* values are given in Hz. Low resolution electrospray ionisation mass spectra (ESI) were recorded using a Micromass Platform II API QMS Electrospray mass spectrometer in both positive ($ESI^+$) and negative ($ESI^-$) polarity. High-resolution electrospray mass spectra (HRMS) were recorded on a Bruker BioApex 47e Fourier Transform mass spectrometer. Mixtures were sonicated in a 37kHz/150W Elmasonic S100 ultrasonic cleaning unit. Polymers were ground using a Retsch PM 200 Planetary Ball Mill and sized on a Retsch AS 200 sieve shaker.

**Synthesis of Polyphenolic Selectophores**

**3,5-Diacetoxybenzoic acid**

A suspension of 3,5-dihydroxybenzoic acid (15.40 g, 0.100 mol) in ethyl acetate (220 mL) was cooled in an ice-bath. Acetic anhydride (24.52 mL, 0.2421 mol), pyridine (16.16 mL, 0.1998 mol) and 4-(dimethylamino)pyridine (100 mg, 0.81855 mmol) were added and the reaction stirred at 0 °C for 60 minutes and then at room temperature overnight. Formic acid (5.12 mL, 0.1357 mmol) was added and the reaction poured onto ice (ca. 500 g). Further ethyl acetate (300 mL) was added and the organic phase separated and washed with water (2 x 200 mL), sat. aq. $NaHCO_3$ (100 mL), further water (2 × 200 mL), and then dried and evaporated to a white solid. Recrystallization from 5:1 EtOAc/hexane (120 mL) gave 2 crops of 3,5-diacetoxybenzoic acid (combined weight 17.07 g, 72%) as a white powder. $R_f$ 0.20 (1:1 EtOAc /hexane), 0.39 (3:1 EtOAc/hexane); mp 161-162°C (from EtOAc/hexane) (lit.[9] mp: 157-159°C); $\delta_H(CDCl_3)$ 2.29 (s, 6H, 2 × OAc), 7.18 (pseudo t, 1H, *J 2.1,* 4-H) and 7.70 (pseudo d, 2H, *J* 2.1, 2-H, 6-H); $\delta_C(CD_3OD)$ 18.43, 118.94, 119.02, 131.70, 150.19, 165.46 and 168.17; *m/z* (ESI) 261 ($MNa^+$, 100%).

**(*E*)-3,4',5-Triacetoxystilbene**

3,5-Diacetoxybenzoic acid (8.022 g, 33.706 mmol) suspended in a mixture of toluene (130 mL), DMF (500 μL) and thionyl chloride (16.00 mL, 220.6 mmol) was heated at 100 °C for three hours under an argon gas atmosphere. The solvents were removed by vacuum distillation and the residue re-suspended in toluene (85 mL) and sonicated under vacuum to remove dissolved gases. 4-Acetoxystyrene (5.74 mL, 37.5 mmol), *N*-ethylmorpholine (4.31 mL, 33.9 mmol) and palladium diacetate (35 mg, 0.16 mmol, 0.46 mole%) were added and the reaction heated to reflux for 2 hours. Further palladium diacetate (116 mg, 0.52 mmol, 1.54 mole%) was added and the reaction left to reflux overnight. On return to room temperature, ethyl acetate (500 mL) was added, the solution was washed with 0.1 M HCl (2 × 300 mL) and water (300 mL) and then dried and evaporated to return a brown solid. Purification with column chromatography (isocratically eluted with 2:1 $Et_2O$/hexane) gave 7.888 g of a white solid, shown by $^1H$ NMR to be predominantly the desired adduct. Further chromatography (gradient eluted starting with 4:1 hexane/EtOAc and finishing with 2:1 hexane/EtOAc) returned pure (*E*)-3,4',5- triacetoxystilbene (6.071 g, 51%) as a white solid. $R_f$ 0.29 (2:1 hexane/EtOAc); mp 112.5-113.0 °C (lit[4a] mp 116 °C); $\delta_H$ ($CDCl_3$) 2.27 (s, 9H, 3 × OAc), 6.80 (pseudo t, 1H, *J* 2.1, 4'-H), 6.93 (d, 1H, *J* 16.3, $H_{trans}$), 7.03 (d, 1H, *J* 16.3, $H_{trans}$), 7.04-7.09 (m, 4H, 3-H, 5-H, 2'-H, 6'-H) and 7.44-7.47 (m, 2H, 2-H, 6-H); $\delta_C(CDCl_3)$ 20.07, 113.39, 115.88, 120.88, 126.19, 126.64, 128.64, 133.45, 138.53, 149.46, 150.34, 167.91 and 168.30; *m/z* (ESI) 377 ($MNa^+$, 100%), 378 (21).

**Alkene 1; (*E*)-Resveratrol**

The reaction was conducted under an argon gas atmosphere. Potassium hydroxide (22 mg, 0.3922 mmol) dissolved in methanol (3.0 mL) was added to a suspension of (*E*)-3,4',5- triacetoxystilbene (113 mg, 0.319 mmol) in methanol (10 mL). The solid immediately dissolved and the clear solution was gently heated to reflux for 60 minutes and a marked darkening in colouration noted. The volume was then reduced to half with rotary evaporation and the remaining solution acidified (pH 2) with 1M aq. HCl. Ethyl acetate (150 mL) was added and the reaction washed with sat. brine (3 × 20 mL), dried and evaporated to return a dark red solid. Purification with column chromatography (isocratically eluted with 100% EtOAc) gave (*E*)-resveratrol (58 mg, 79%) as a pale beige coloured solid. $R_f$ 0.65 (EtOAc); mp 261.0-263.0 °C (lit[4b] mp 255-260 °C); $\delta_H(CD_3OD)$ 6.13 (pseudo t, 1H, *J* 2.2, 4-H), 6.41-6.42 (m, 2H, 2-H, 6-H), 6.71-6.79 (m, 3H, $H_{trans}$, 3'-H, 5'-H), 6.93 (d, 1H, *J* 16.3, $H_{trans}$) and 7.29-7.36 (m, 2H, $J_{ortho}$ 8.6, 2'-H, 6'-H); $\delta_C$(CD30D) 100.30, 103.47, 114.12, 124.64, 126.43, 127.06, 128.07, 138.97, 155.89 and 157.20; *m/z* (ESI) 229($MH^+$, 100%), 230(23).

**Imine 2; (*E*)-5-[(4-Hydroxy-phenylimino)-methyl]-benzene-1,3-diol**

A mixture of 3,5-dihydroxybenzaldehyde (300 mg, 2.174 mmol), 4-aminophenol (237 mg, 2.174 mmol) and anhyd. sodium sulphate (309 mg, 2.176 mmol) in dichloromethane (15 mL) was vigorously stirred at room temperature for 3 hours. Further anhyd. sodium sulphate (309 mg, 2.176 mmol) was added and the stirring continued for an additional

one hour. Dichloromethane was removed by rotary evaporation and the white residue re-suspended in boiling ethanol (10 mL) and filtered whilst hot. The solid was washed in the funnel with further hot ethanol (10 mL). The clear filtrates were combined and rotary evaporated to dryness to give (*E*)-5-[(4-hydroxy-phenylimino)-methyl]-benzene-1,3-diol (501 mg, 100%) as a pale pink solid. $R_f$ 0.48 (2:1 EtOAc/hexane); darkens with heating with mp > 340 °C; $\delta_H$($d_6$-DMSO) 6.36 (pseudo t, 1H, *J* 2.2, 4-H), 6.80-6.85 (m, 4H, 2-H, 6-H, 3'-H, 5'-H), 7.16-7.22 (m, 2H, $J_{ortho}$ 8.8, 2'-H, 6'-H), 8.43 (s, 1H, imine-H), 9.47 (bs, 3H, 3 × phenolic-OH); $\delta_C$($d_6$-DMSO) 106.27, 107.41, 116.70, 123.42, 139.35, 143.61, 157.16, 158.38 and 159.62; *m/z* (ESI) 230($MH^+$, 100%), 231(13); *m/z* (HRESI) 252.0633 ($[M + Na]^+$ $C_{13}H_{11}NO_3Na^+$ requires 252.0637).

**Amide 3; 3,5-Dihydroxy-*N*-(4-hydroxyphenyl)benzamide**

The reaction was conducted under a positive pressure of argon gas. 3,5-Dihydroxybenzoic acid (2.310 g, 15.000 mmol) and 4-aminophenol (1.962 g, 18.000 mmol) were dissolved in DMF (90.0 mL). *N*-Ethyl-*N'*-(3-dimethylaminopropyl)carbodiimide hydrochloride (3.450 g, 17.997 mmol) was added and the reaction stirred at room temperature overnight. The solvent was removed by rotary evaporation under high vacuum and the oily residue co-distilled with water until a solid formed. This solid was triturated with cold 0.01 M HCl (50 mL), filtered, and the pale purple coloured product washed in the funnel with cold water (4 × 20 mL). The solid was dried with desiccant under vacuum to give pure 3,5-dihydroxy-*N*-(4-hydroxyphenyl)benzamide (2.791g, 76%); $R_f$ 0.39 (4:1 EtOAc/hexane); mp 266.0-266.5 °C; $\delta_H$($CD_3OD$) 6.47 (pseudo t, 1H, $J_{meta}$ 2.2, 4-H), 6.77-6.82( m, 4H, 3'-H, 5'-H, 2-H, 6-H), 7.43-7.46 (m, 2H, $J_{ortho}$ 8.9, 2'-H, 6'-H); $\delta_H$($d_6$-DMSO) 6.42 (pseudo t, 1H, $J_{meta}$ 2.2, 4-H), 6.72-6.78 (m, 4H, 3'-H, 5'-H, 2-H, 6-H), 7.51-7.56 (m, 2H, $J_{ortho}$ 8.9, 2'-H, 6'-H), 9.20 (s, 1H), 9.51 (s, 2H) and 9.83 (s, 1H); $\delta_C$($CD_3OD$) 104.41, 104.67, 113.92, 122.13, 129.10, 136.08, 153.26, 157.41 and 166.70; *m/z* (ESI) 244 ($[M-H]^-$, 100%), 489($[2M-H]^-$, 29); *m/z* (HRESI) 246.0764 ($[M + H]^+$ $C_{13}H_{12}N_1O_4^+$ requires 246.0766).

**Preparation of Molecularly Imprinted Polymers**

Molecularly imprinted polymers (MIPs) were prepared by dissolving the selectophore template (1 mmol) in porogen (5:1 acetonitrile/EtOH, 6 ml) in a disposable glass test tube. Acetone (6 mL) was the preferred porogen with the amide 3 due to its incomplete solubilization in the original mixture volume.[10] The functional monomer 4 vinylpyridine (322 μL, 3 mmol) was added and the mixture sonicated for 10 minutes. Ethyleneglycol dimethacrylate (2.314 mL, 15 mmol) was then added as cross-linker and 2,2-azobis(isobutyro)nitrile (51 mg, 0.31 mmol) added as radical initiator. This pre-polymerisation mixture was sparged with nitrogen gas for 5 minutes before being heated in a thermostatic water bath at 50 °C for 24 hours. The solid polymers were removed from the reaction tubes, crushed with a mortar and pestle and finely ground using a Retsch 200 ball mill. Grounds were sieved and the 63 - 100 μm size particles retained. This size range was repeatedly suspended in acetone and the supernatant poured off to remove fines. Bound template was then removed from the polymer by repeated washings with methanolic 10% acetic acid until the washings were free of template (monitored by UV-Vis spectroscopy at 321 nm). Non-imprinted control polymers (NIPs) were prepared using the same procedure in the absence of a template molecule.

**Evaluation of Molecularly Imprinted Polymers**

Experiments were conducted on both MIP and NIP in parallel under identical conditions.

Analyte solution (1.5 mL, 0.5 mM in acetonitrile) was added to the polymer (30 mg) in an Eppendorf tube. This was mixed at 40 rpm for 18 hours and the polymer settled by centrifugation for 15 minutes at 13000 rpm. A 200 μL aliquot of the supernatant was removed and analysed by RP-HPLC with UV detection at 321 nm. Concentration was determined by comparison with a linear 5 point calibration curve. The amount of analyte bound (*B*) was determined as the difference between this and the initial value and reported in units of μmol/g polymer.

**D. Tandem molecularly imprinted solid phase extraction of resveratrol and related polyphenols**

In this example, a rapid tandem MISPE approach for the isolation and concentration of (*E*)-resveratrol and related polyphenols in high purity from peanut meal is reported.

**MATERIALS AND METHODS**

*Compounds*

4-Vinylpyridine (4VP), ethyleneglycol dimethacrylate (EGDMA) and 2,2'-azobis(2-methylpropionitrile) (AIBN), catechin and piceid were obtained from Sigma-Aldrich (Sydney, NSW, Australia). 4-VP and EGDMA were purified

immediately prior to use via vacuum distillation and alumina column chromatography, respectively. All solvents used were HPLC grade.

(*E*)-Resveratrol **1** and the structurally related polyphenolic analogue 3,5-dihydroxy-*N*-(4-hydroxyphenyl)benzamide 2 were synthesized as described above.

*MIP preparation*

Several MIPs and their non-templated counterparts (NIPs) were prepared as summarized in **Table 9.** $MIP_{RES}$ was prepared at 50 °C (18 hours) followed by a thermal annealing treatment at 60 °C (24 hours). $MIP_{AMIDE}$ was produced with a different porogen at 55 °C (40 hours). Both these preparations produced MIPs having comparable binding performance.

**Table 9.** Synthesis conditions of the MIPs used.

| Polymer | Template | Functional monomer (4VP) | Cross-linker (EGDMA) | Porogen |
|---|---|---|---|---|
| + $MIP_{RES}$ | 1 mmol (1) | 3 mmol | 15 mmol | $CH_3CN$/EtOH 5:1 v/v |
| +$NIP_{RES}$ | - | 3 mmol | 15 mmol | $CH_3CN$/EtOH 5:1 v/v |
| * $MIP_{AMIDE}$ | 1 mmol **(2)** | 3 mmol | 15 mmol | $CH_3COCH_3$ |
| * $NIP_{AMIDE}$ | - | 3 mmol | 15 mmol | $CH_3COCH_3$ |

+ Prepared at 50 °C with thermal annealing treatment at 60 °C in 6 mL of porogen.
* Prepared at 55 °C in 5 mL porogen.

*Reversed-phase chromatography (RP-HPLC)*

The RP-HPLC separation was performed on an Agilent Technologies 1100 LC system (Waldbronn, Germany) consisting of a binary pump with a vacuum degasser, autosampler with a 900 μL sample loop, thermostated column compartment and a diode-array detector. Injected samples were analysed on a Zorbax Eclipse XDB-$C_{18}$ column (4.6 × 150 mm, 5 μm particle size) at 40 °C. The mobile phase consisted of 0.1 % AcOH in $H_2O$ (solvent A) and 0.1 % AcOH in EtOH/$H_2O$ or MeOH/$H_2O$ (8:2 v/v) (solvent B), applying the following gradient: 0-2 min: 25 % B isocratic, 2-6 min: 25-37.5 % B, 6-9 min: 37.5 % B isocratic, 9-12 min: 37.5-62.5 % B, 12-15 min: 62.5 % B isocratic, 15-18 min: 62.5-100 % B, 18-22 min: 100 % B isocratic, 22-25 min: 100-25 % B, 25-29 min: 25 % B isocratic. The flow rate was 0.5 mL $min^{-1}$. The injection volume was 5 μL with the UV-Vis diode array detector (80 Hz) set to the absorbance wavelengths of $\lambda$ = 280, 321 and 370 nm.

*Preparation of peanut meal extract*

Peanut meal (200 g) in EtOH:$H_2O$ (1000.0 mL, 4:1 v/v) was sonicated for 60 min, after which the mixture was filtered and the solvent evaporated to return 17.9 g of extract. A peanut meal extract for use in subsequent experiments was prepared by adding 10.0 g of this material to a solution of EtOH/$H_2O$ (1:1 v/v) and diluting to 500 mL in a volumetric flask and stored at 4 °C until required. Prior to use, the peanut meal extract was brought to room temperature and sonicated to clarify the solution.

*Resveratrol saturation studies*

(*E*)-Resveratrol saturation studies over the concentration range of 0-2 mM were conducted for both MIPs and their NIP counterparts using a constant amount of polymer (30 mg). The NIP response was used to determine the extent of non-specific binding resulting from interactions with the cross-linker and randomly dispersed functional monomer. The polymers were incubated with mixing at 40 rpm for 18 hours in 1.5 mL of acetonitrile containing resveratrol and then centrifuged at 13000 rpm for 15 minutes to pellet the polymers. An aliquot (200 μL) of the supernatant was removed and analysed by RP-HPLC with UV detection at 321 nm, and the concentration of unbound (*E*)-resveratrol was determined from a linear 5 point calibration curve. This concentration was subtracted from the total analyte solution concentration to derive the amount of analyte bound **(B)** expressed as μmol/g polymer.

*Static selectivity studies*

Selectivity studies were conducted for both MIPs and NIPs using a constant amount of polymer (30 mg). The polymers were separately incubated with either resveratrol or the structurally similar analogues 3,5-dihydroxy-*N*-(4-hydroxyphenyl)benzamide **2,** catechin **3** and (E)-piceid **4.** Binding of each of these compounds was evaluated as described above.

*MISPE of peanut meal extract*

Preliminary MISPE studies were conducted using $MIP_{RES}$ or $MIP_{AMIDE}$ (250 mg) to investigate their ability to selectively retain and enrich resveratrol and other polyphenols from the peanut meal extract. A solution of the peanut meal extract (25 mL) was applied to each column and the breakthrough fractions were collected under vacuum and recycled back through the columns to maximize the interaction between the stationary phase and the polyphenolic components within the peanut meal extract. Each column was then washed with $EtOH/H_2O$ (1:1 v/v, 3 column volumes, 6 mL), then with $H_2O$ (10 mL) to remove unbound and/or weakly bound water-soluble components of the extract. A selective clean up step with aqueous acetone (acetone/$H_2O$, 1:1 v/v, 2 mL) was used to disrupt non-specific binding. An acidic elution step with 10 % AcOH in MeOH (10 $\times$ 1 mL) was then used to desorb and remove the specifically bound compounds of interest from each polymer. The elution fractions were combined, then evaporated to dryness and reconstituted in 1.0 mL in $EtOH/H_2O$ (1:1 v/v). These concentrated eluates were clarified by centrifugation and the supernatant removed for RP-HPLC analysis.

*Tandem MISPE of peanut meal extract*

Separate $MIP_{RES}$ and $MIP_{AMIDE}$ columns (1000 mg each) were pre-conditioned with $EtOH/H_2O$ (1:1 v/v) containing 1% AcOH (15 mL). Peanut meal extract (100 mL) was loaded onto the first of two MIP columns in series ($MIP_{RES}$) as illustrated in **Figure 21A.** The flowthrough from the $MIP_{RES}$ column was then loaded onto the $MIP_{AMIDE}$ column and the flowthrough from $MIP_{AMIDE}$ was collected for further investigations. The $MIP_{RES}$ column was then washed with 100 mL of $EtOH/H_2O$ (1:1 v/v) containing 1 % AcOH and the wash fraction loaded onto $MIP_{AMIDE}$. Finally, both MIP columns were separately eluted using 3 $\times$ 10 mL of 10 % AcOH in MeOH and the eluates were collected in 2 mL fractions **(Figure 21B).** Samples of each of the flowthroughs and wash fractions were retained for analysis.

Prior to the second cycle **(Figure 21C),** the $MIP_{RES}$ and $MIP_{AMIDE}$ columns were reconditioned with $EtOH/H_2O$ (1:1 v/v) containing 0.1 % AcOH and then the combined resveratrol-depleted extract and wash fractions were reloaded onto these columns in series as described above in order to isolate any remaining polyphenolic compounds. $MIP_{RES}$ column was then washed with 25 mL of $EtOH/H_2O$ (1:1 v/v) containing 1 % AcOH and the wash fraction was loaded onto the $MIP_{AMIDE}$ column and eluted using $EtOH_2O$ (1:1 v/v) containing 1 % AcOH.

The processed extract from two cycles of tandem MISPE was subsequently reloaded onto a larger column of $MIP_{RES}$ (5 g) to extract any remaining polyphenolic compounds in the largest quantity possible. This column was preconditioned with MeOH containing 10 % AcOH (3 column volumes), MeOH (3 column volumes) and 1 % AcOH in $EtOH/H_2O$ (1:1, v/v) (3 column volumes). Remaining processed peanut meal extract (comprising the flowthrough and washes from first MISPE application described above) was applied (100 mL) and the subsequent flowthrough collected. The column was then eluted with 1 % AcOH in $EtOH/H_2O$ (1:1, v/v) (50 mL).

## RESULTS & DISCUSSION

*Molecularly imprinted polymers*

Molecularly imprinted polymers templated with either resveratrol **1** or 3,5-dihydroxy-*N*-(4-hydroxyphenyl)benzamide **2** were prepared using the functional monomer 4VP. The use of 4VP provided the opportunity for hydrogen bonding or ionic interactions between the electron rich pyridine nitrogen and the acidic phenolic groups in both of these template compounds. Aromatic $\pi$-$\pi$ interactions may also participate in stabilising the pre-polymerisation complex which is subsequently 'frozen' during polymerization in the presence of the cross-linker EGDMA. The binding characteristics and performance of $MIP_{RES}$ is reported above describing the preparation of an *(E)*-resveratrol selective MIP that is capable of the specific recognition of (*E*)-resveratrol present in a complex mixture comprising multiple structurally related analogues and other polyphenolics.

3,5-Dihydroxy-*N*-(4-hydroxyphenyl)benzamide **2** was synthesised as a template for preparing $MIP_{AMIDE}$ that should generate "pseudo" (*E*)-resveratrol cavities within an imprinted polymer that are capable of binding not only the template molecule, but also (*E*)-resveratrol and potentially other structurally related compounds that are present in complex food waste matrices.

(*E*)-resveratrol saturation studies were conducted to determine the (*E*)-resveratrol binding affinity of $MIP_{AMIDE}$, **(Figure 21).** Binding results for solutions containing *(E)*-resveratrol above 2 mM, which are beyond the naturally occurring range of this compound, were not reproducible and are not shown. $MIP_{AMIDE}$ displayed *(E)*-resveratrol binding capacity of approximately 15 μmol/g as determined from the asymptote of the static binding isotherm **(Figure 21A),** which is about 20% lower than that obtained with the corresponding (*E*)-resveratrol-imprinted polymer. The extent of non-specific binding of (*E*)-resveratrol to $NIP_{AMIDE}$ clearly shows that that the 3,5-Dihydroxy-*N*-(4-hydroxyphenyl)benzamide-imprinted cavities are responsible for the increased binding capacity of $MIP_{AMIDE}$. The selective binding capacity (expressed as MIP-NIP) for $MIP_{AMIDE}$ (5-7 μmol/g, **Figure 21B)** was however, considerably less than that observed for $MIP_{RES}$ (9-11μmol/g). This observation may be attributed to the 3-dimensional structural differences between the respective MIP cavities. These MIPs were generated using different template molecules, thus determining the complementary spatial orientation of the immobilized functional groups of the monomers within the respective MIP cavities and thereby maximizing the opportunity for interaction with their binding partners upon template rebinding. Hence, preparing $MIP_{AMIDE}$ with the structurally similar resveratrol analogue 3,5-Dihydroxy-*N*-(4-hydroxyphenyl)benzamide **2** as the templating molecule produced molecular cavities having subtle differences in conformation with respect to the relative orientation of the functional monomers that reduced their capability to form strong interactions with the target molecule, (*E*)-resveratrol.

The cross-reactivity of both $MIP_{RES}$ and $MIP_{AMIDE}$ was evaluated via binding site interrogation using several structurally related polyphenols (**Figure** 22). The order of selective recognition (MIP-NIP) of $MIP_{RES}$ was (*E*)-resveratrol **1** > amide **2** > catechin **3** > (*E*)-piceid **4,** while the selective recognition of $MIP_{AMIDE}$ was amide **2** ≥ *(E)*-resveratrol **1** > catechin **3** > (*E*)-piceid **4.** $MIP_{AMIDE}$ displayed a high cross-reactivity towards (*E*)-resveratrol, displaying comparable selective binding for both this compound and the templating molecule 3,5-Dihydroxy-*N*-(4-hydroxyphenyl)benzamide 2. Both $MIP_{RES}$ and $MIP_{AMIDE}$ demonstrated similar binding capacities for catechin, albeit with reduced selectivity, that are comparable to binding of the respective template molecules. The presence of an increased number of possible H-bonding groups on the molecule resulted in a larger number of non-selective associations, thus leading to greater affinity by the NIPs, thereby decreasing specific recognition of catechin compared to that for the respective template molecules. $MIP_{RES}$ and $MIP_{AMIDE}$ displayed essentially no recognition for the glucosylated (*E*)-resveratrol molecule (*E*)-piceid, a result that is presumably due to the bulky glucose substituent preventing access to the MIP binding cavities.

*MISPE of peanut meal extract*

Peanut meal or peanut press is generated during peanut oil preparation and constitutes the remains of the peanut and husk after pressing. Peanuts contain variable amounts of (*E*)-resveratrol (0.02 - 1.79 μg/g) (*11*), amounts which exceed what we have observed in grape marc. Accordingly, binding of components present in a peanut meal extract, a complex mixture of bioactive polyphenols including (*E*)-resveratrol and catechin-based oligomers, was evaluated using $MIP_{res}$ and $MIP_{AMIDE}$. A liquid peanut meal extract was prepared and separately applied to either $MIP_{RES}$ or $MIP_{AMIDE}$ columns or the respective NIP controls. RP-HPLC chromatograms of the peanut meal extract and subsequent MIP eluates are shown in **Figure 23.** $MIP_{RES}$ clearly significantly cleaned up the peanut meal extract and enriched (*E*)-resveratrol, which elutes at $R_t$ = 12.2 min, and several unidentified compounds **(Figure 23B).** In contrast, $MIP_{AMIDE}$ displayed preferential selectively for the unidentified compound(s) that eluted at $R_t$ = 9.7 min, whilst displaying negligible retention of (*E*)-resveratrol, which is present in the eluate at approximately similar levels as the peanut meal extract **(Figure 23C).** Although $MIP_{AMIDE}$ displays affinity for (*E*)-resveratrol as described above, the apparent lack of affinity for this molecule from complex mixtures may be a consequence of either higher affinity for other molecules that are present in the mixture or from Law of Mass Action effects arising from high abundance molecules having variable affinities for the MIP cavity. The level of non-specific binding of (*E*)-resveratrol by the NIP control column **(Figure 23D)** was only marginally elevated above that present in the extract and also that bound by $MIP_{AMIDE}$ although in the later case it is possible that the presence of the unknown effectively saturated the binding surface..

The eluate from $MIP_{RES}$ **(Figure 23B)** was analysed using tandem liquid chromatography-ESI-mass spectrometry (LC-ESI-MS) in positive ion mode, which confirmed that the peak at $R_t$ = 12.2 minutes consisted solely of (*E*)-resveratrol by comparison to an (*E*)-resveratrol standard which has a molecular mass of 229 *m*/*z* as the singly charged ion ($[M+H]^+$). LC-ESI-MS was also used to examine the unknown compound(s) present in the $MIP_{AMIDE}$ eluate at $R_t$ = 9.7 minutes, which revealed that this peak comprises a compound having a molecular mass of 577 *m*/*z* ($[M+H]^+$) that is consistent with that of A-type procyanidins. Low molecular weight catechin oligomers, of which the A-type procyanidins predominate, are known antioxidants that are present in peanut skin in quantities approaching 9.5 % by mass (*12*).

The concept of employing multiple MIPs in a tandem separation and isolation strategy, whereby multiple bioactive components may be captured and isolated from a single source, was explored as outlined in **Figure 24.** The peanut meal extract was loaded onto two MIPs in sequence, $MIP_{RES}$ followed by $MIP_{AMIDE}$, to isolate the primary molecule for which the MIPs exhibit the greatest selectivity. Re-loading of the depleted peanut meal extract onto these same eluted and washed tandem MIPs, served to isolate other residual, but equally valuable, polyphenol compounds.

Typical RP-HPLC chromatograms from the tandem MISPE treatment of peanut meal extract are shown in

**Figure 25.** The chromatogram of the peanut meal extract **(Figure 25A)** shows a small peak for (*E*)-resveratrol at $R_t$ = 17 minutes, corresponding to 64 μg total (*E*)-resveratrol. A peak corresponding to (*E*)-resveratrol was similarly observed in the elutates from $MIP_{RES}$ **(Figure 25B)** and then from $MIP_{AMIDE}$ **(Figure 25C).** These results clearly illustrate the refining potential of $MIP_{AMIDE}$ to also specifically capture (*E*)-resveratrol while excluding many of the non-bound compounds that remain in the flowthrough from $MIP_{RES}$.

The tandem combination of $MIP_{RES}$ and $MIP_{AMIDE}$ enabled the near quantitative (96 %) recovery of resveratrol as summarised in **Table 10.** This result clearly signifies that both MIP columns are capable of selective retention and enrichment of (*E*)-resveratrol with almost complete recovery from the crude feed stock.

**Table 10.** Recovery of (*E*)-resveratrol from peanut meal extract by tandem MISPE in different fractions of the eluant.

| **Eluant Fractions (mL)** | **Resveratrol Recovery (□g)[1]** | | **Total Recovery** | |
|---|---|---|---|---|
| | **$MIP_{RES}$** | **$MIP_{AMIDE}$** | **□g** | **%** |
| 1 | 1.61 | 0.78 | 2.39 | 3.72 |
| 2 | 5.16 | 4.35 | 9.51 | 14.83 |
| 3 | 5.47 | 5.14 | 10.61 | 16.54 |
| 4 | 4.40 | 5.78 | 10.18 | 15.88 |
| 5 | 3.01 | 3.68 | 6.69 | 10.43 |
| 6 | 2.34 | 3.01 | 5.35 | 8.34 |
| 7 | 2.43 | 2.38 | 4.81 | 7.50 |
| 8 | 2.26 | 2.09 | 4.35 | 6.78 |
| 9 | 1.91 | 1.57 | 3.48 | 5.43 |
| 10 | 1.80 | 0.69 | 2.49 | 3.88 |
| 11 | 1.39 | 0.46 | 1.85 | 2.88 |
| 12 | *ND*[2] | *ND* | *ND* | *ND* |
| Total | 31.81 | 29.93 | 61.71 | 96.21 |

[1] Recovery determined for 1 mL reconstituted samples
[2] *ND* = Not detectable.

Surprisingly, the $MIP_{AMIDE}$ column also displayed preferential selectivity towards (*E*)-resveratrol over the likely type A procyanidins *($R_t$* = 15 minutes, 577 *m/z* $[M+H]^+$), a result in contrast to observations that showed that $MIP_{AMIDE}$ preferentially bound the unknown compound compared to (*E*)-resveratrol **(Figure 23C).** However, the tandem MISPE experiment was performed with a substantially larger peanut meal extract volume than that used in small-scale experiments. Therefore, it is apparent that $MIP_{AMIDE}$ has selectively retained the larger quantity of (*E*)-resveratrol present that is in this peanut meal extract to the exclusion of procyanidin and that the $MIP_{AMIDE}$ binding sites are effectively saturated with (*E*)-resveratrol, to the exclusion of other compounds which may be competing for these $MIP_{AMIDE}$ binding sites. Our observation that $MIP_{AMIDE}$ is capable of binding both (*E*)-resveratrol and procyanidin in small scale experiments clearly reflects the fact that saturation of available $MIP_{AMIDE}$ binding sites was not achieved by the small quantities of available (*E*)-resveratrol thereby allowing procyanidin to bind to the remaining unoccupied $MIP_{AMIDE}$ binding sites. Further, that procyanidin did not bind at all in the large scale experiment signifies that $MIP_{AMIDE}$ exhibits lower affinity, specificity and selectivity for procyanidin than for (*E*)-resveratrol.

Since the tandem MISPE columns had selectively removed most of the (*E*)-resveratrol (96%) from the initial feed stock, it was anticipated that with diminished competition for available MIP binding sites both $MIP_{RES}$ and $MIP_{AMIDE}$ would selectively bind the procyanidin remaining in the resveratrol-deplected peanut meal extract. Consequently, the flowthrough and wash fractions from the processed peanut meal extract were combined and re-applied to the reconditioned tandem $MIP_{RES}$ and $MIP_{AMIDE}$ columns. The RP-HPLC chromatograms of the extracts recovered from the $MIP_{RES}$ and $MIP_{AMIDE}$ columns, obtained using the same conditions as above, showed that (*E*)-resveratrol did not bind or elute from either column **(Figure 26).** However, as expected, the peak corresponding to procyanidin ($R_t$ = 15 min) was clearly present in the chromatograms from both MIP columns. Both $MIP_{RES}$ and $MIP_{AMIDE}$ retained similar amounts of procyanidin **(Figure 26B and 26C).** These results confirm that (i) all of the measurable (*E*)-resveratrol had been successfully removed from the extract by the first round of tandem MISPE treatment of the peanut meal extract and (ii) that both $MIP_{RES}$ and $MIP_{AMIDE}$ could successfully concentrate the A-type procyanidins upon the depletion of (*E*)-resveratrol from this extract.

**CONCLUSIONS**

The findings reported here demonstrate the ability of molecularly imprinted polymers (MIPs) to selectively fractionate a mixture comprising polyphenols such as resveratrol and A-type procyanidins from peanut meal extract without the requirement for extensive sample pre-treatment. These MIPs may also be utilized in such a manner that more than one polyphenol may be separately isolated or enriched from the same feed stock with a rapid tandem MISPE (*i.e.,* MIPs in series) approach.

**H. The use of "teabag" MIPs in separating components of a complex feedstock**

This technology represents a new approach for the practical application of MIPs. Extraction of bioactives from processing waste streams (e.g., peanut by-products, winery grape seeds and skins, apple juice production wastes) may be a considerable logistic exercise, due to the large quantities of material, from geographically diverse locations, to isolate and return much smaller amounts of target bioactives. Therefore, it may be more advantageous to consider initial separation or pre-concentration of bulk-scale materials at the site of waste generation, which would result in material that is more easily handled and combined for final separations and purifications at a single dedicated processing facility. Alternatively, extraction may be performed at, or close to, the site of waste generation using low technology applications for the initial process of extraction of valuable materials from waste streams. An exemplar methodology could be based on the use of "tea bags" filled with MIPs for isolating target molecules from the feed stock.

"Teabags" for use in these investigations have been made from cotton-based materials, Gilson® 63$\mu$m sieve mesh and Sigma Aldrich dialysis tubing cellulose membrane (12 kDa MWCO). The bags were held in an electroplated metal tea infuser to protect them from physical damage during stirring. They are robust for periods of several days and tolerate soaking in mixtures of ethanol, water and acetic acid.

Binding experiments using MIPs in a teabag were conducted. The MIPs used in the resveratrol binding experiments were MIP8 (as numbered in the patent application), MIPE, MIP AMIDE, and MIPIMINE as set out in the Examples above. _Use of "Teabag" MIPs in Static Binding Systems for Phytosterols:_ MIPs were templated with ergosterol, campesterol, cholesterol, stigmasterol, cholesteryl ferrulate, ferrulic acid and coumarin as described above. MIPs were packed in teabag polymer bags, and then placed in the crude plant extract for 18 hours. The teabag was subsequently removed from the mixture and transferred to a solution of 10% acetic acid in MeOH for 4 hours.

The teabag was removed and the solution was evaporated to dryness; the resulting solid was reconstituted to approx 0.5mM in 20% ACN/MeOH, and analyzed by HPLC and ESI/ MS. The performance MIPs were evaluated using extracts from avocado oil, sesame seed oil, wheat oil, grape seed oil, $\beta$ -sitosterol vitamin supplement, and a green tea extract, derived from Lipton Green Tea™. The individual components extracted were identified. Several different polymers could be place singularly or severally in individual bags to simultaneously and concurrent extract the maximum amount and number of compounds of interest from the extract solutions. Recycling of individual MIPs was also established and the results demonstrated the reusability of these MIPs without significant deterioration in performance. The peaks in the $^{13}$C NMR spectrum allow for an analysis of the various captured and isolated phytosterols. For example, with the bound fraction eluted from a loaded campesterol-templated MIP, the peaks in the $^{13}$C NMR spectrum reveal only pure campesterol confirming that campesterol only has been specifically and selectively absorbed by this MIP. On the other hand with the cholesterol-templated MIP, the stigmasterol present in the crude extract was significantly bound suggesting that cholesterol can be used as a biomimetic template (in a manner analogous to the green resveratrol - the imine analogue of resveratrol), for molecular imprinting of more expensive and difficult to produce polymers.

_Use of "Teabag " MIPs in Static Binding Systems for Resveratrol_

A corresponding experiment was carried out for binding of resveratrol to "teabag" MIPs using $MIP_8$ (as numbered in the patent application), MIPE, MIP AMIDE, and MIPiMiNE using the following feedstock examples: peanut skin and mash by-products, winery grape seeds and skins, apple juice production wastes and a green tea extract, derived from Lipton Green Tea™.

**I Sequential application of MIPs**

Figure 27 provides an example of a sequential application of MIPs for the isolation of multiple targets from a feed extract.

Throughout this specification the word "comprise", or variations such as "comprises" or "comprising", will be understood to imply the inclusion of a stated element, integer or step, or group of elements, integers or steps, but not the exclusion of any other element, integer or step, or group of elements, integers or steps.

Any discussion of documents, acts, materials, devices, articles or the like which has been included in the present

specification is solely for the purpose of providing a context for the present invention. It is not to be taken as an admission that any or all of these matters form part of the prior art base or were common general knowledge in the field relevant to the present invention as it existed in Australia or elsewhere before the priority date of each claim of this application.

It will be appreciated by persons skilled in the art that numerous variations and/or modifications may be made to the invention as shown in the specific embodiments without departing from the spirit or scope of the invention as broadly described. The present embodiments are, therefore, to be considered in all respects as illustrative and not restrictive.

**Non-Patent References**

1. Xiang, H.-Y.; Zhou, C.-S.; Zhong, S.-A.; Lei, Q.-F., Synthesis of resveratrol imprinted polymer and its application in separation of active ingredient in Polygonum cuspidatum extracts. Yingyong Huaxue 2005, 22, (7), 739-743.

2. Ma, S.; Zhuang, X.; Wang, H.; Liu, H.; Li, J.; Dong, X., Preparation and characterisation of trans-Resveratrol imprinted polymers. Analytical Letters 2007, 40, 321-333.

3. Cao, H.; Xiao, J. B.; Xu, M., Evaluation of new selective molecularly imprinted polymers for the extraction of resveratrol from Polygonum cuspidatum. Macromolecular Research 2006, 14, (3), 324- 330.

4. Ki, C. D.; Oh, C.; Oh, S.-G.; Chang, J. Y., The use of a thermally reversible bond for molecular imprinting of silica spheres. Journal of the American Chemical Society 2002, 124, (50), 14838-14839.

5. Spencer, A., "Selective Preparation of Non-Symmetrically Substituted Divinylbenzenes by Palladium Catalysed Arylations of Alkenes with Bromobenzoic Acid Derivatives", J. Organomet. Chem., 1984, 265, 323-331

6. Romero-Peréz et al6 J. Agric. Food Chem., Vol. 49, No. 1, 2001, 210-215.

7. D. A. Learmonth Bioconjugate Chem., 2003, 14, 262-267

8. Roberts et al, Eur. J. Med. Chem., (1994), 29, 841-854

9. Dolle, R.E.; Kruse, L.I. J. Org. Chem. 1985, 51, 4047-4053

10. Condo Jr, A.M.; Baker, D.C.; Moreau, R.A.; and Hicks, K.B., J. Agric. Food Chem., (2001), 49, 4961-4964, "Improved Method for the Synthesis of trans-Feruloyl-β-sitostanol."


**Claims**

**1.** A molecularly imprinted polymer (MIP) imprinted with (*E*)-5-[(4-hydroxy-phenylimino)-methyl]-benzene-1,3-diol or 3,5-dihydroxy-*N*-(4-hydroxyphenyl)benzamide.

**Patentansprüche**

**1.** Molekular geprägtes Polymer (MIP) geprägt mit (*E*)-5-[(4-Hydroxy-phenylimino)-methyl]-benzol-1,3-diol oder 3,5-Dihydroxy-*N*-(4-Hydroxyphenyl)benzamid.

**Revendications**

**1.** Polymère à empreinte moléculaire (PEM) imprimé avec (E)-5-[(4-hydroxy-phénylimino)-méthyl]-benzène-1,3-diol ou 3,5-dihydroxy-*N*-(4-hydroxyphényl)benzamide.

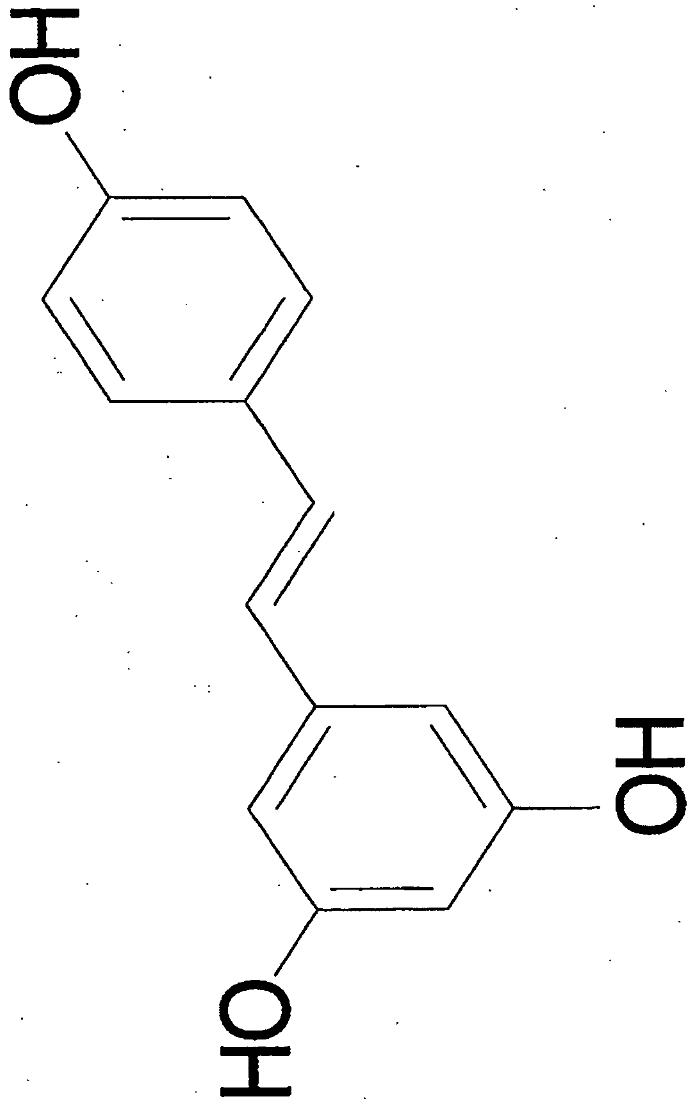

Figure 1A

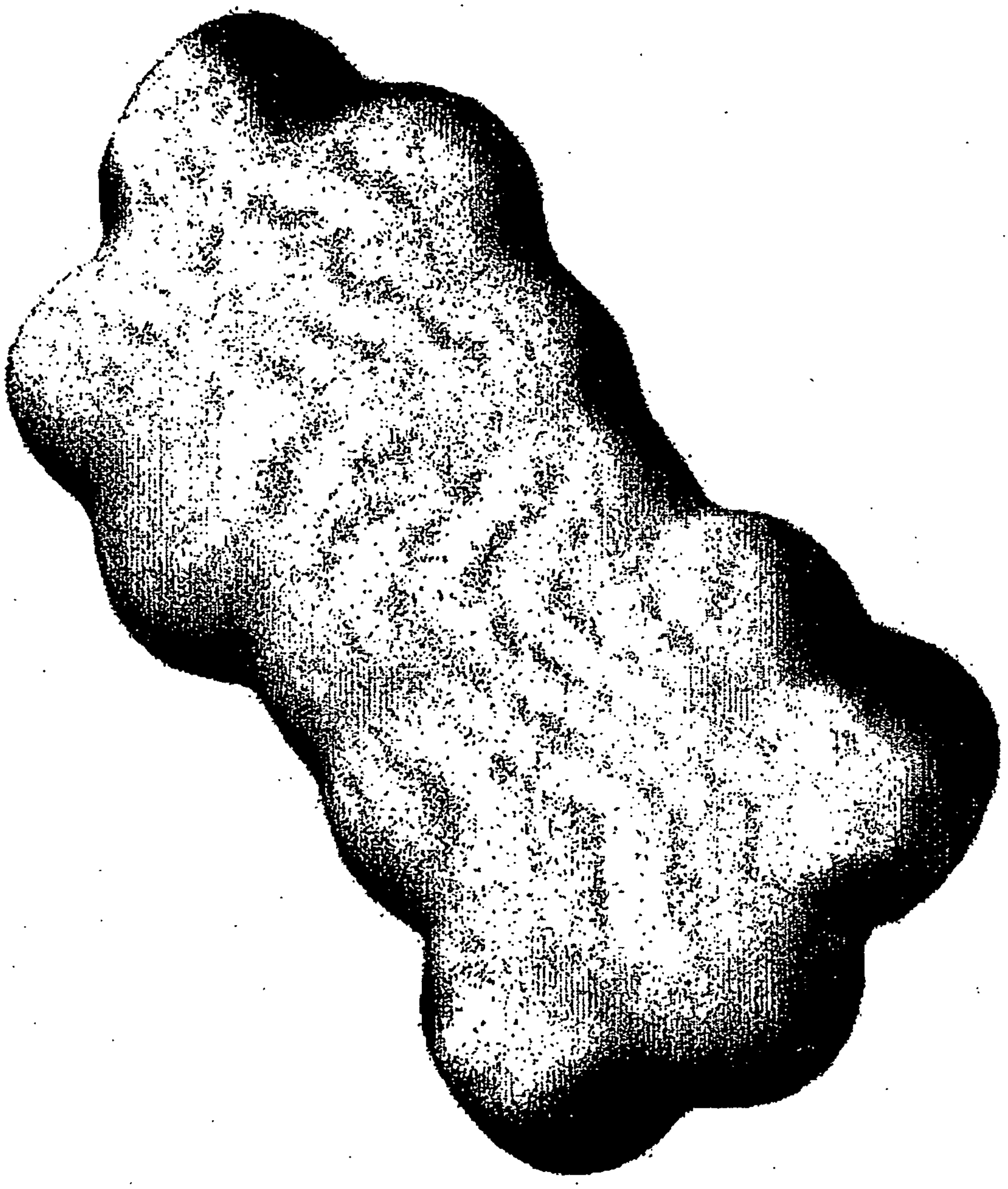

Figure 1B

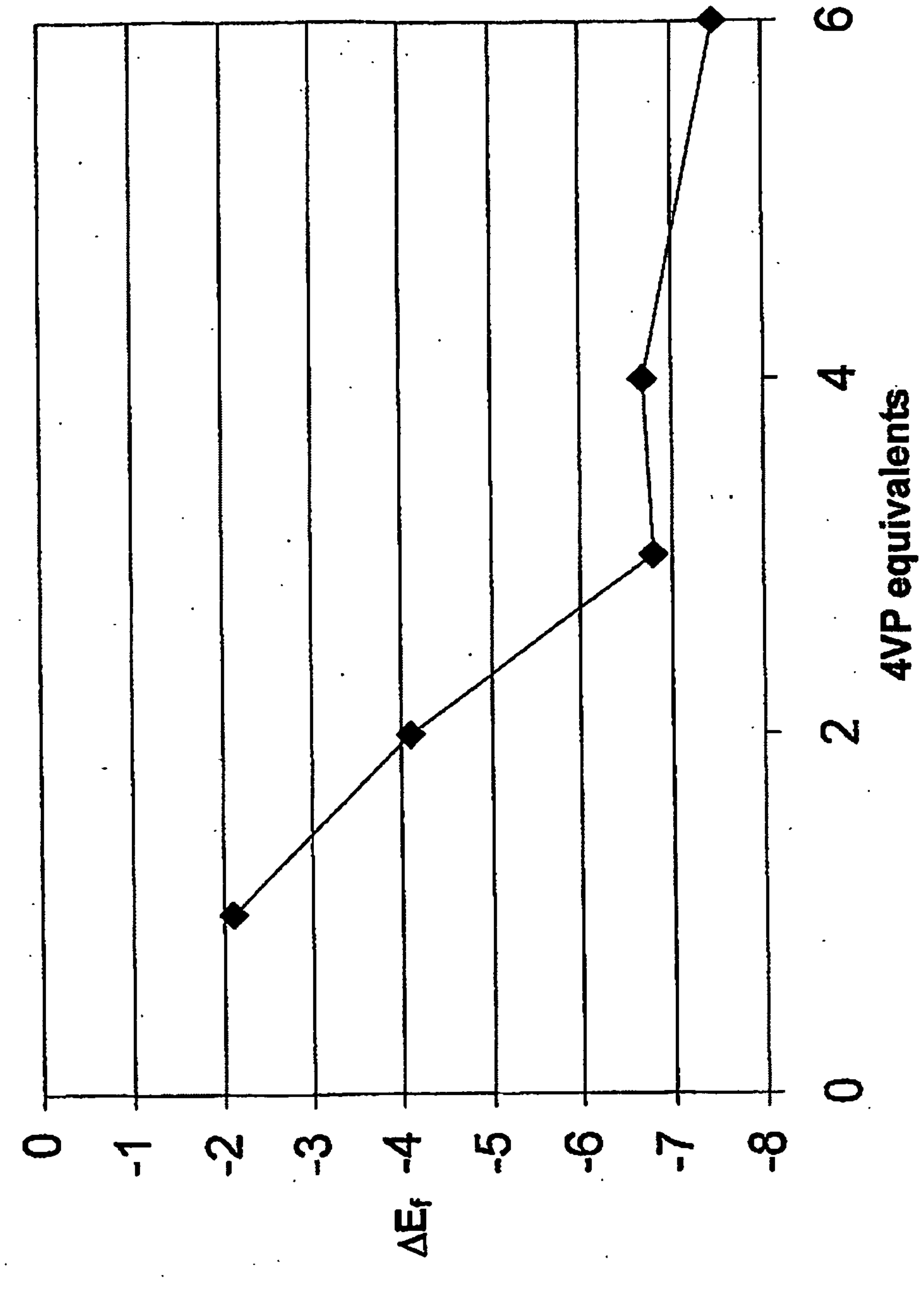


Figure 2

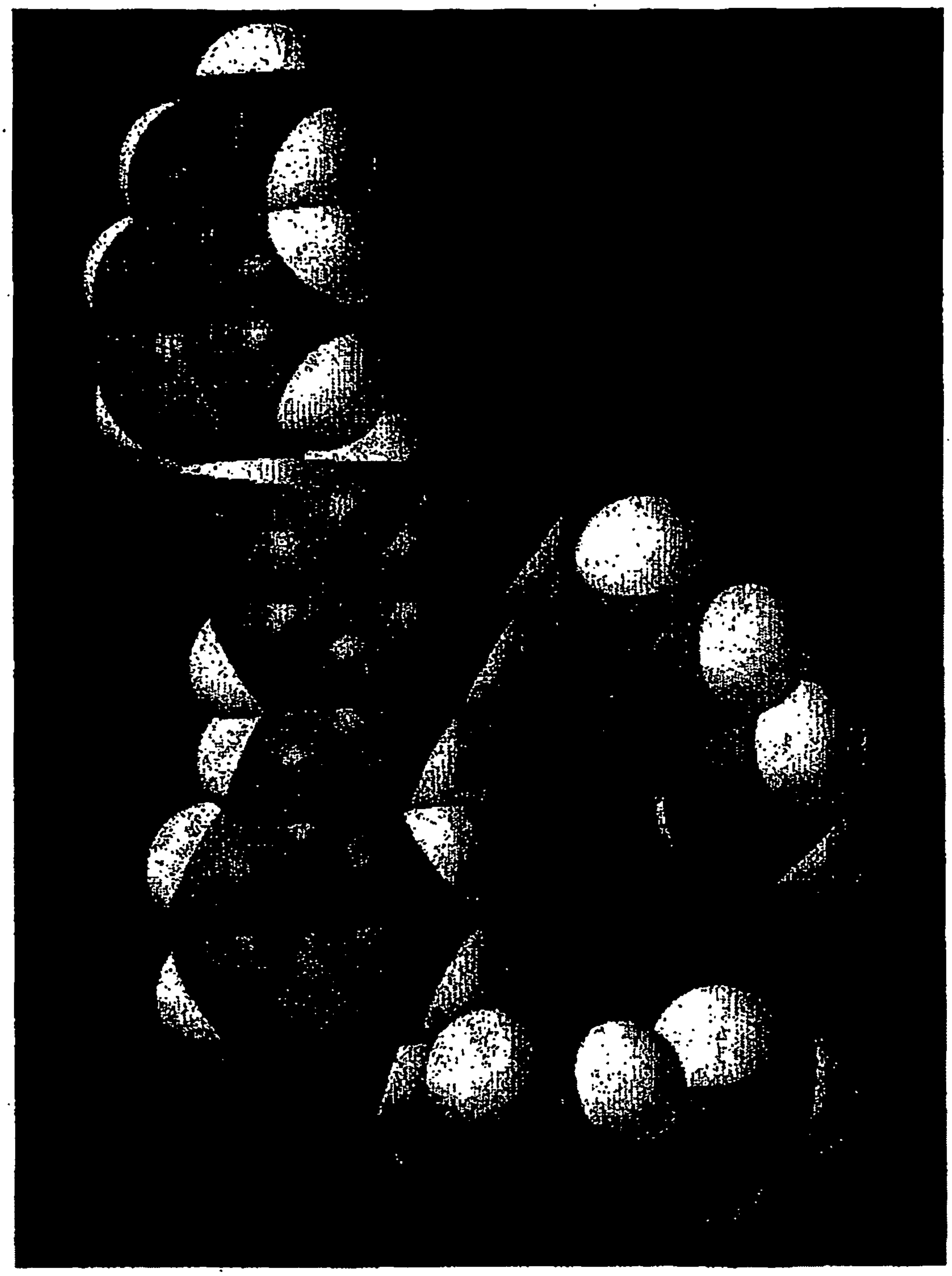

Figure 3

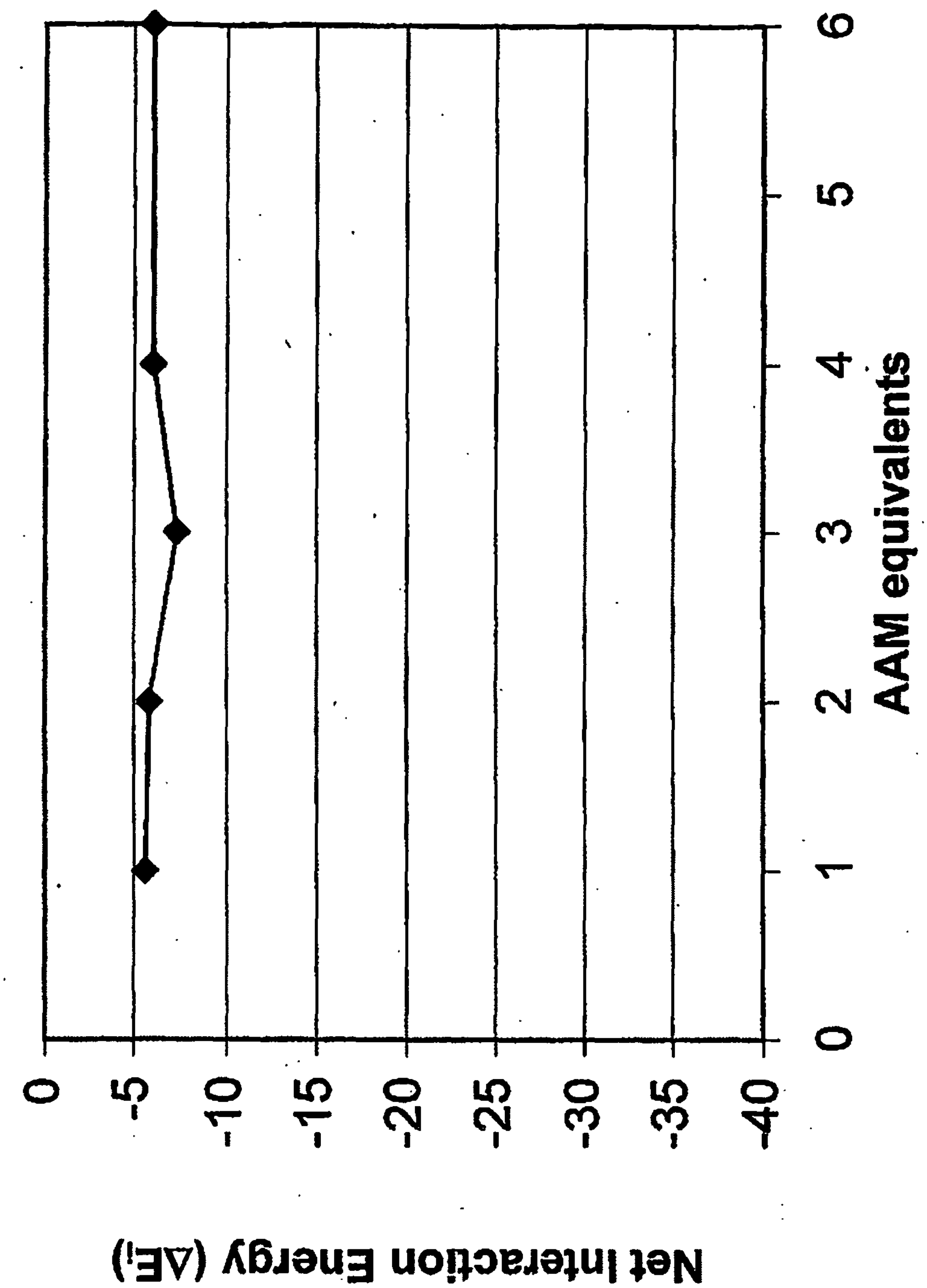
Net Interaction Energy (ΔEi)
0
-5
-10
-15
-20
-25
-30
-35
-40
AAM equivalents
0
1
2
3
4
5
6

Figure 4A

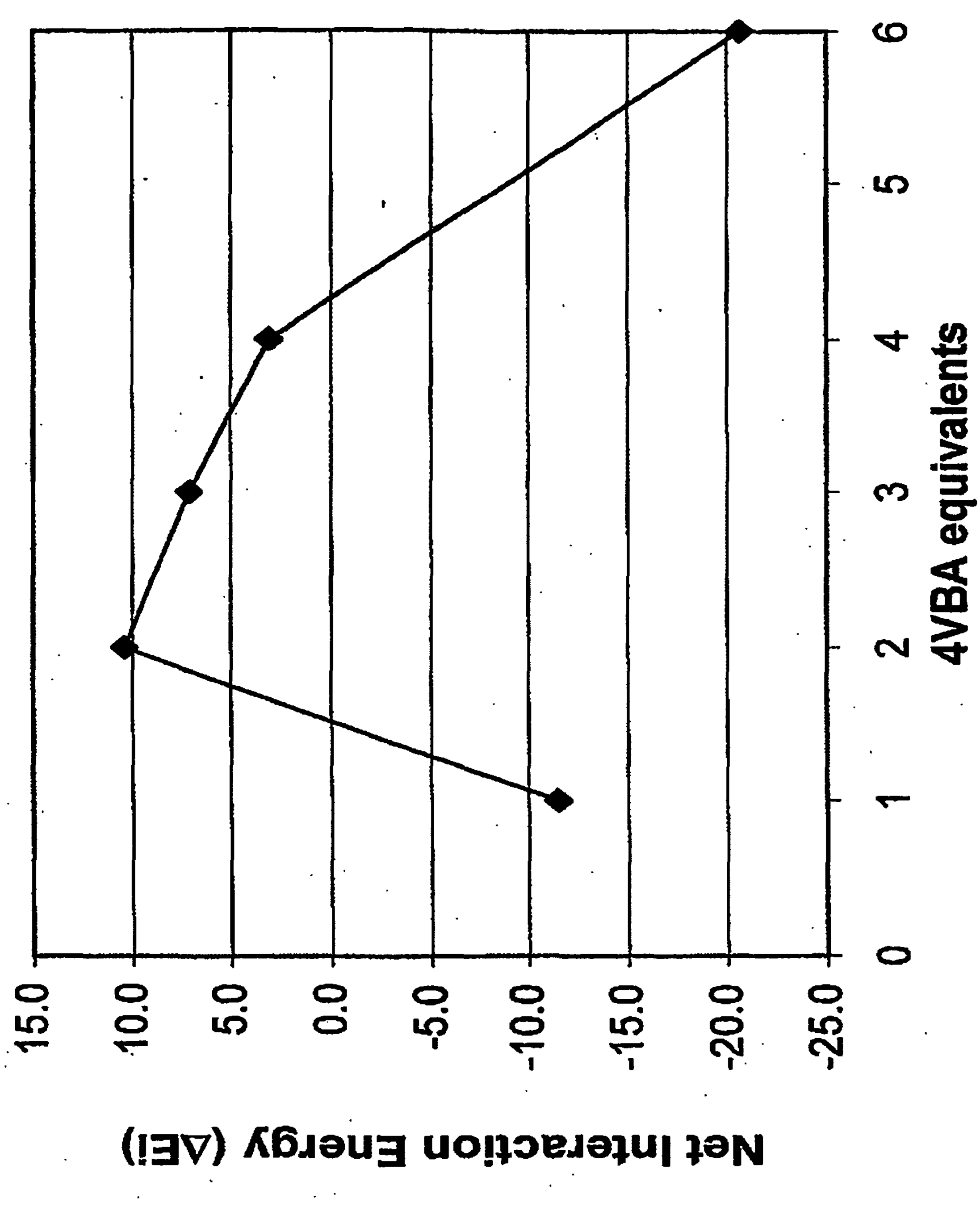
Net Interaction Energy (ΔEi)
15.0
10.0
5.0
0.0
-5.0
-10.0
-15.0
-20.0
-25.0
0
1
2
3
4
5
6
4VBA equivalents

Figure 4B

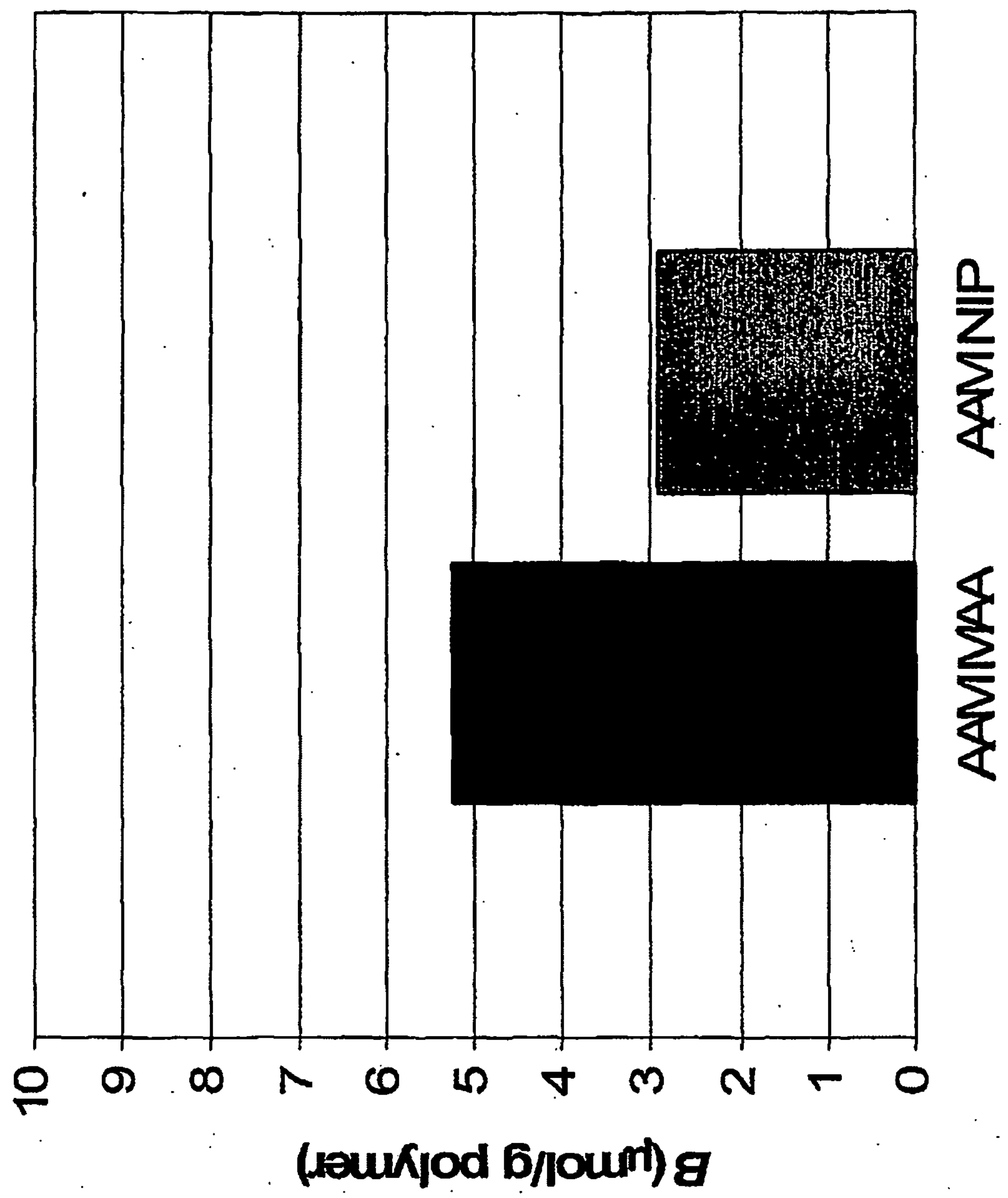


Figure 4C

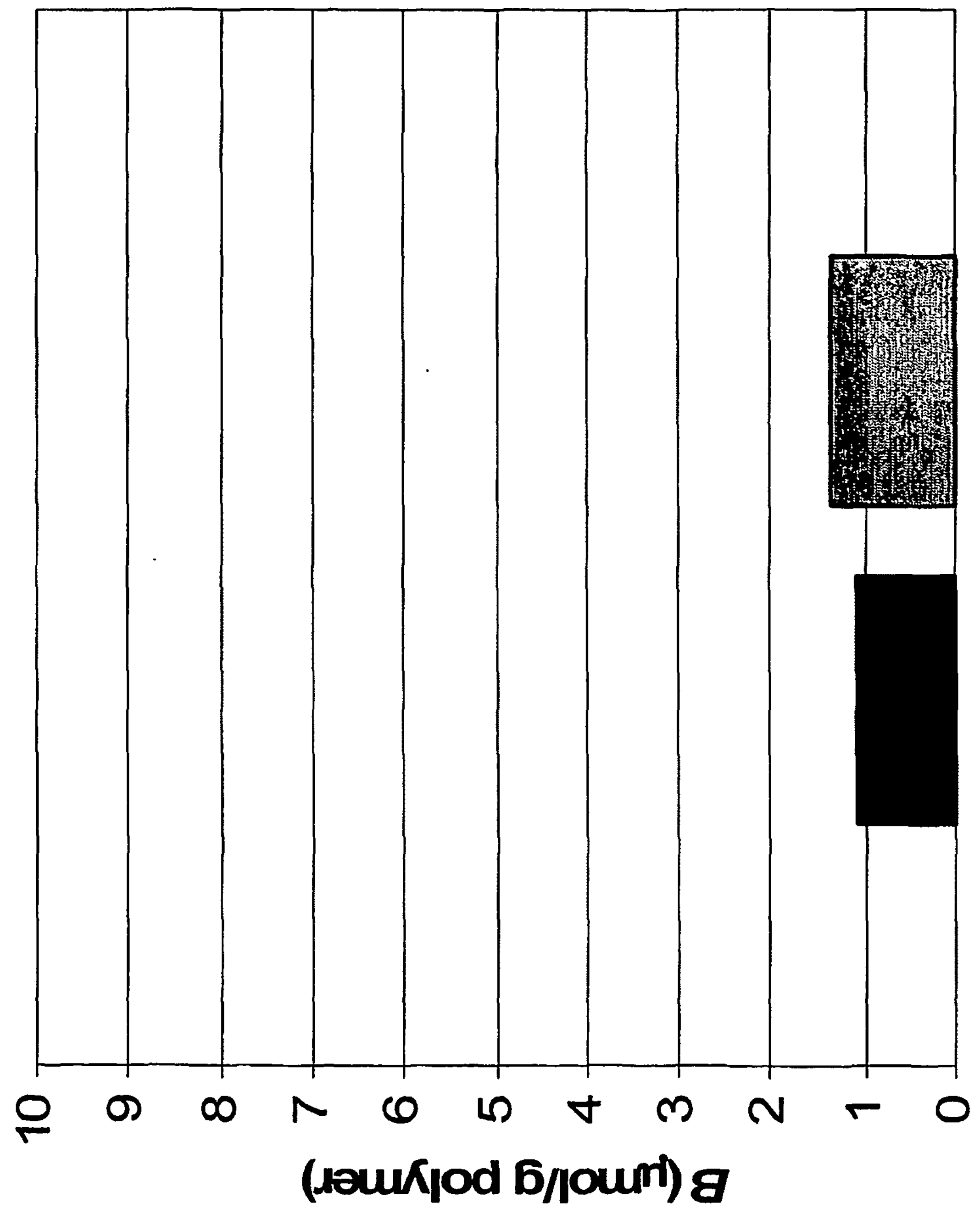


Figure 4D

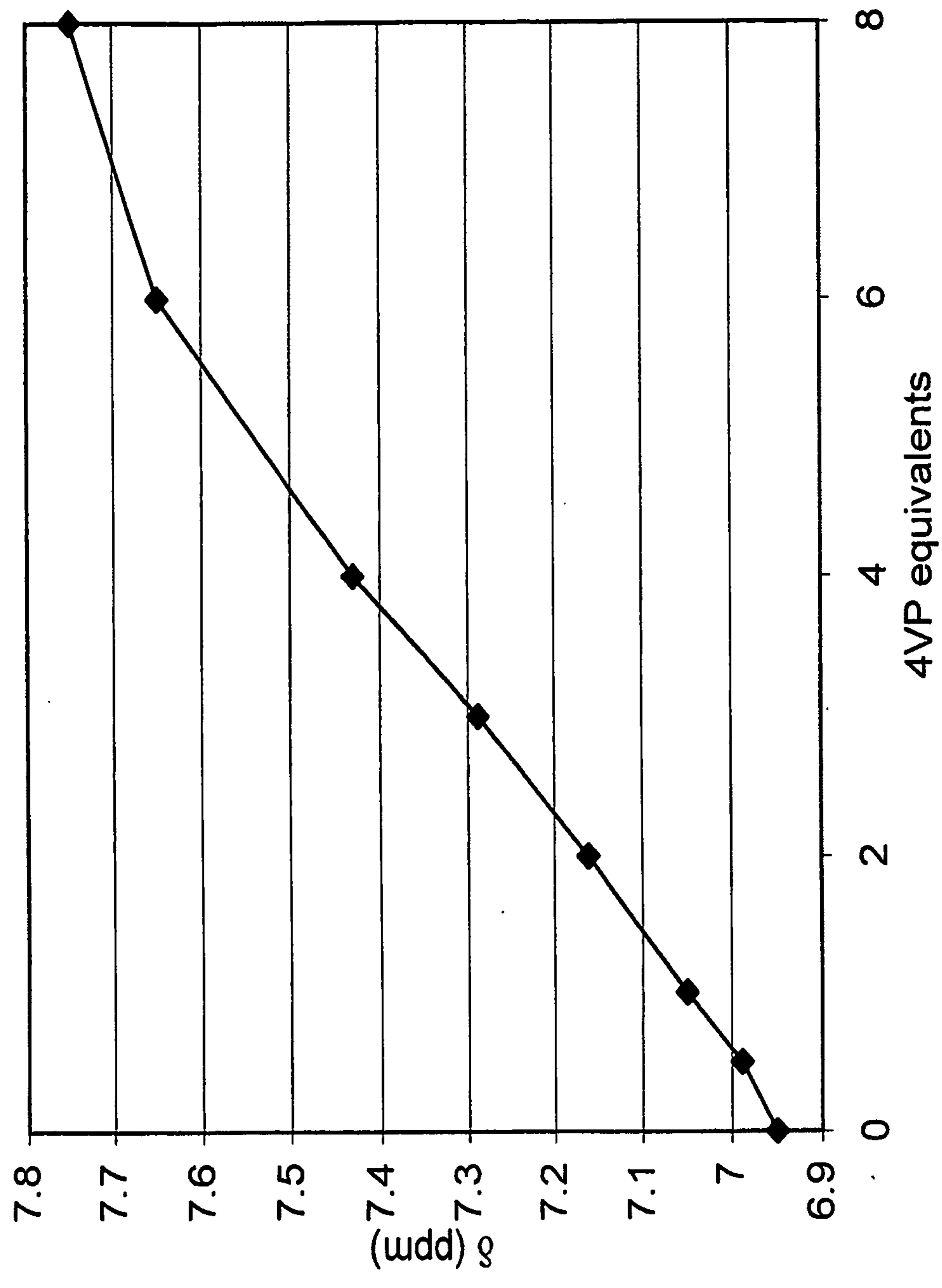


Figure 5

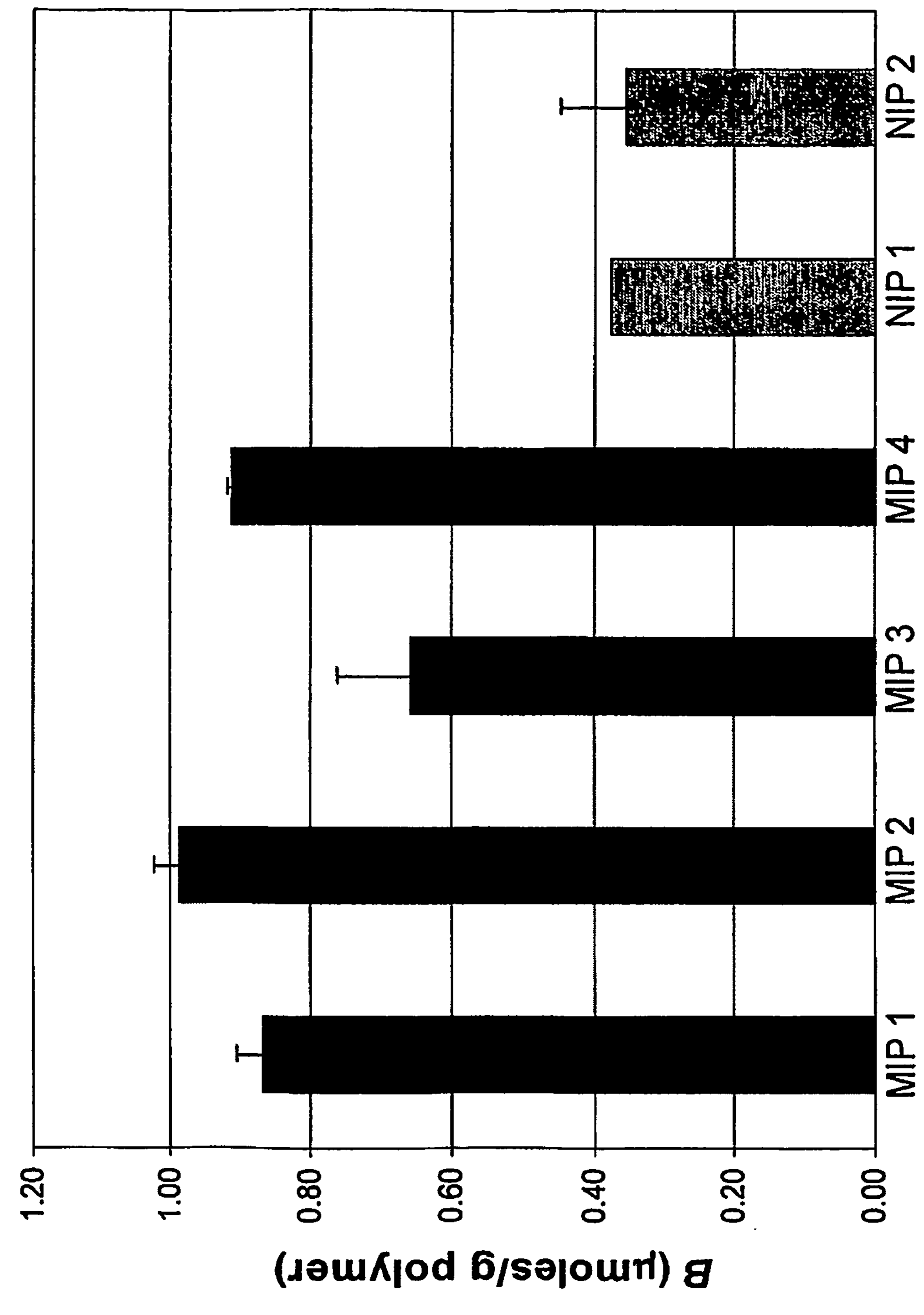
B (μmoles/g polymer)
1.20
1.00
0.80
0.60
0.40
0.20
0.00
MIP 1
MIP 2
MIP 3
MIP 4
NIP 1
NIP 2

Figure 6

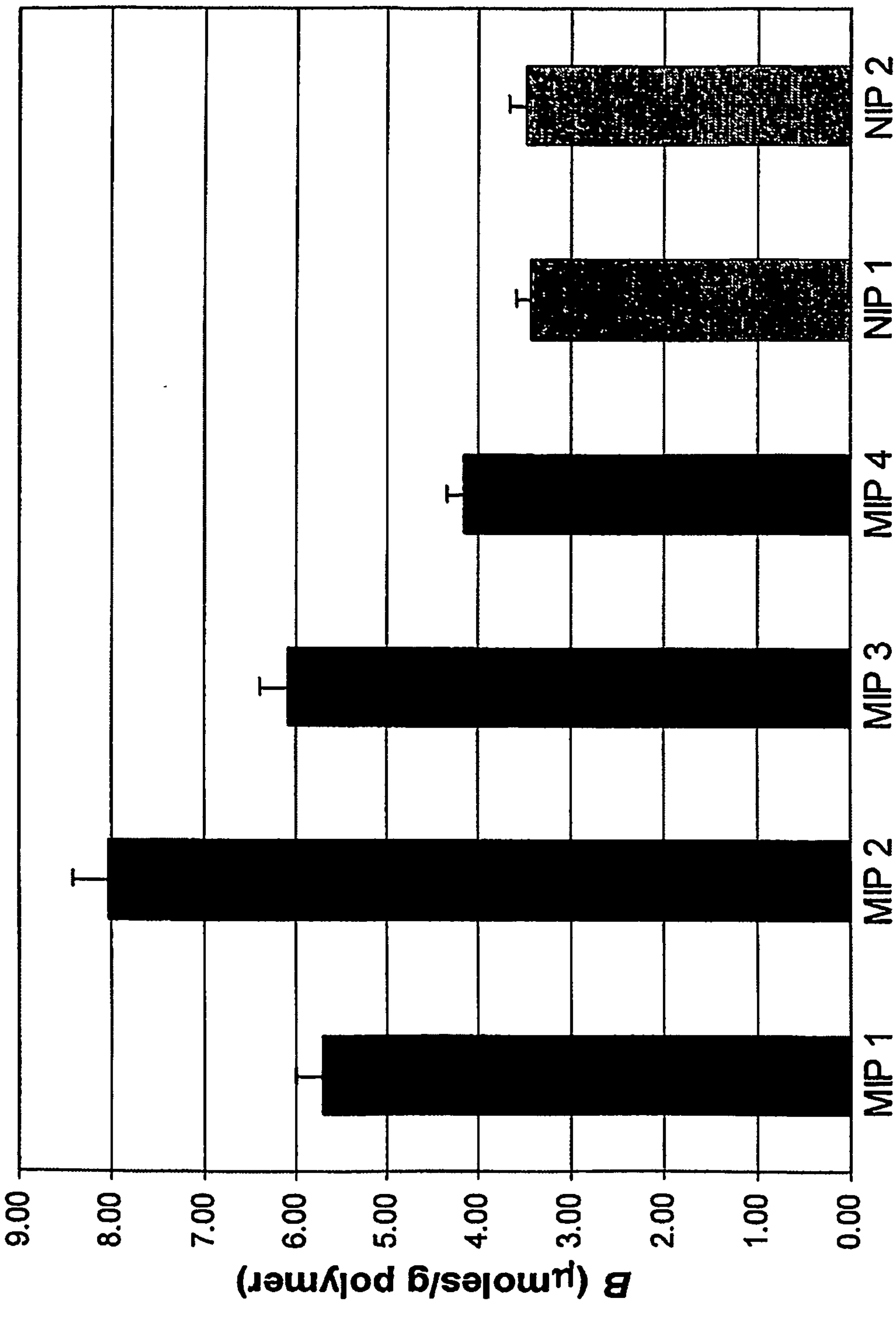
B (μmoles/g polymer)
9.00
8.00
7.00
6.00
5.00
4.00
3.00
2.00
1.00
0.00
MIP 1
MIP 2
MIP 3
MIP 4
NIP 1
NIP 2

Figure 7

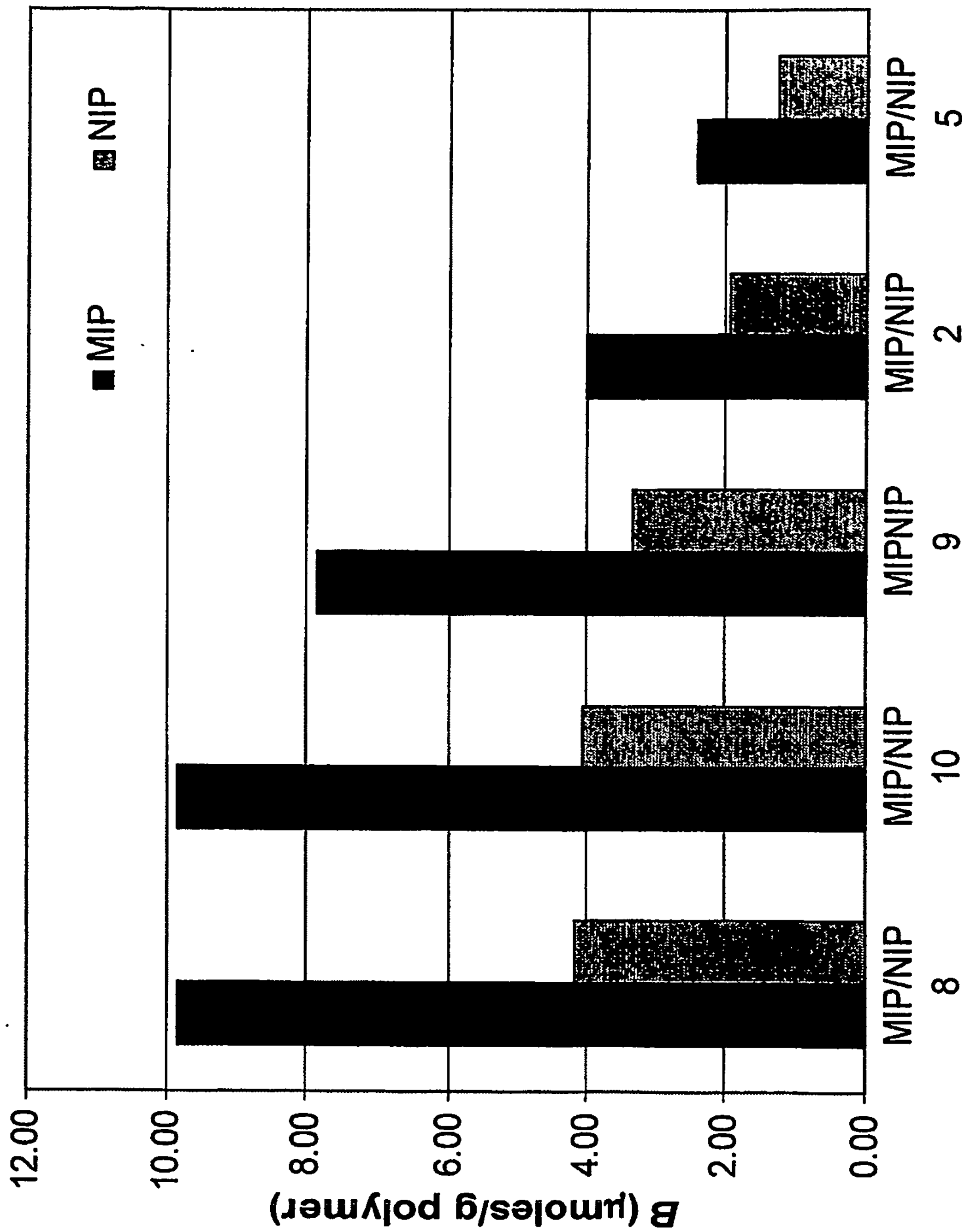

MIP
NIP
12.00
10.00
8.00
6.00
4.00
2.00
0.00
B (μmoles/g polymer)
MIP/NIP 8
MIP/NIP 10
MIPNIP 9
MIP/NIP 2
MIP/NIP 5


Figure 8

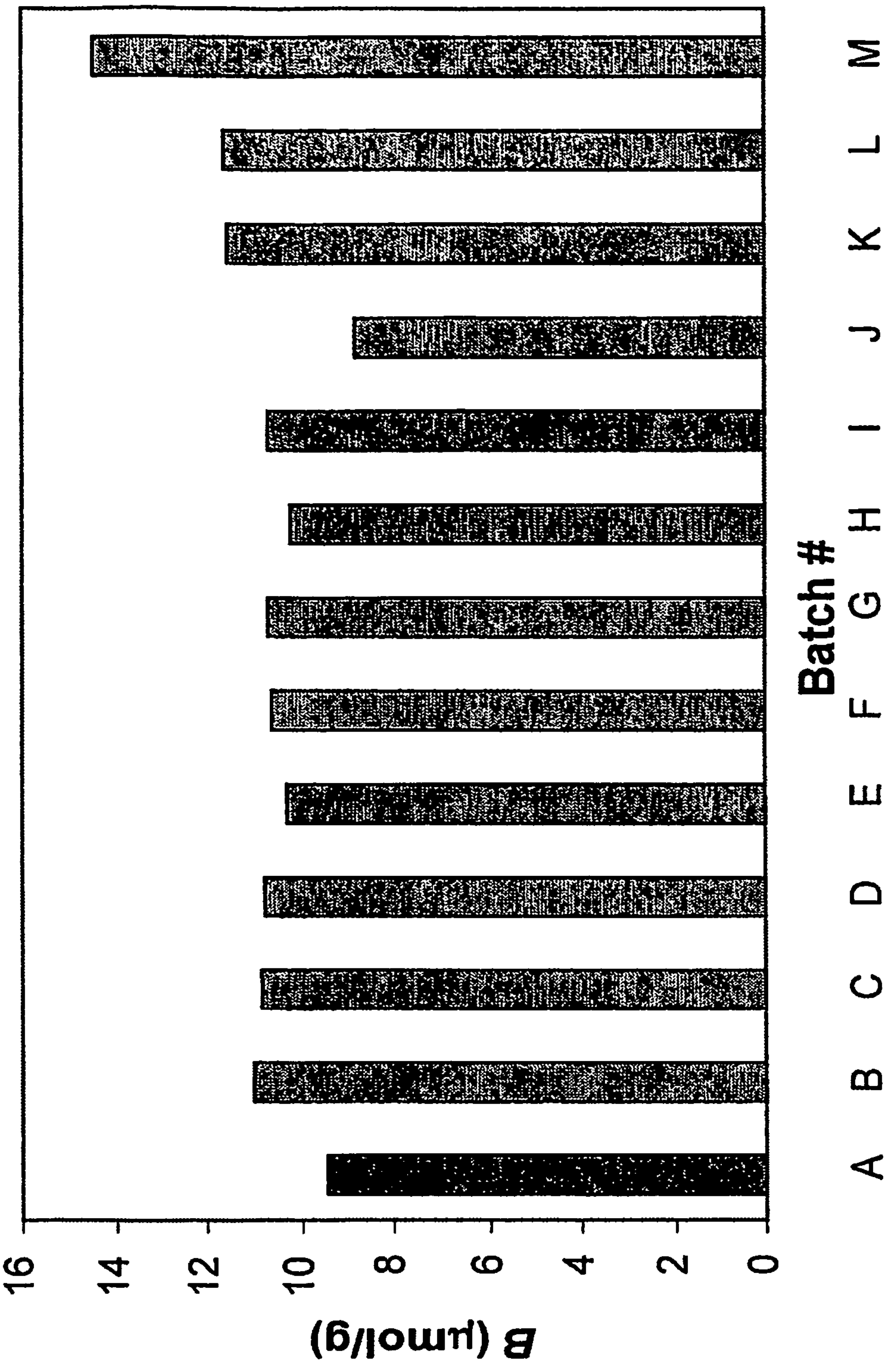


Figure 9

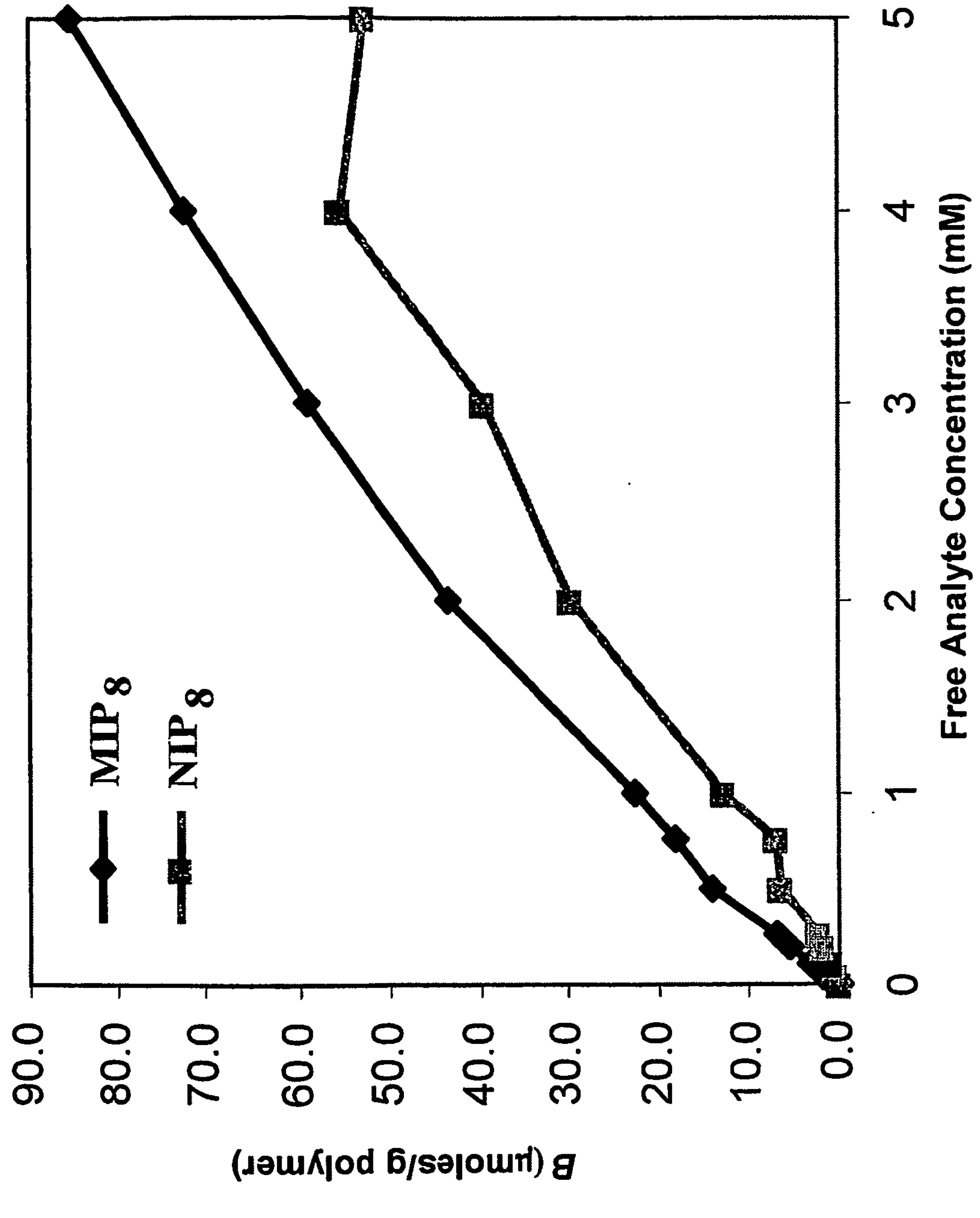
MIP8
NIP8
B (µmoles/g polymer)
90.0
80.0
70.0
60.0
50.0
40.0
30.0
20.0
10.0
0.0
0
1
2
3
4
5
Free Analyte Concentration (mM)

Figure 10

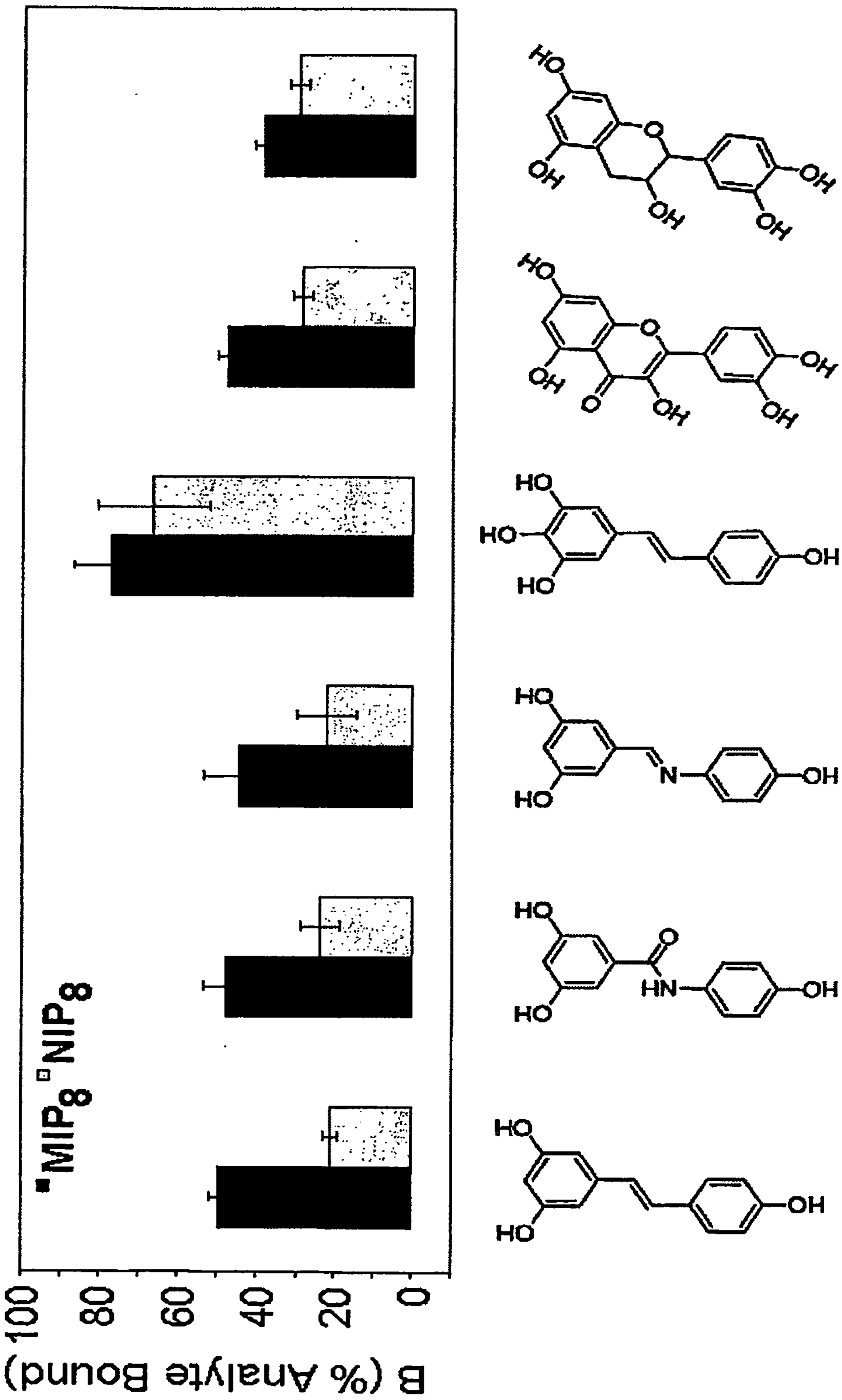


Figure 11A

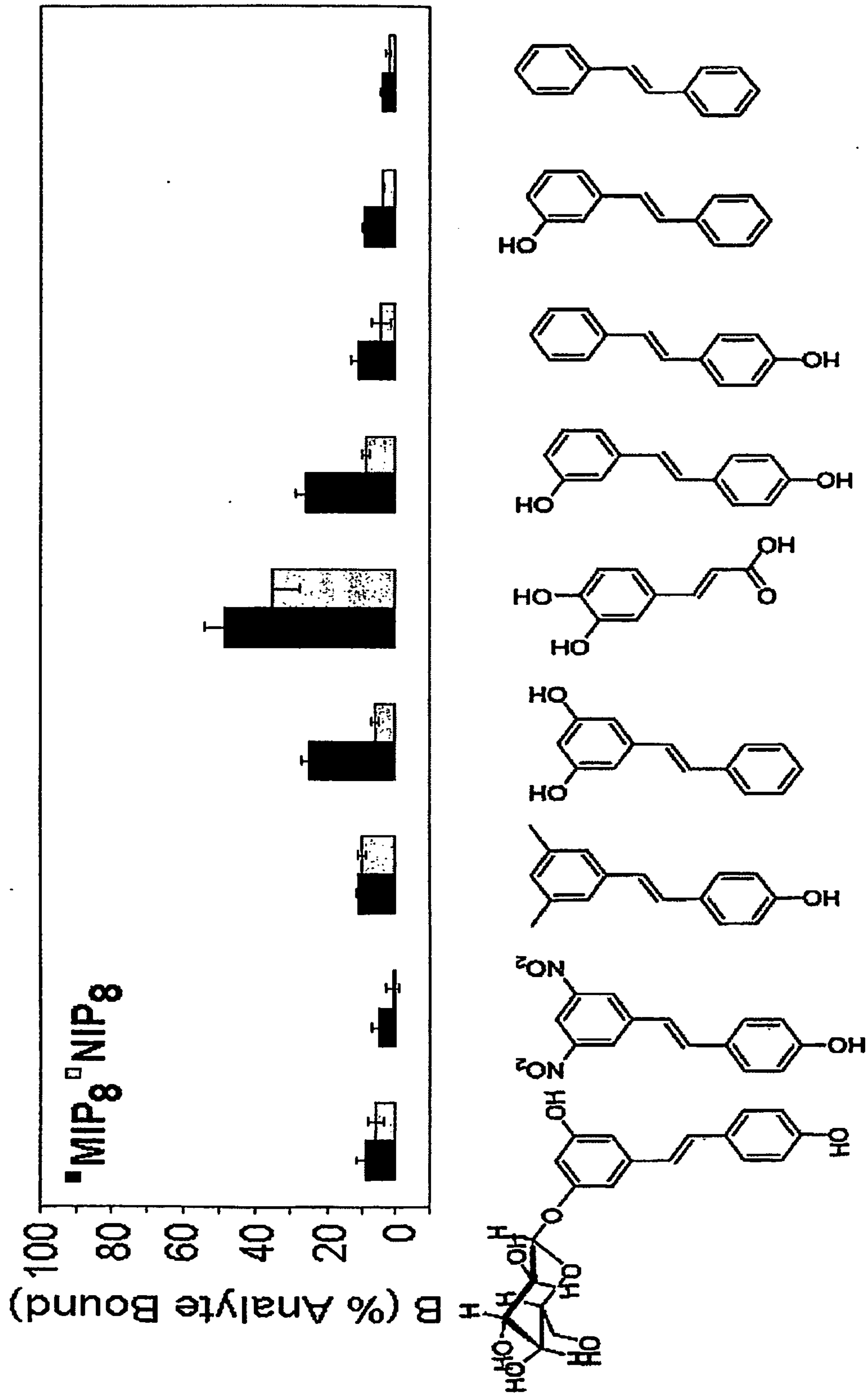


Figure 11B

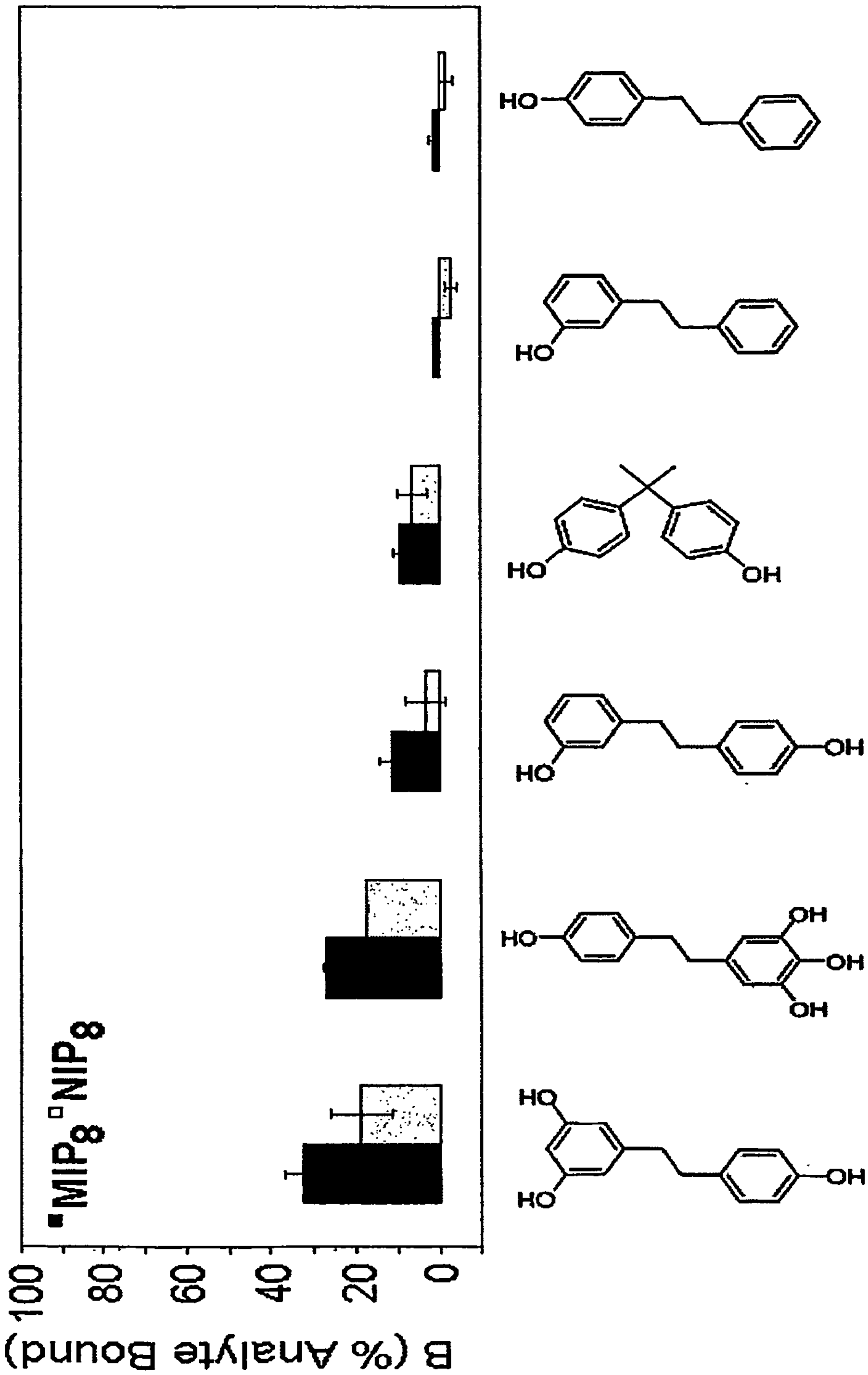


Figure 11C

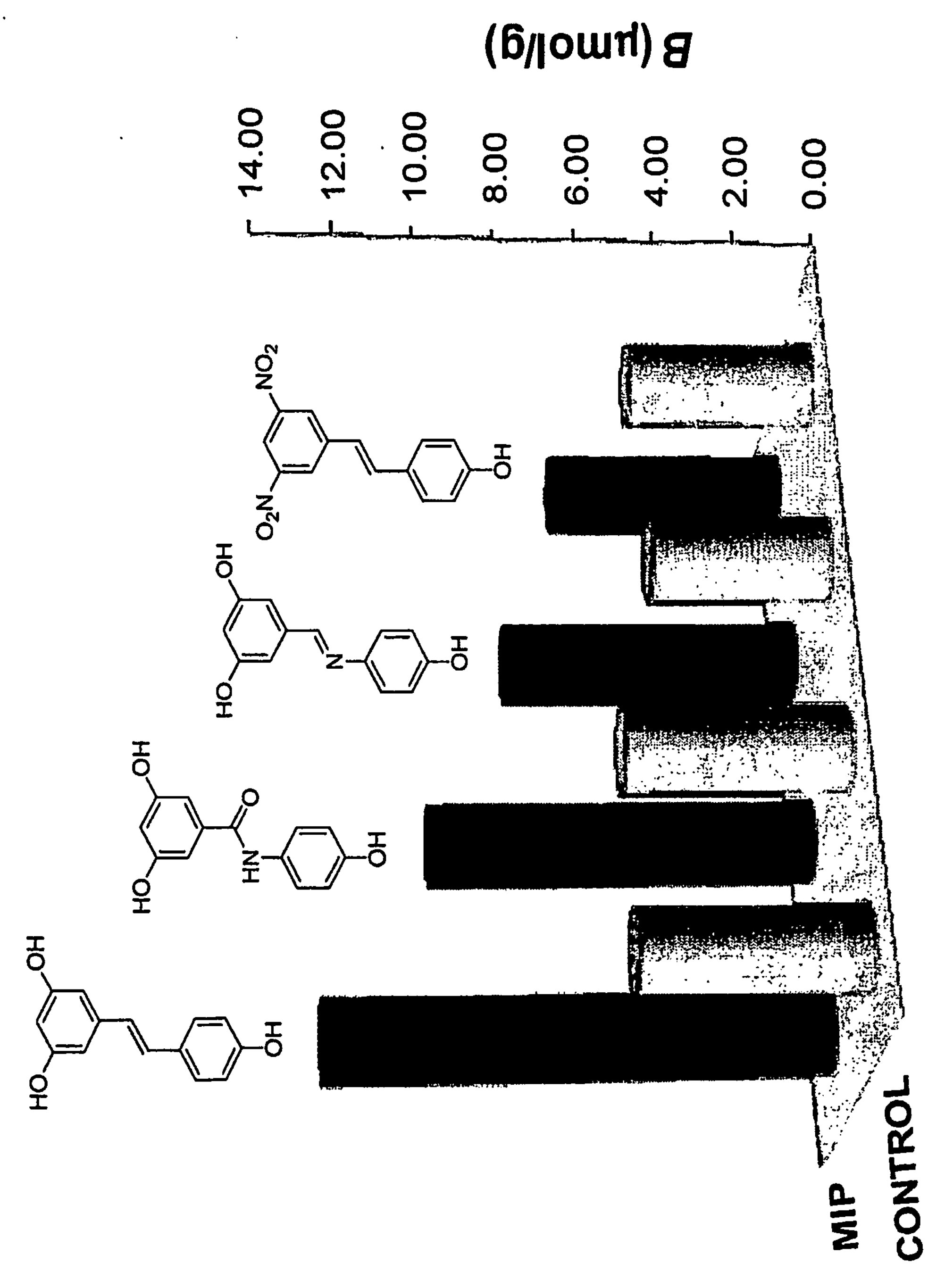


Figure 12

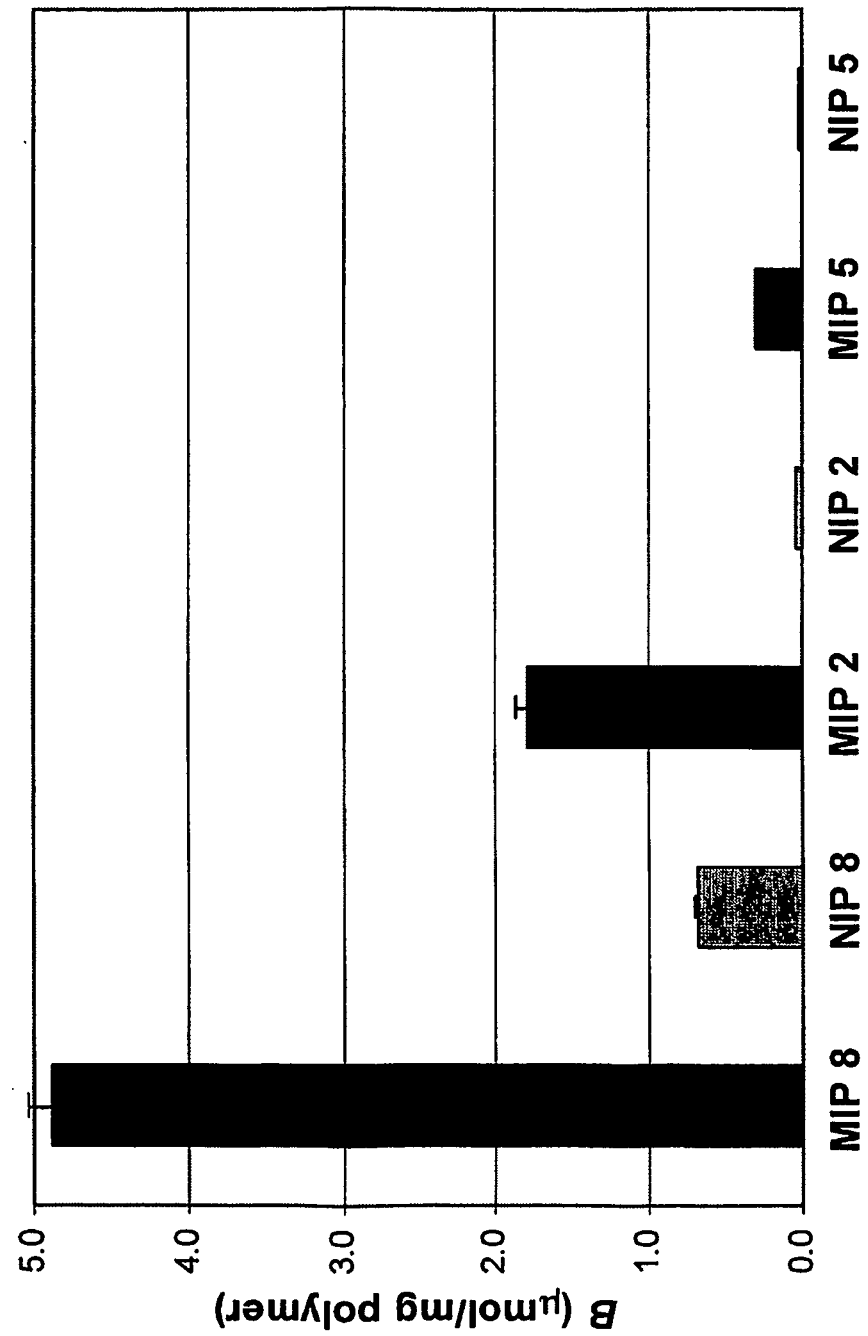
B (μmol/mg polymer)
5.0
4.0
3.0
2.0
1.0
0.0
MIP 8
NIP 8
MIP 2
NIP 2
MIP 5
NIP 5

Figure 13

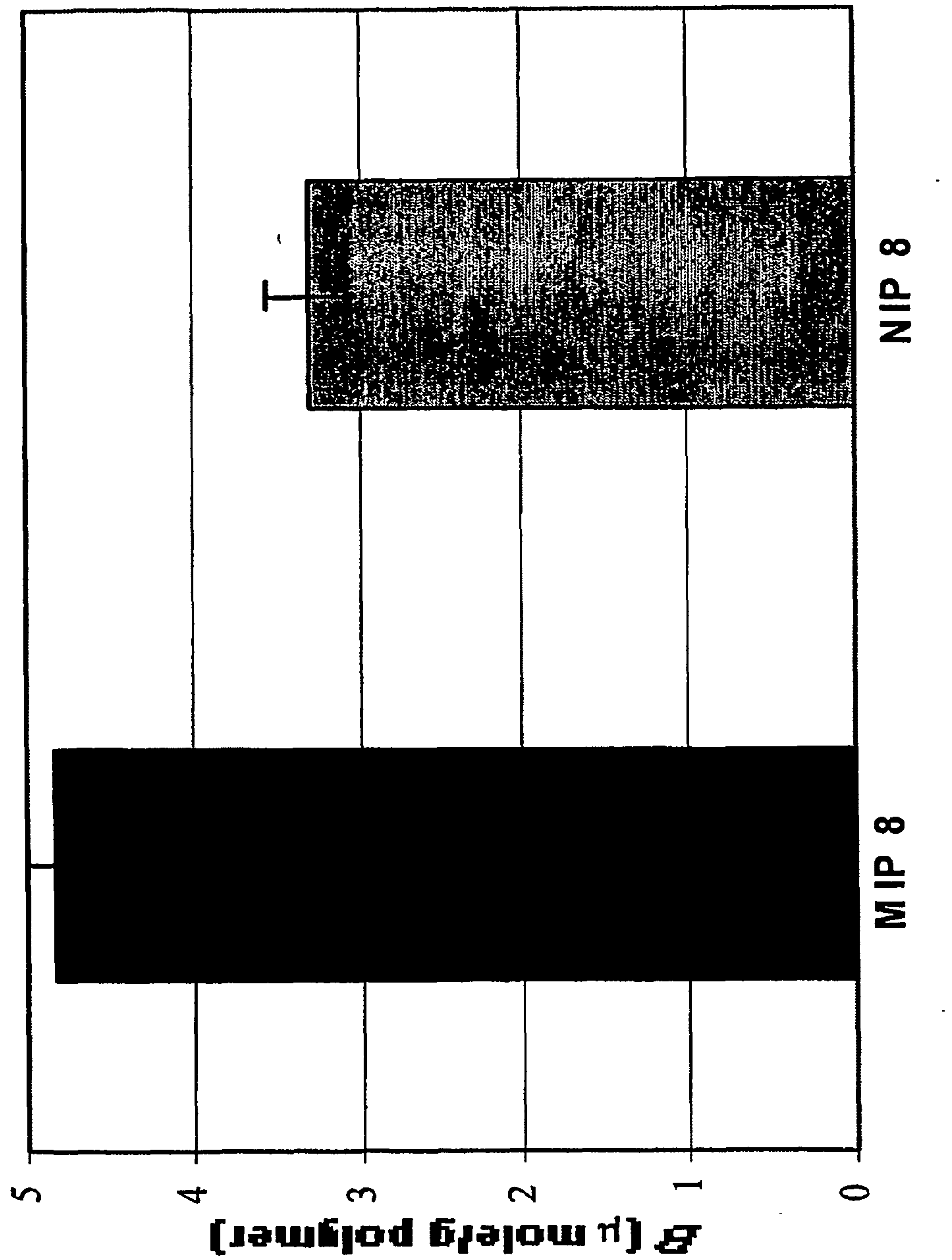


Figure 14A

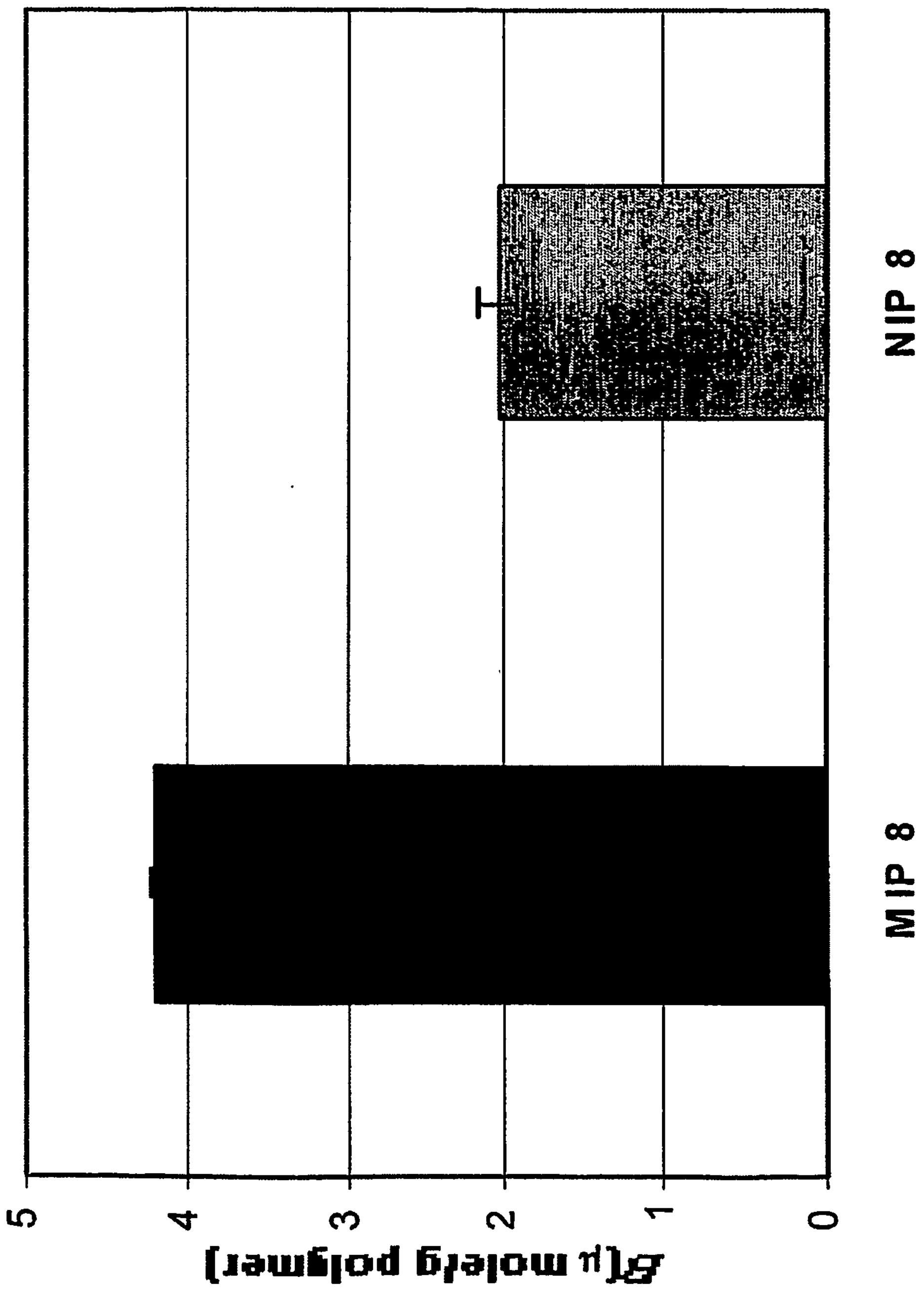


Figure 14B

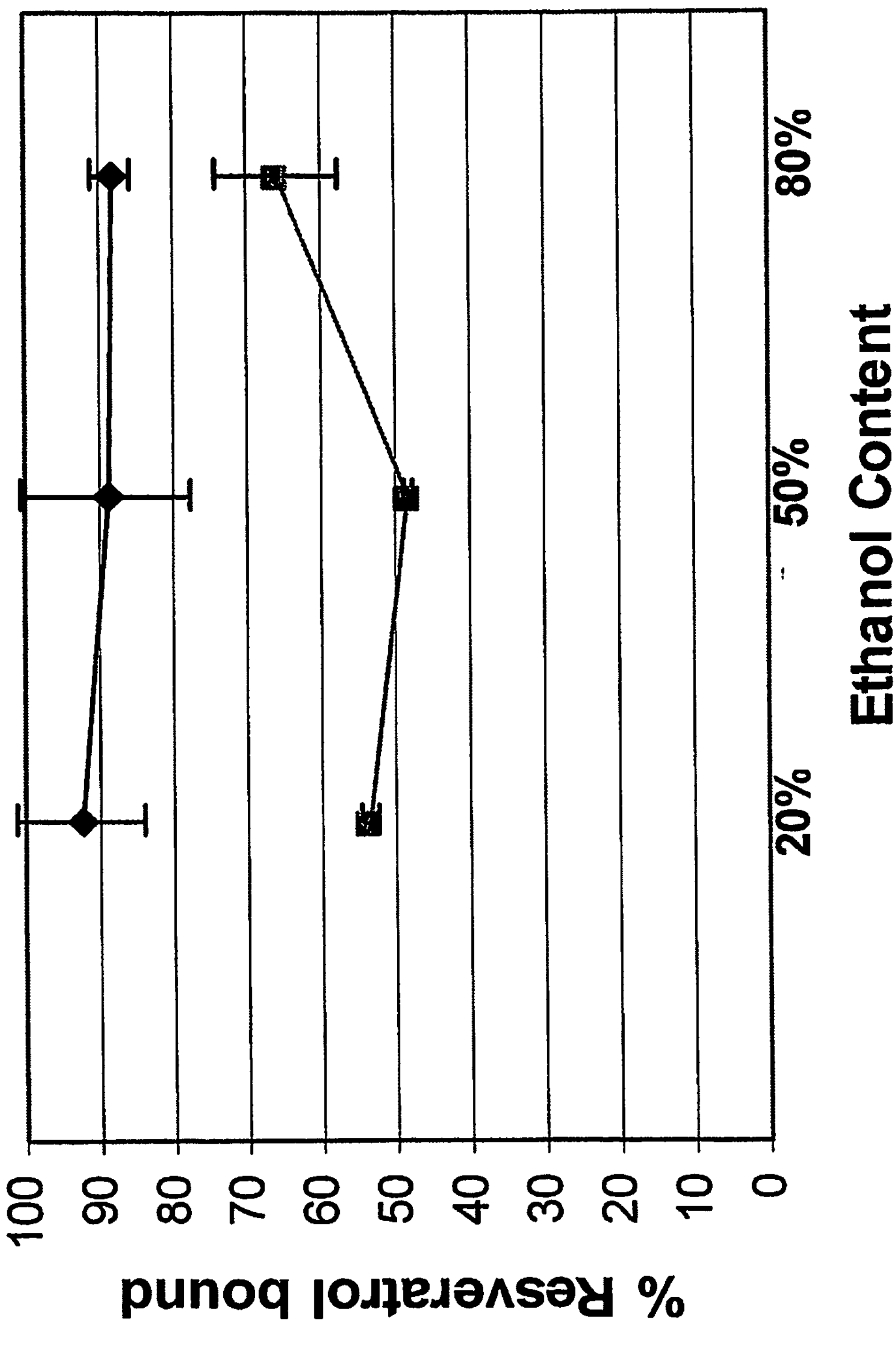
% Resveratrol bound
100
90
80
70
60
50
40
30
20
10
0
20%
50%
80%
Ethanol Content

Figure 15A

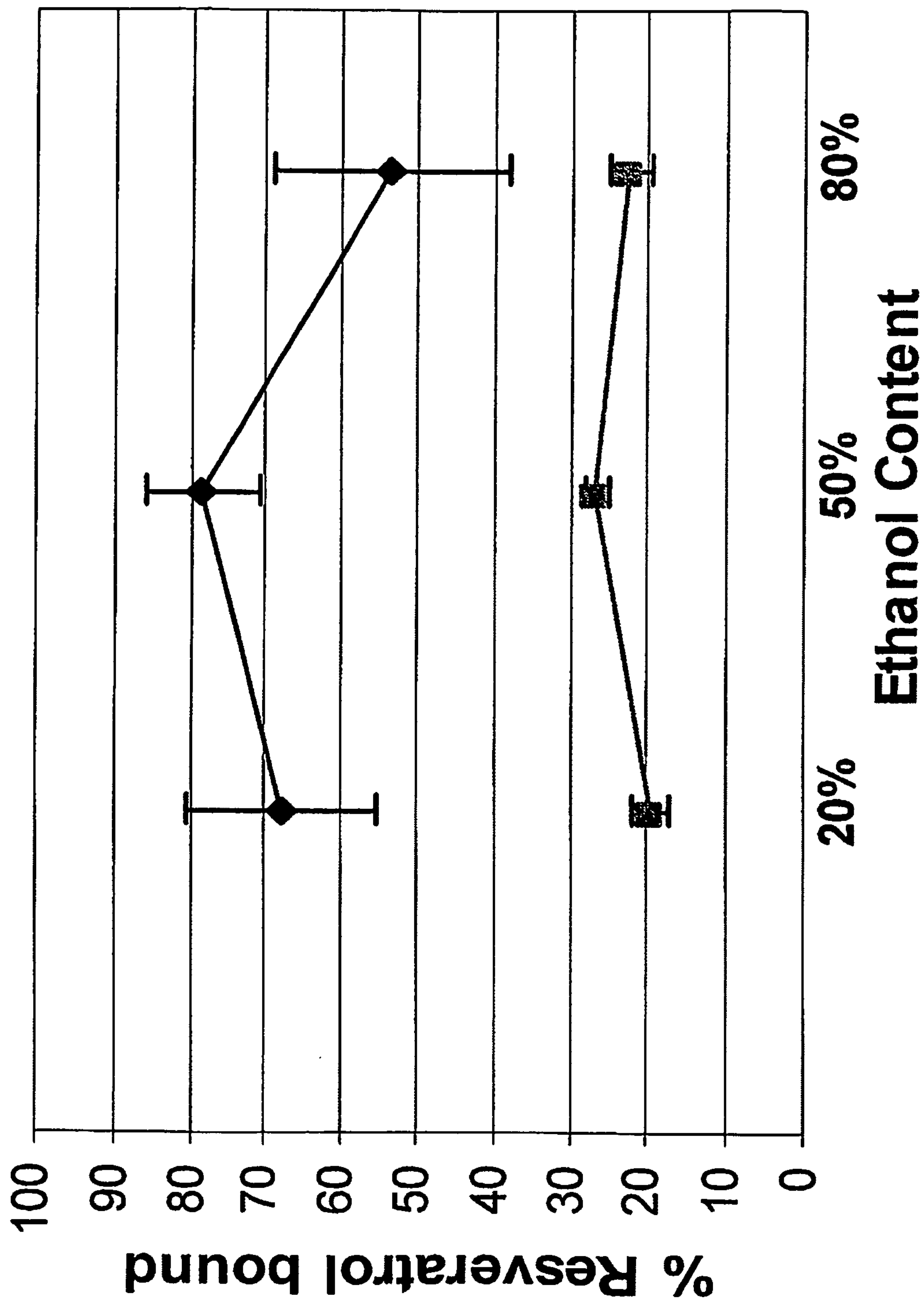
% Resveratrol bound
100
90
80
70
60
50
40
30
20
10
0
20%
50%
80%
Ethanol Content

Figure 15B

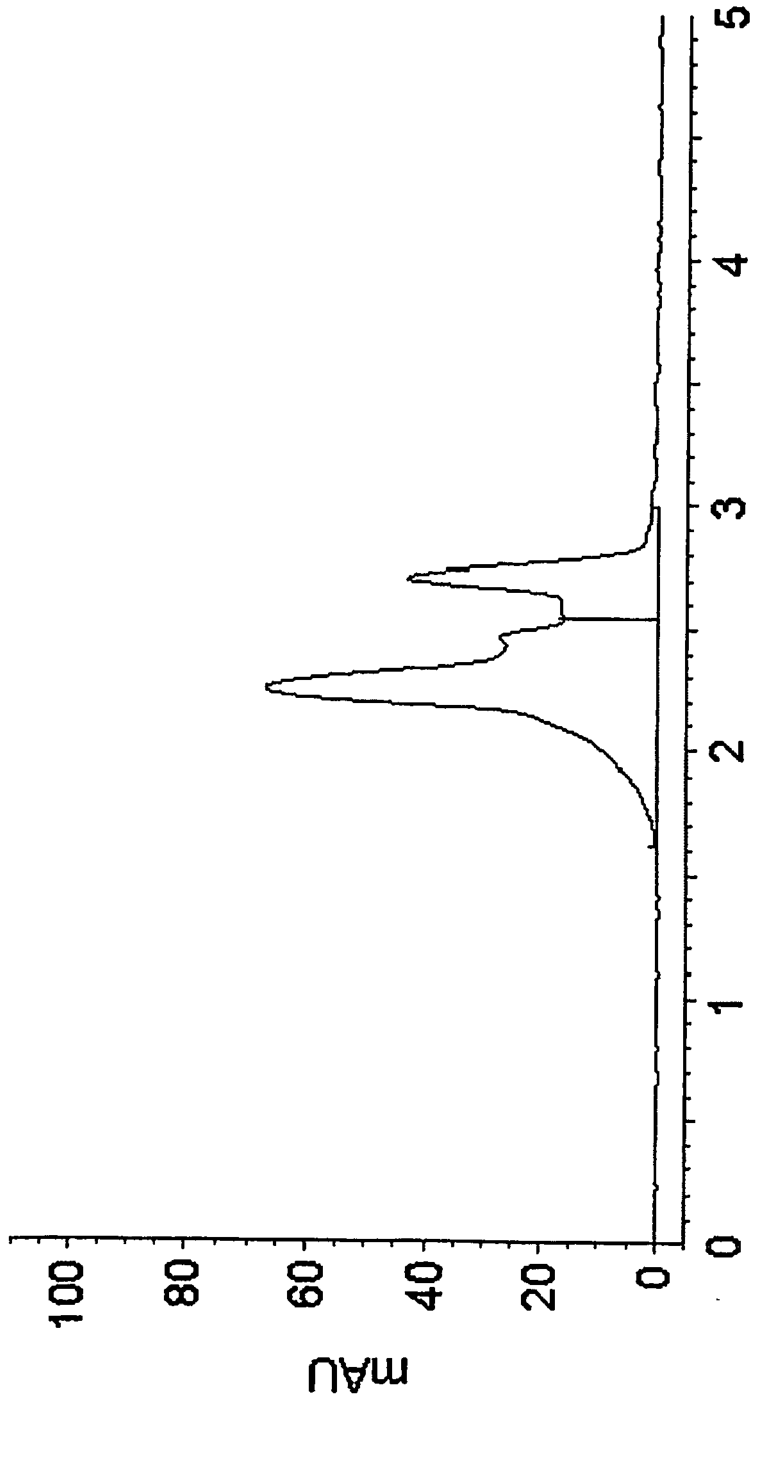


Figure 16A

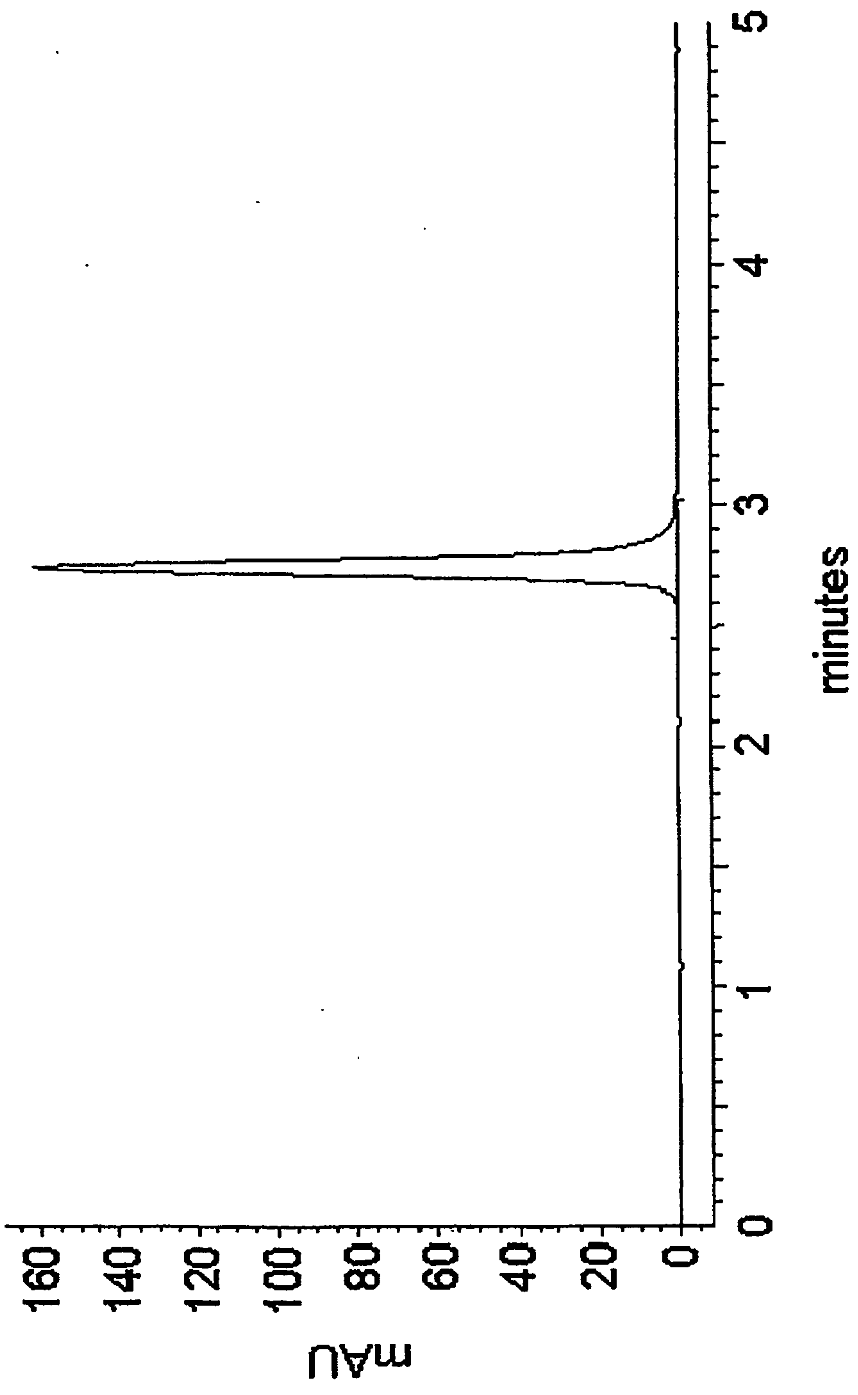


Figure 16B

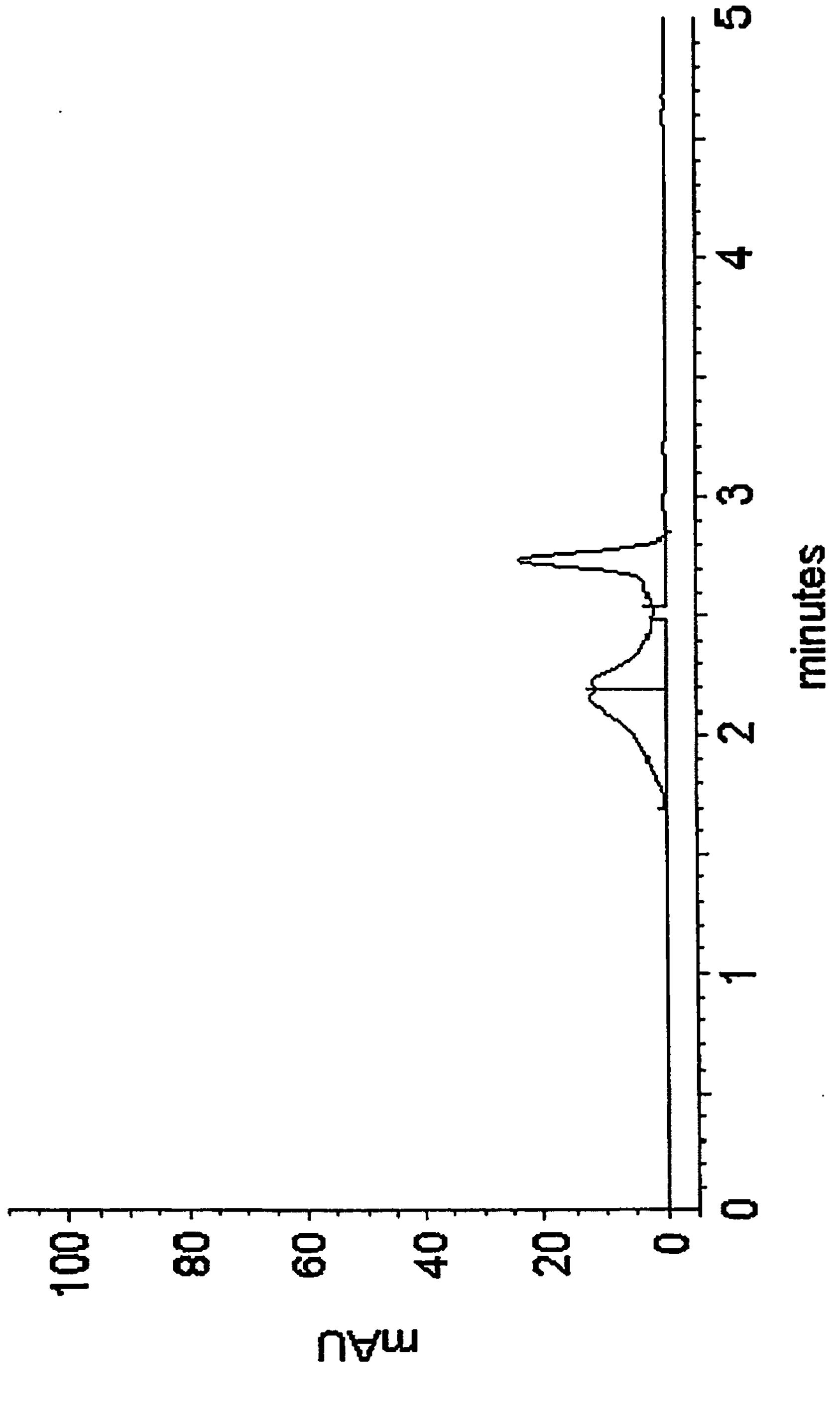


Figure 16C

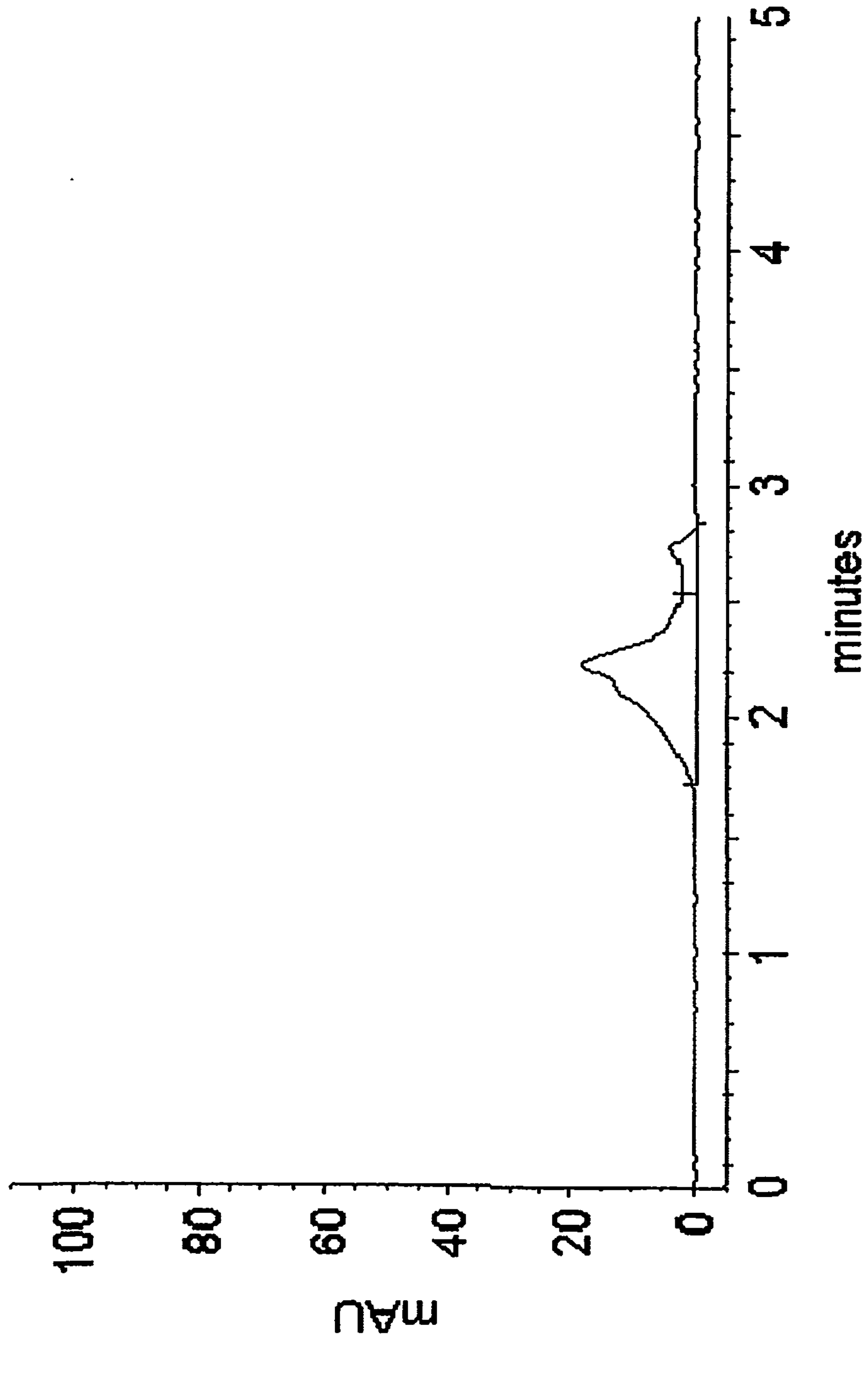


Figure 16D

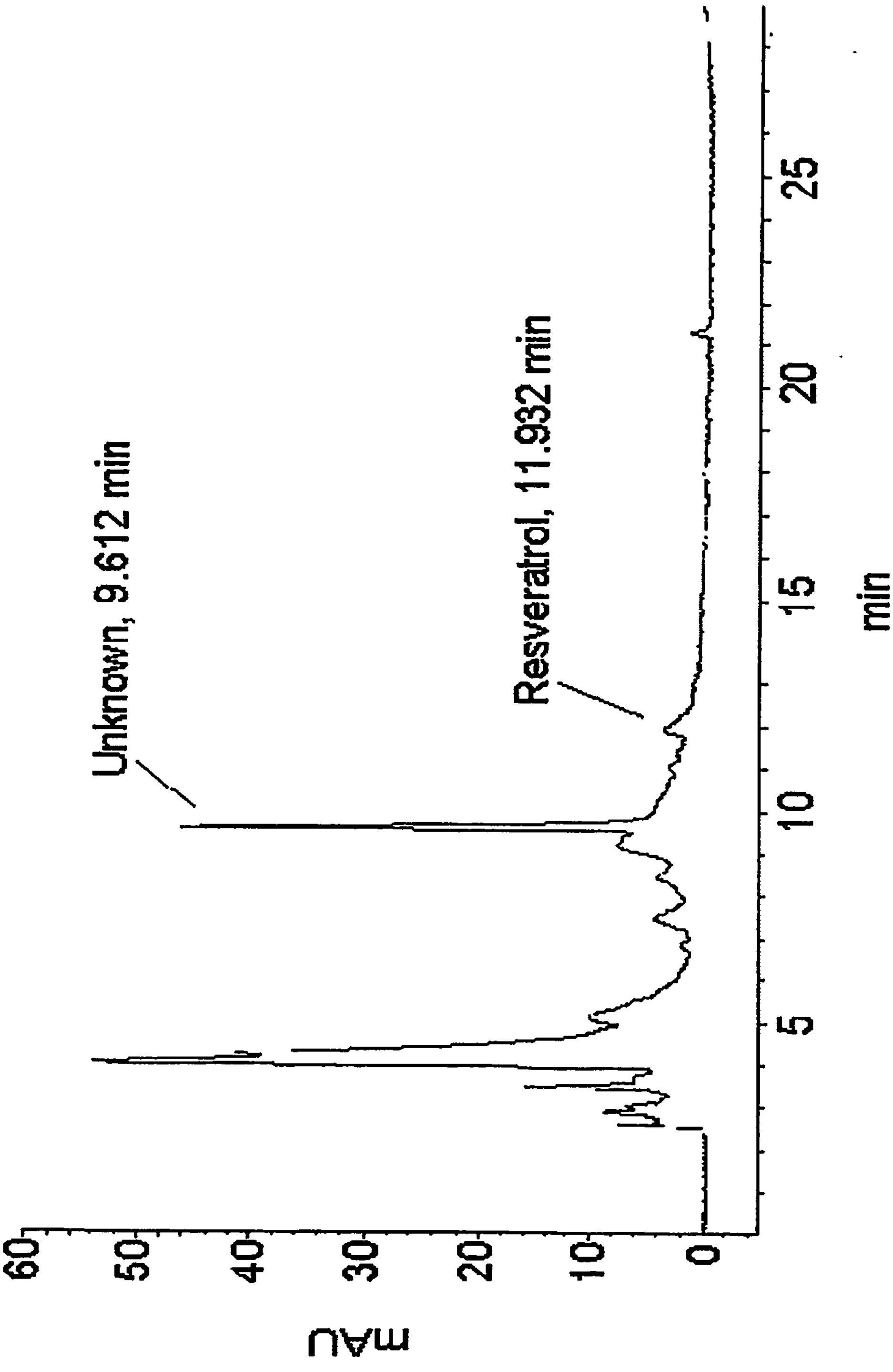
Unknown, 9.612 min
Resveratrol, 11.932 min
mAU
60
50
40
30
20
10
0
5
10
15
20
25
min

Figure 17A

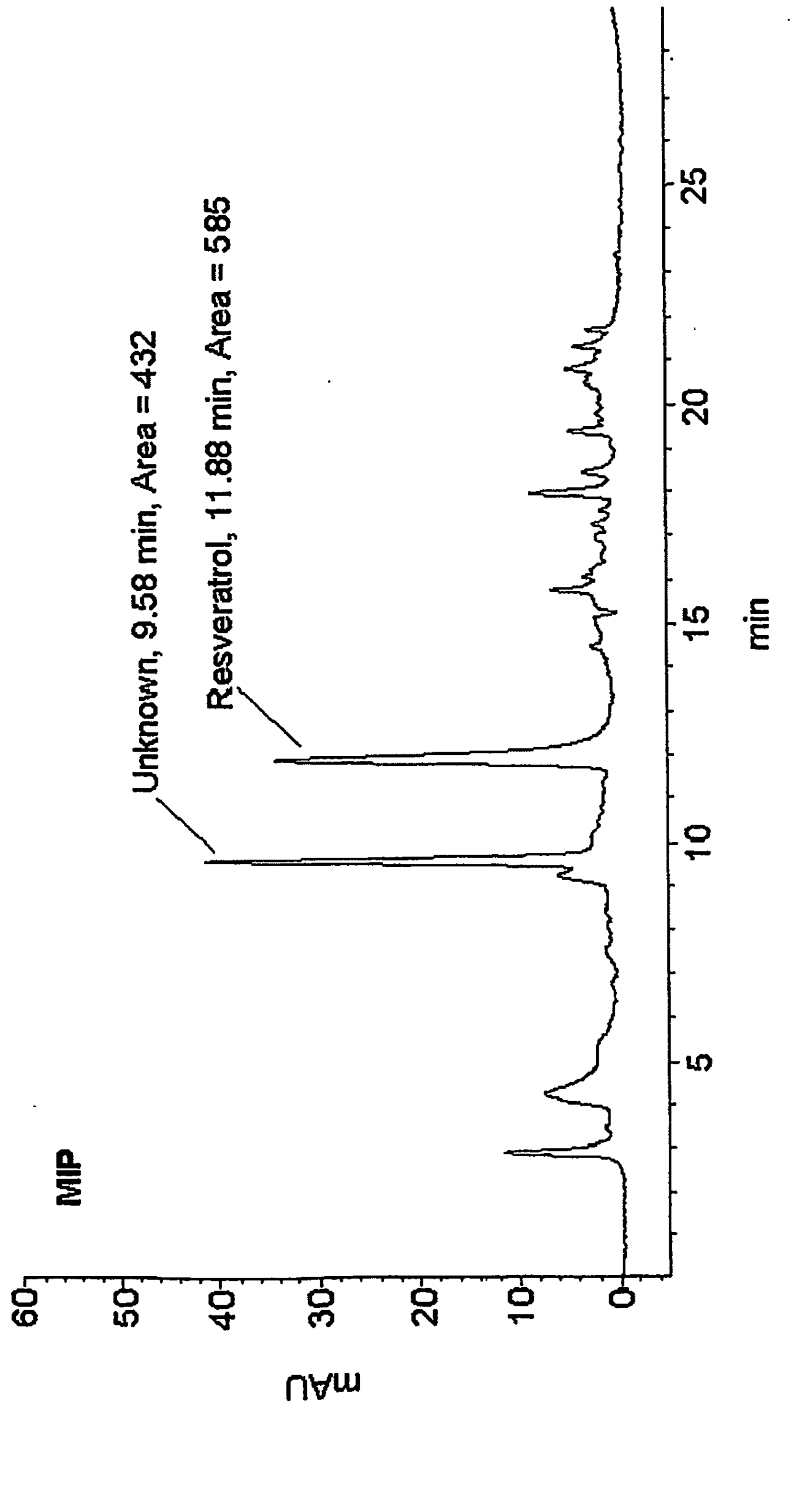
MIP
Unknown, 9.58 min, Area = 432
Resveratrol, 11.88 min, Area = 585
mAU
60
50
40
30
20
10
0
5
10
15
20
25
min

Figure 17B

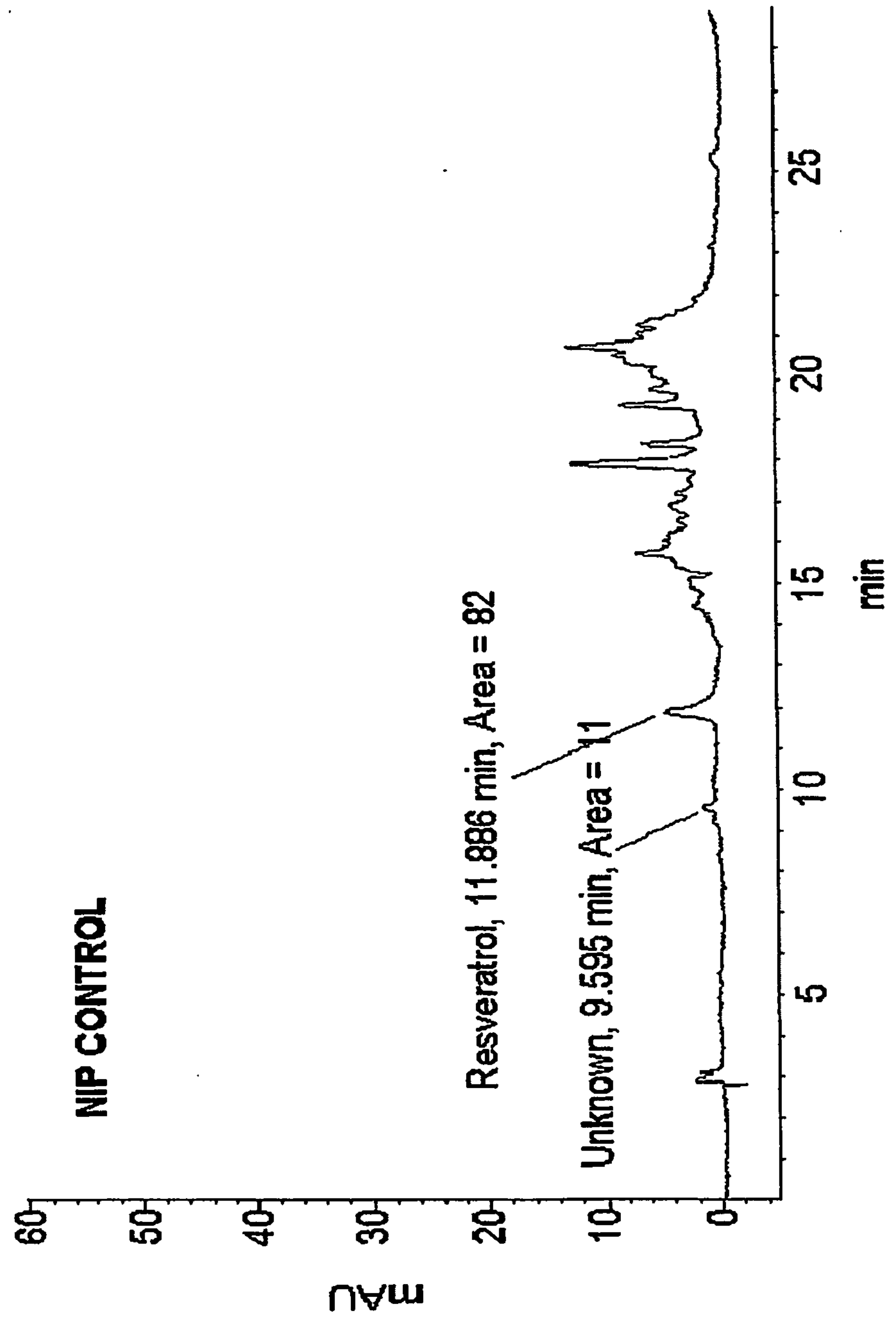

NIP CONTROL
Resveratrol, 11.886 min, Area = 82
Unknown, 9.595 min, Area = 11
mAU
0
10
20
30
40
50
60
min
5
10
15
20
25


Figure 17C

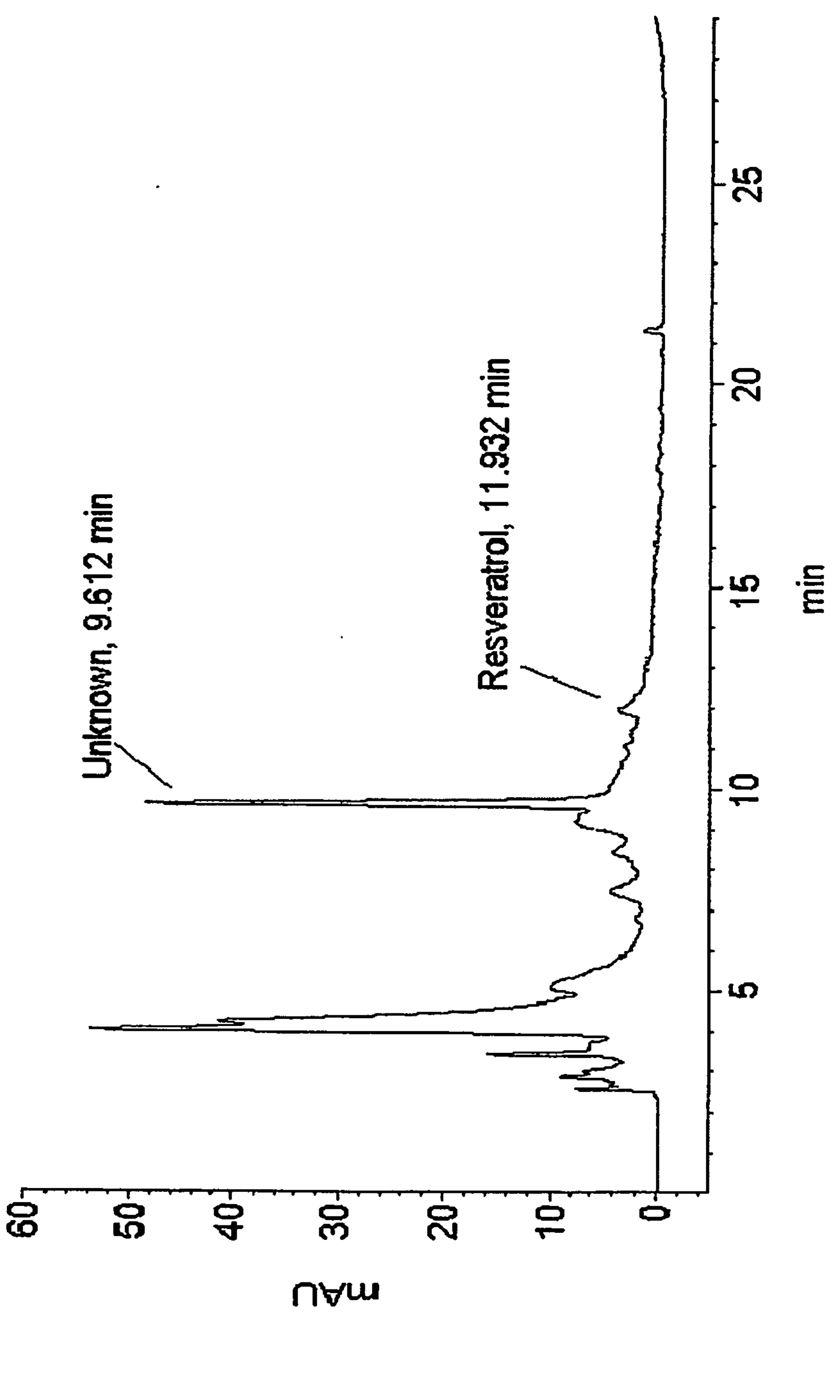

Unknown, 9.612 min
Resveratrol, 11.932 min
mAU
min
0
10
20
30
40
50
60
5
10
15
20
25


Figure 18A

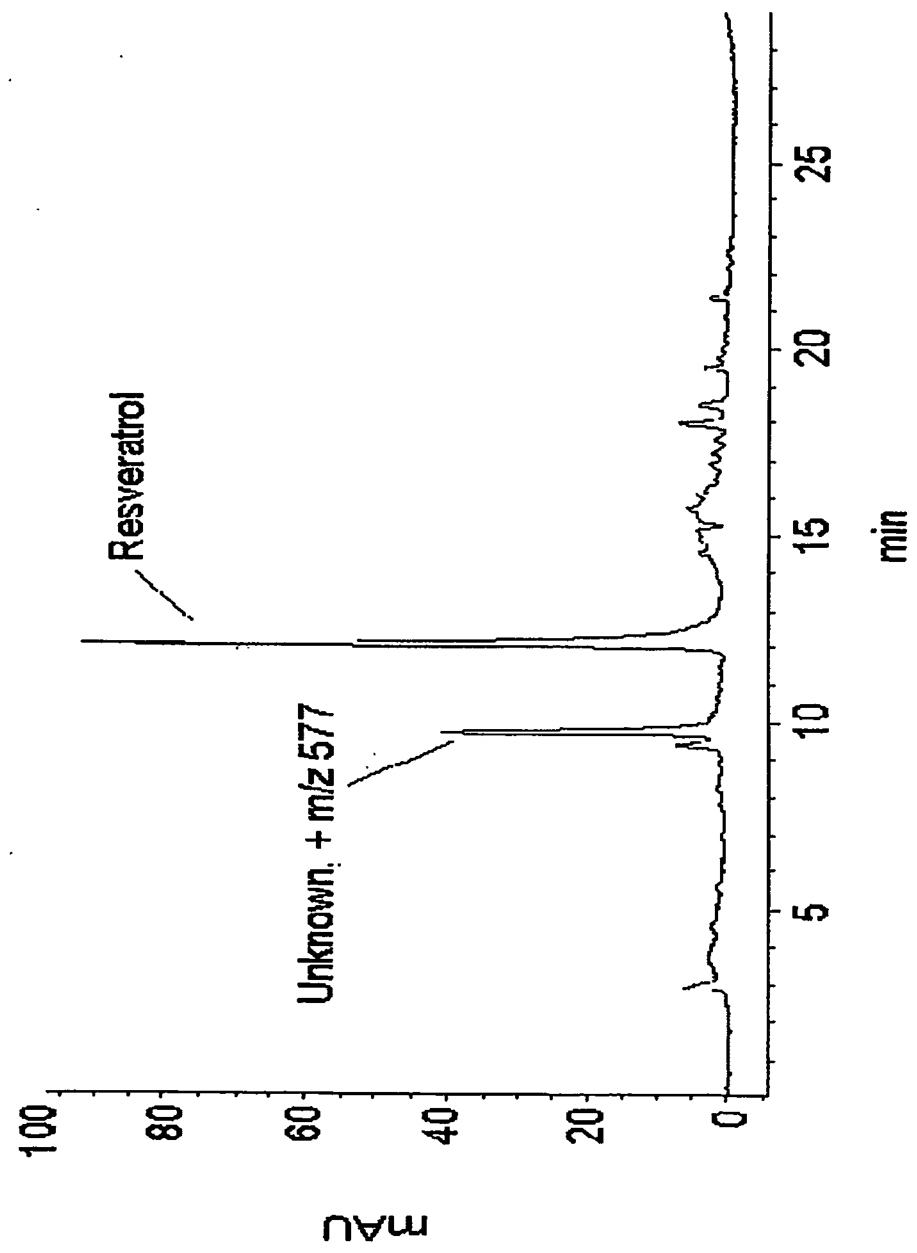
Resveratrol
Unknown + m/z 577
mAU
100
80
60
40
20
0
5
10
15
20
25
min

Figure 18B

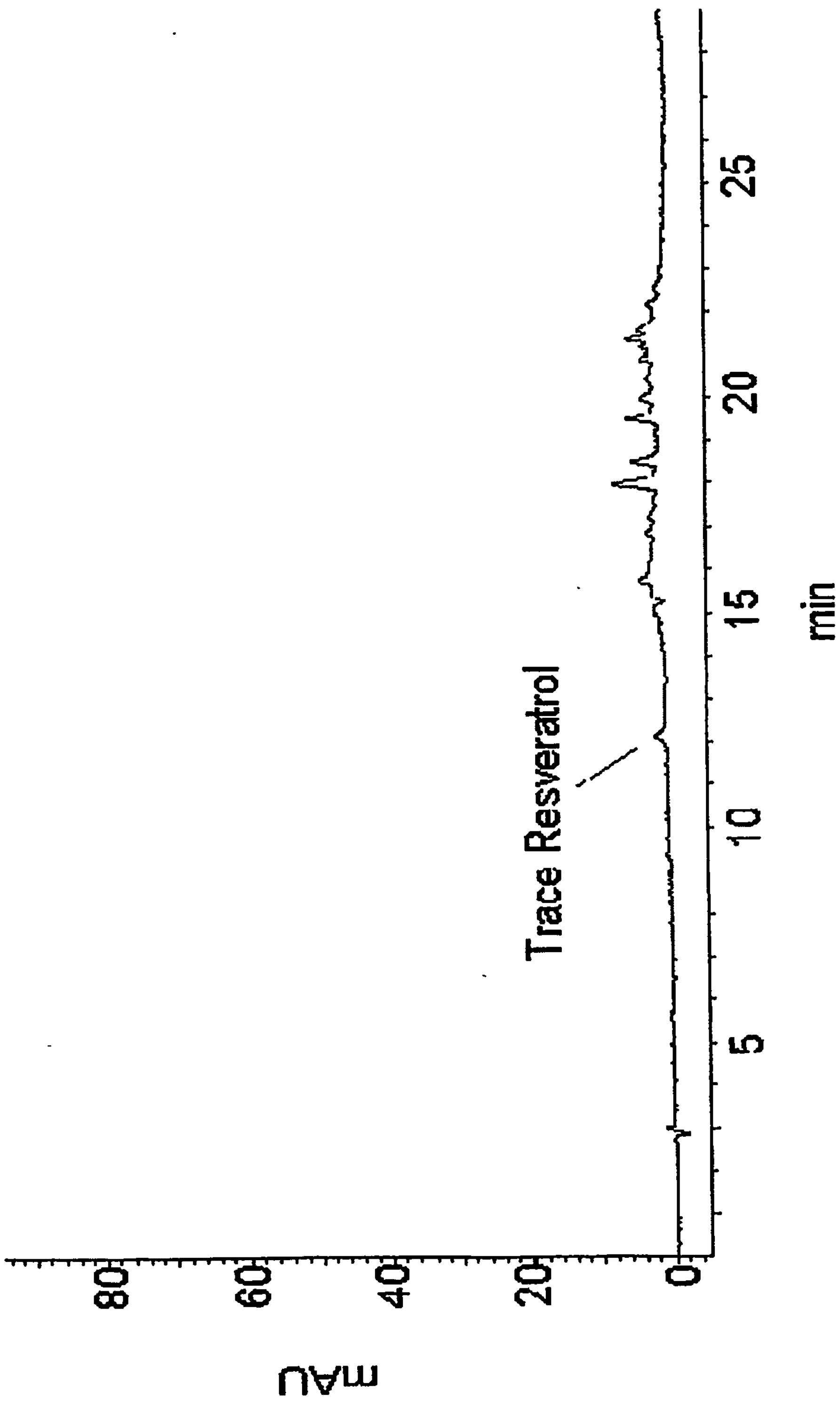


Figure18C

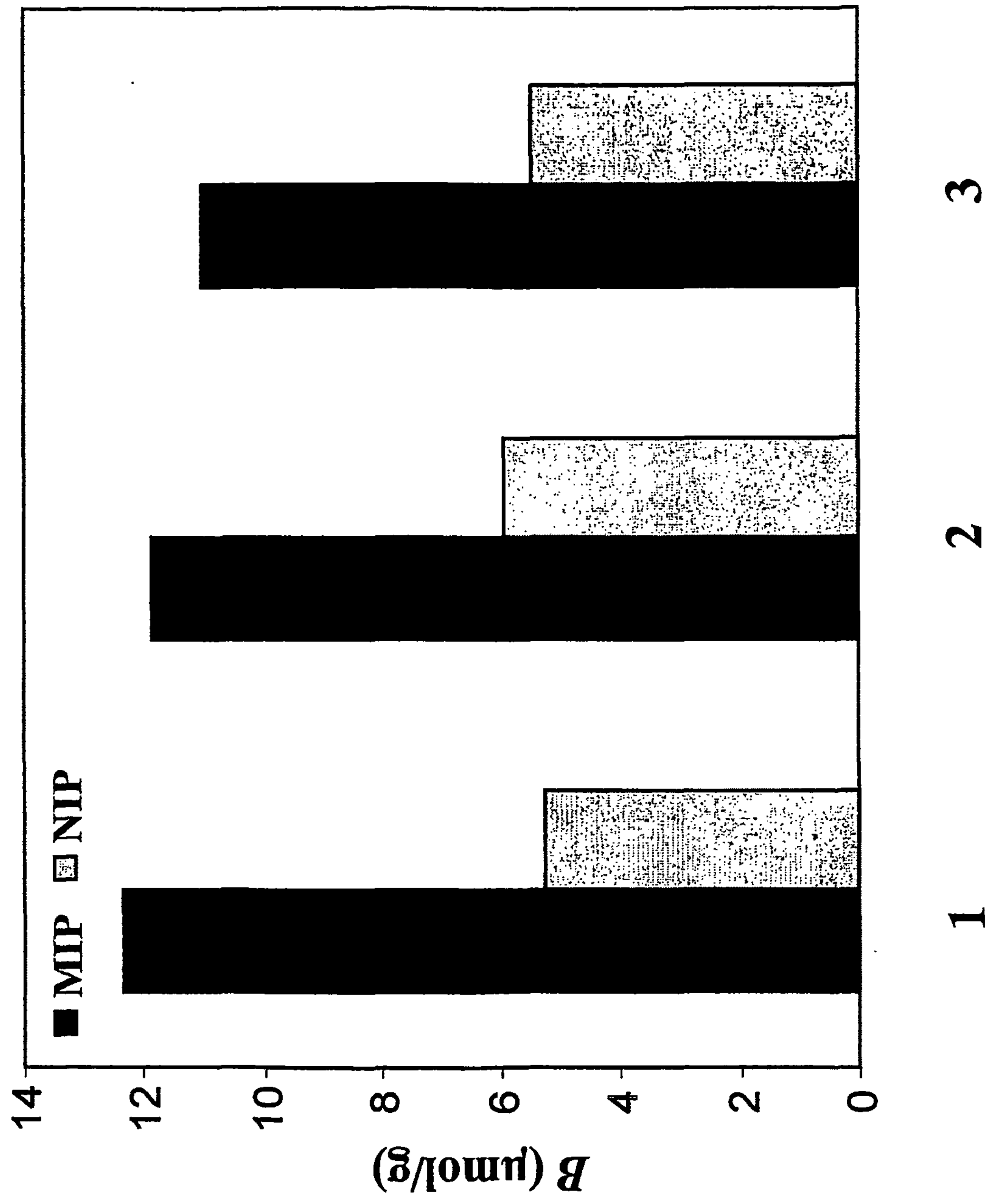


Figure 19

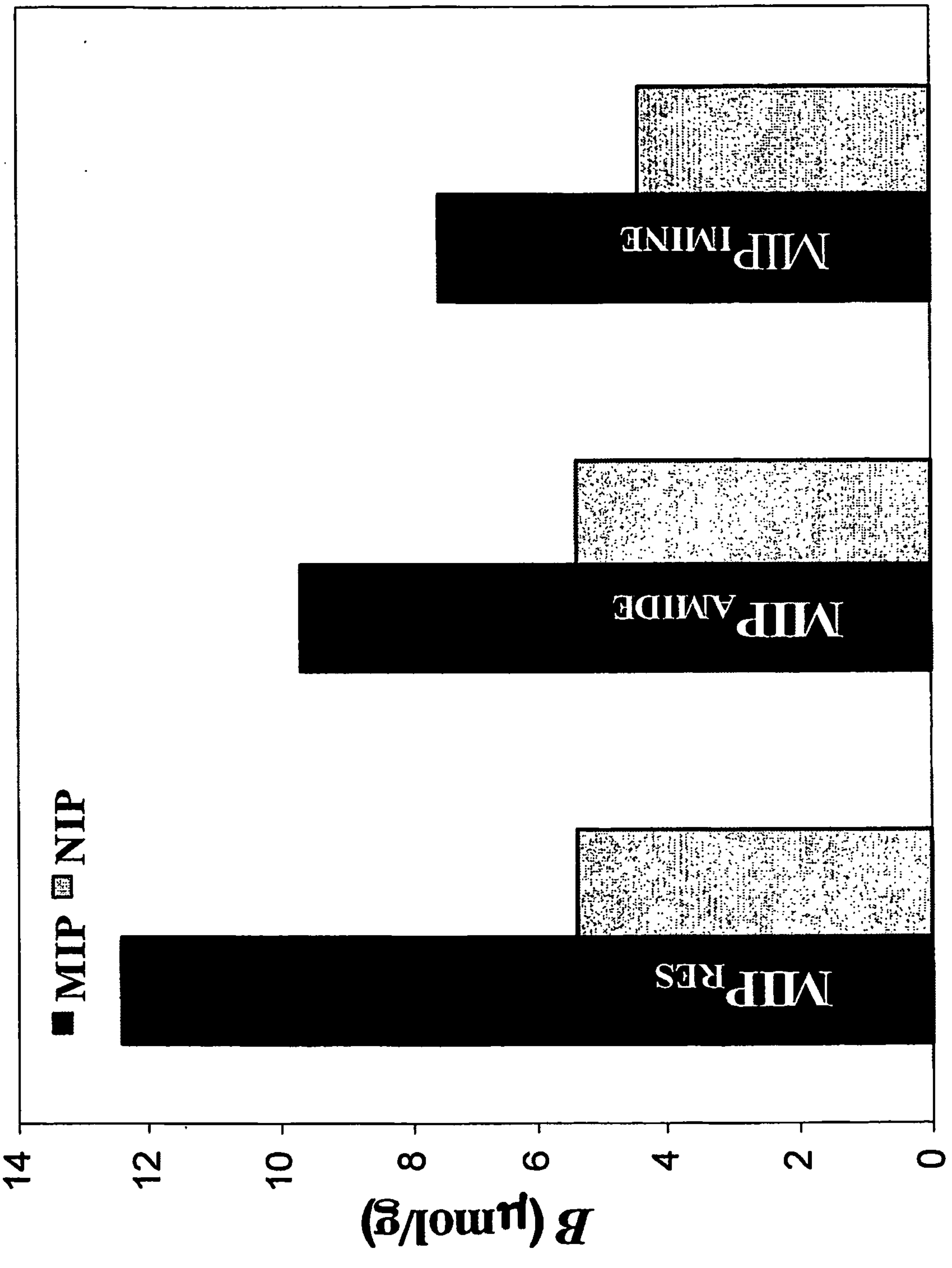


Figrure 20

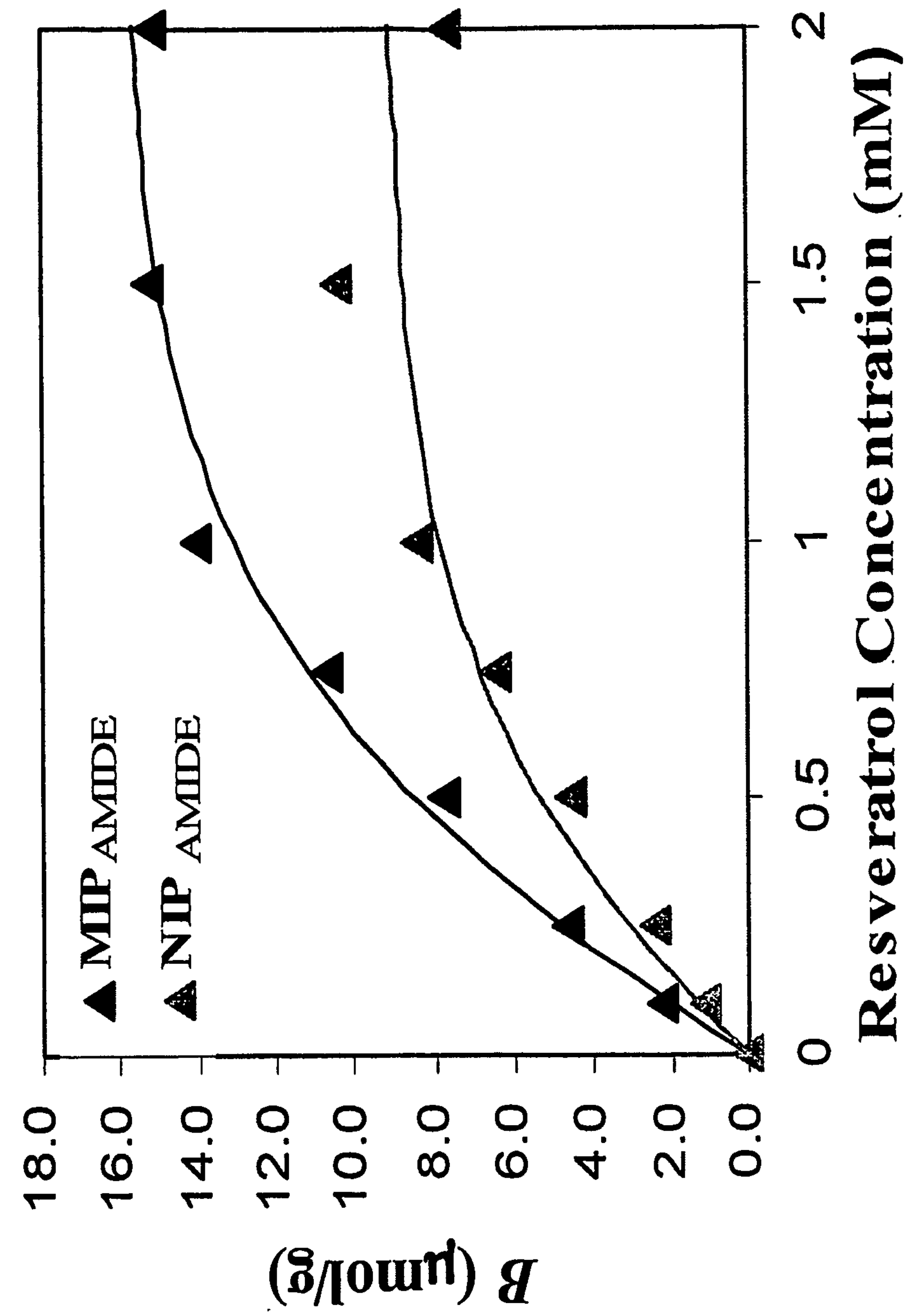

MIP AMIDE
NIP AMIDE
18.0
16.0
14.0
12.0
10.0
8.0
6.0
4.0
2.0
0.0
B (μmol/g)
0
0.5
1
1.5
2
Resveratrol Concentration (mM)


Figure 21A

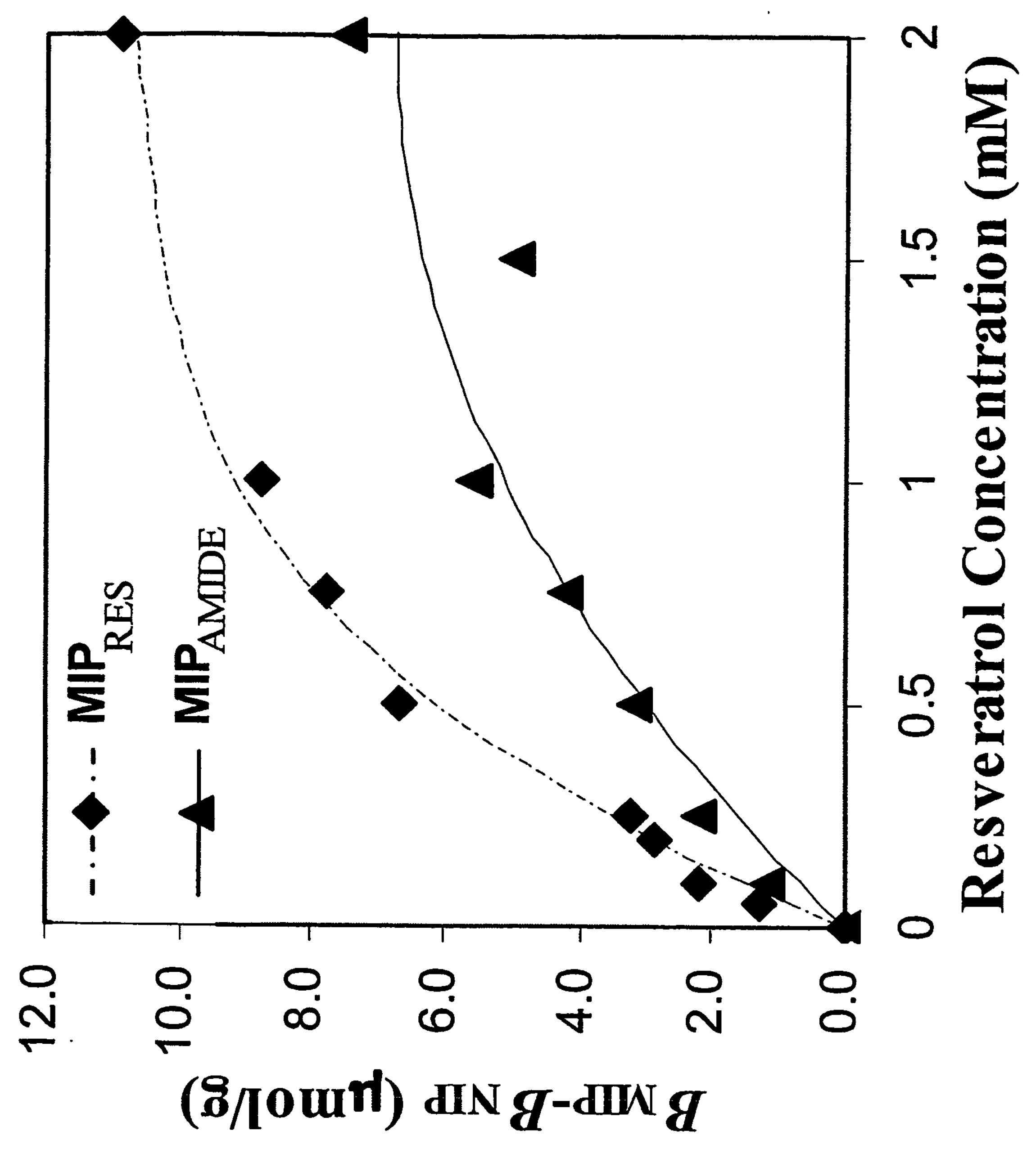

B MIP-B NIP (µmol/g)
12.0
10.0
8.0
6.0
4.0
2.0
0.0
0
0.5
1
1.5
2
Resveratrol Concentration (mM)
MIPRES
MIPAMIDE


Figure 21B

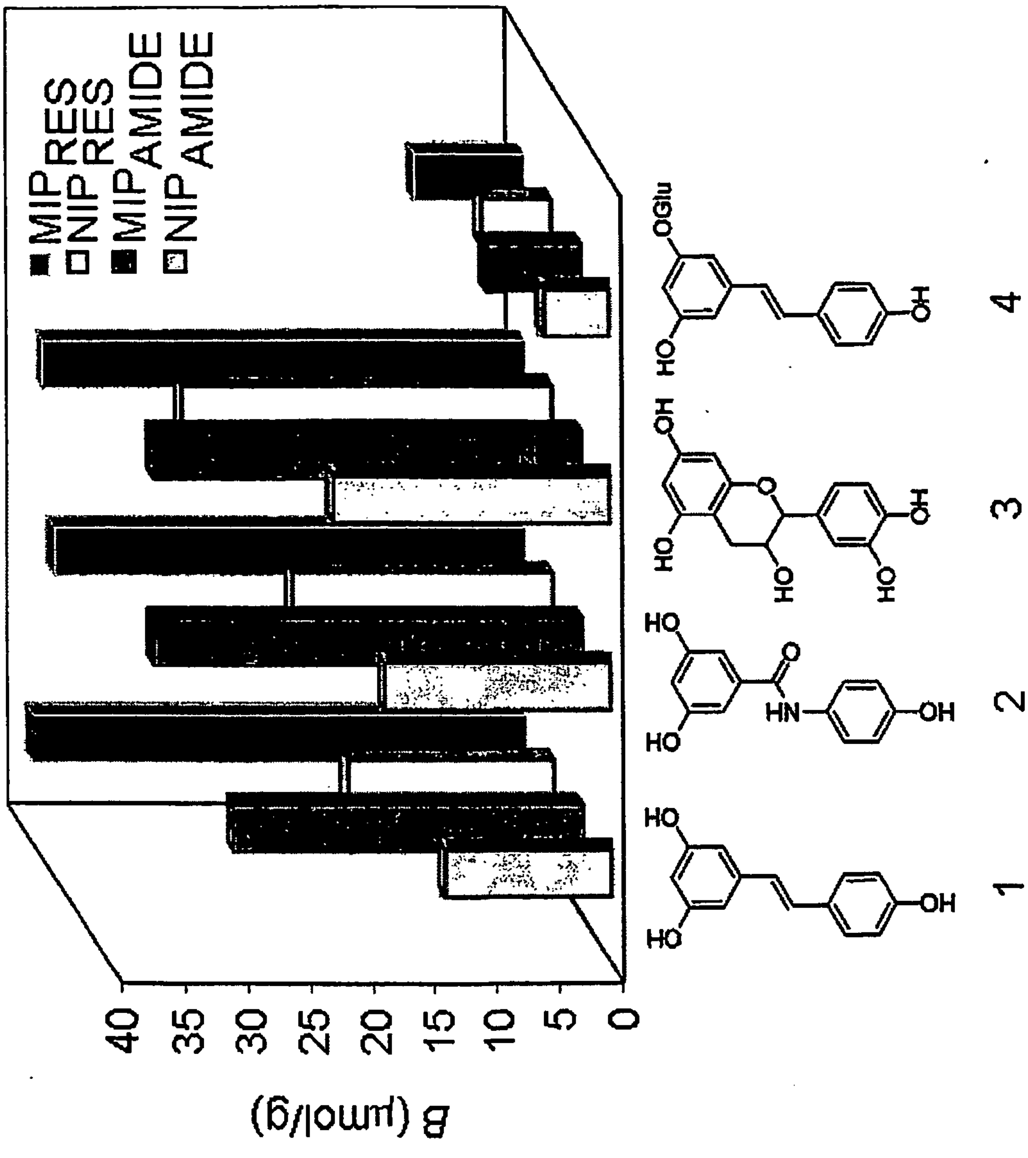
MIP RES
NIP RES
MIP AMIDE
NIP AMIDE
B (µmol/g)
40
35
30
25
20
15
10
5
0
1
2
3
4

Figure 22

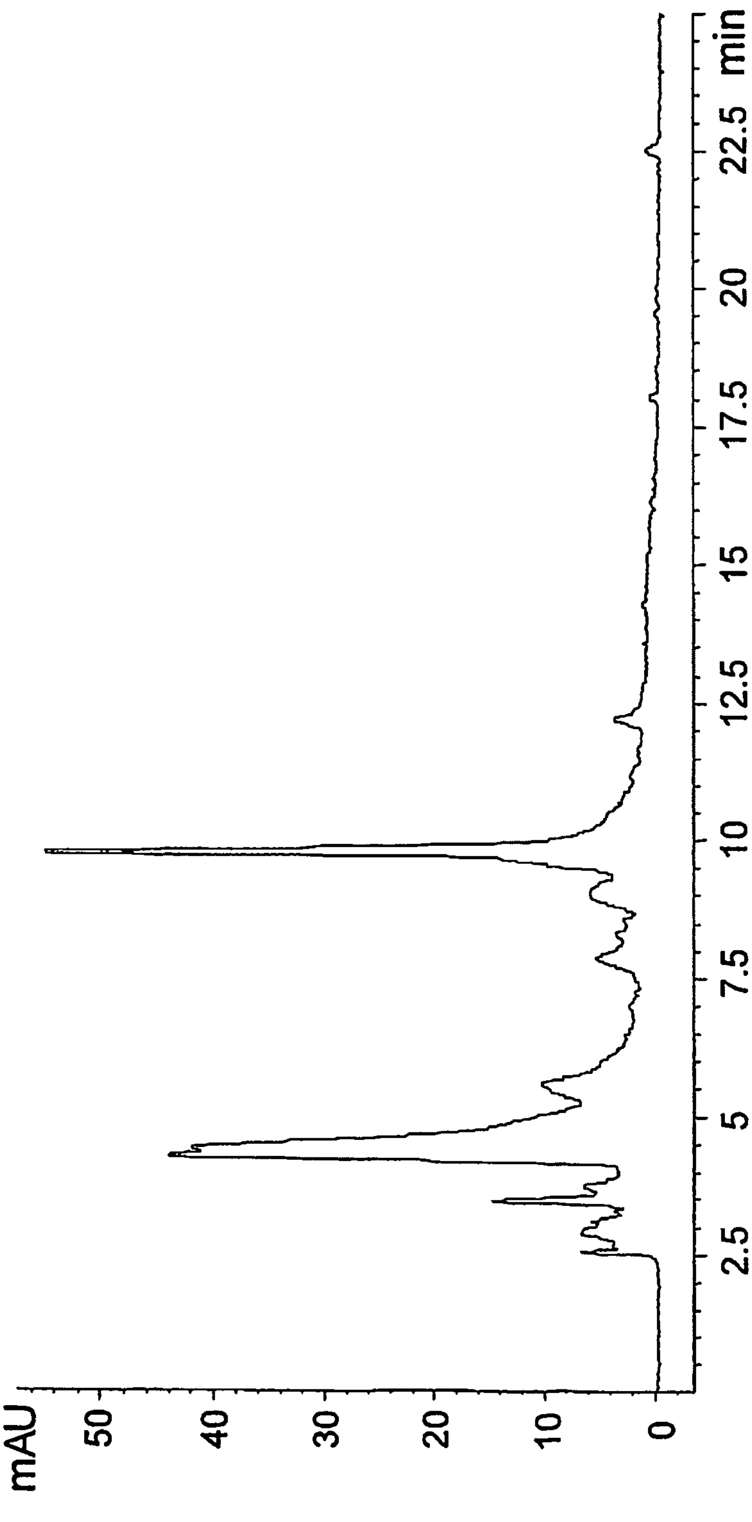


Figure 23A

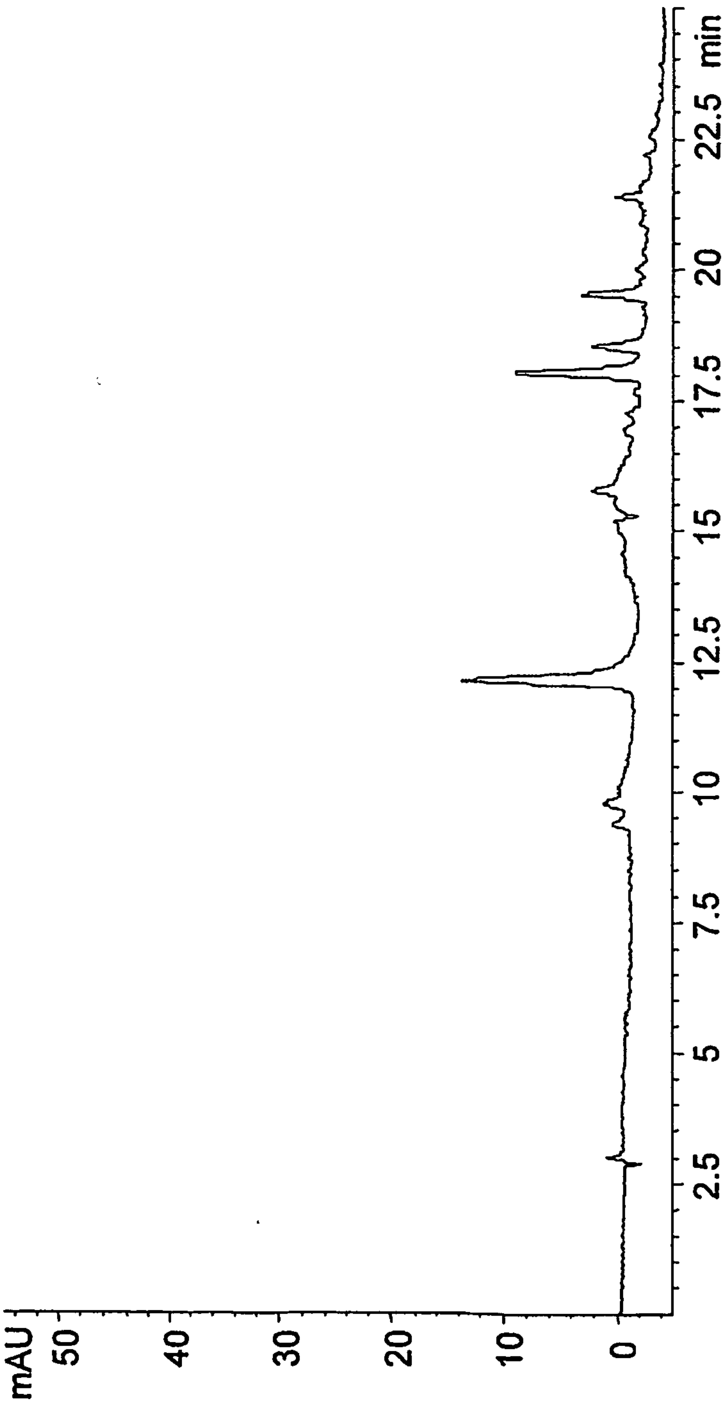


Figure 23B

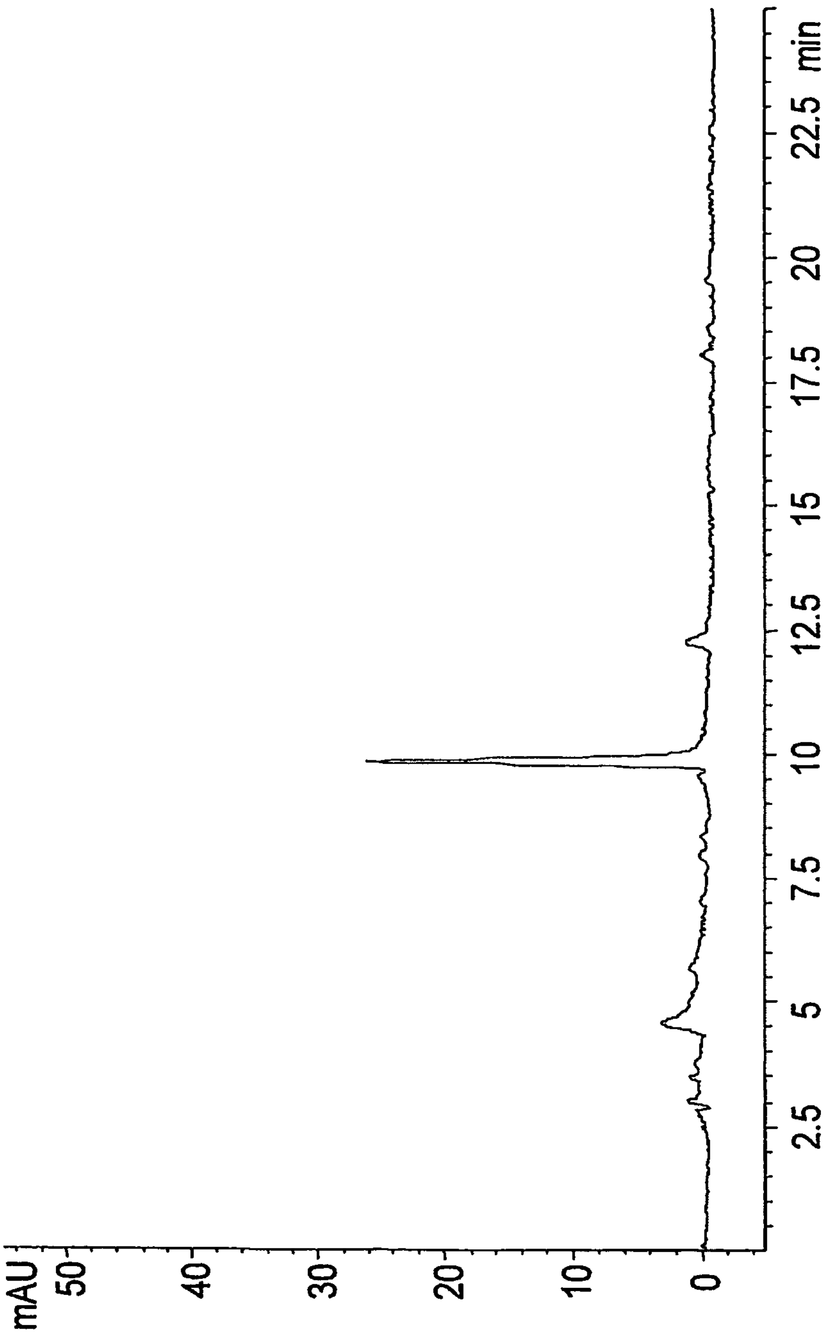


Figure 23C

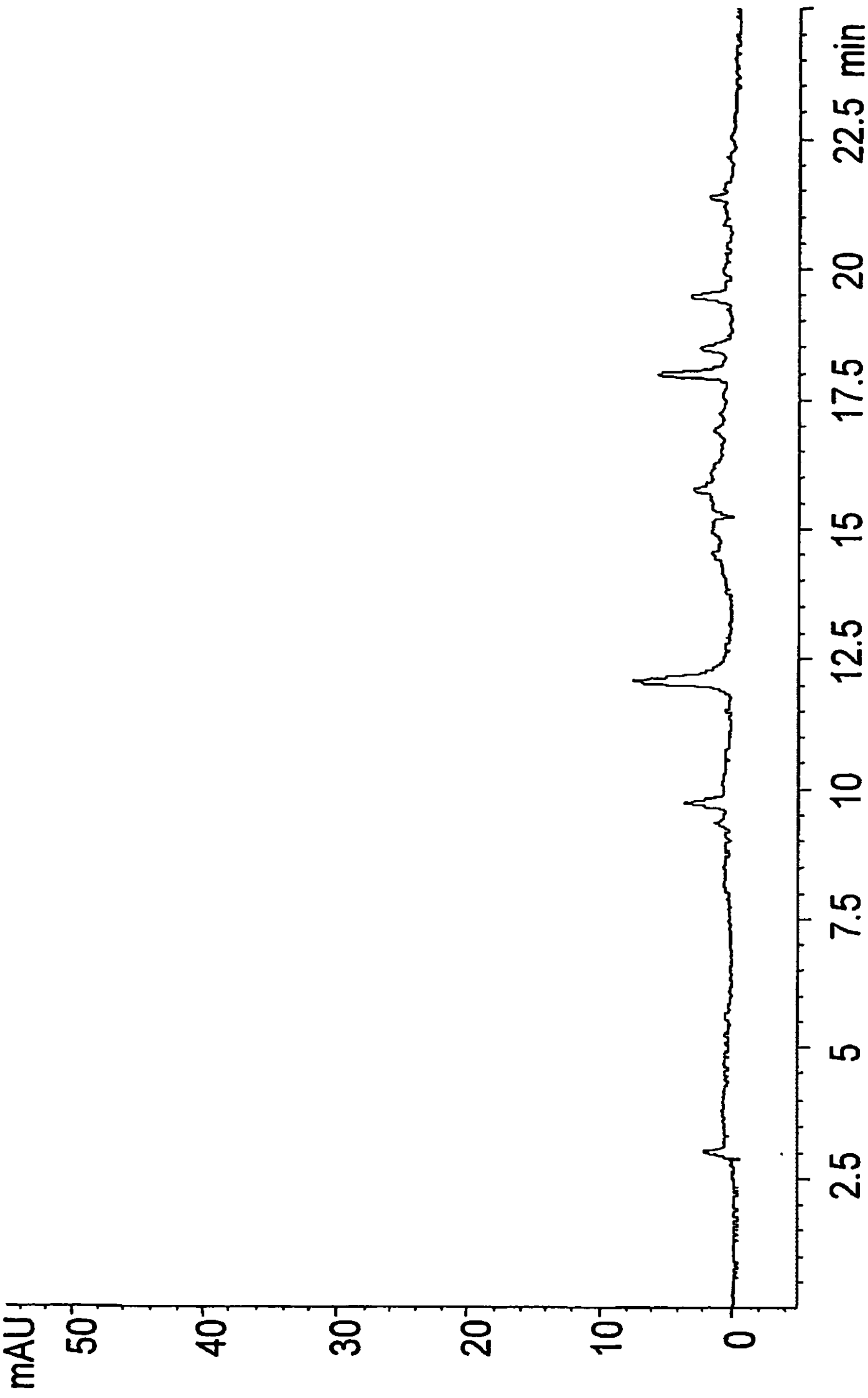


Figure 23D

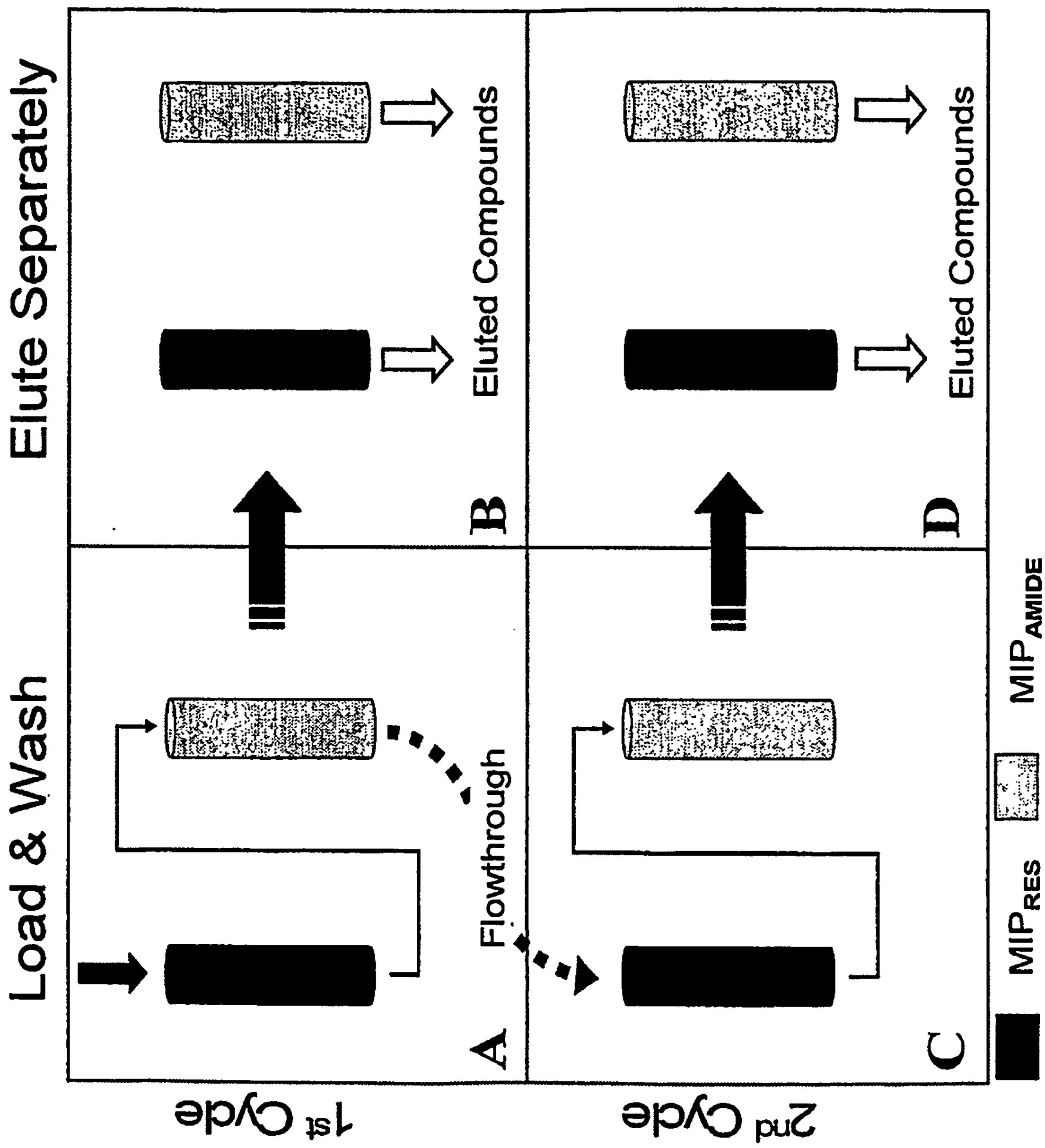
Load & Wash
Elute Separately
1st Cycle
2nd Cycle
A
B
C
D
Flowthrough
Eluted Compounds
Eluted Compounds
$MIP_{RES}$
$MIP_{AMIDE}$

Figure 24

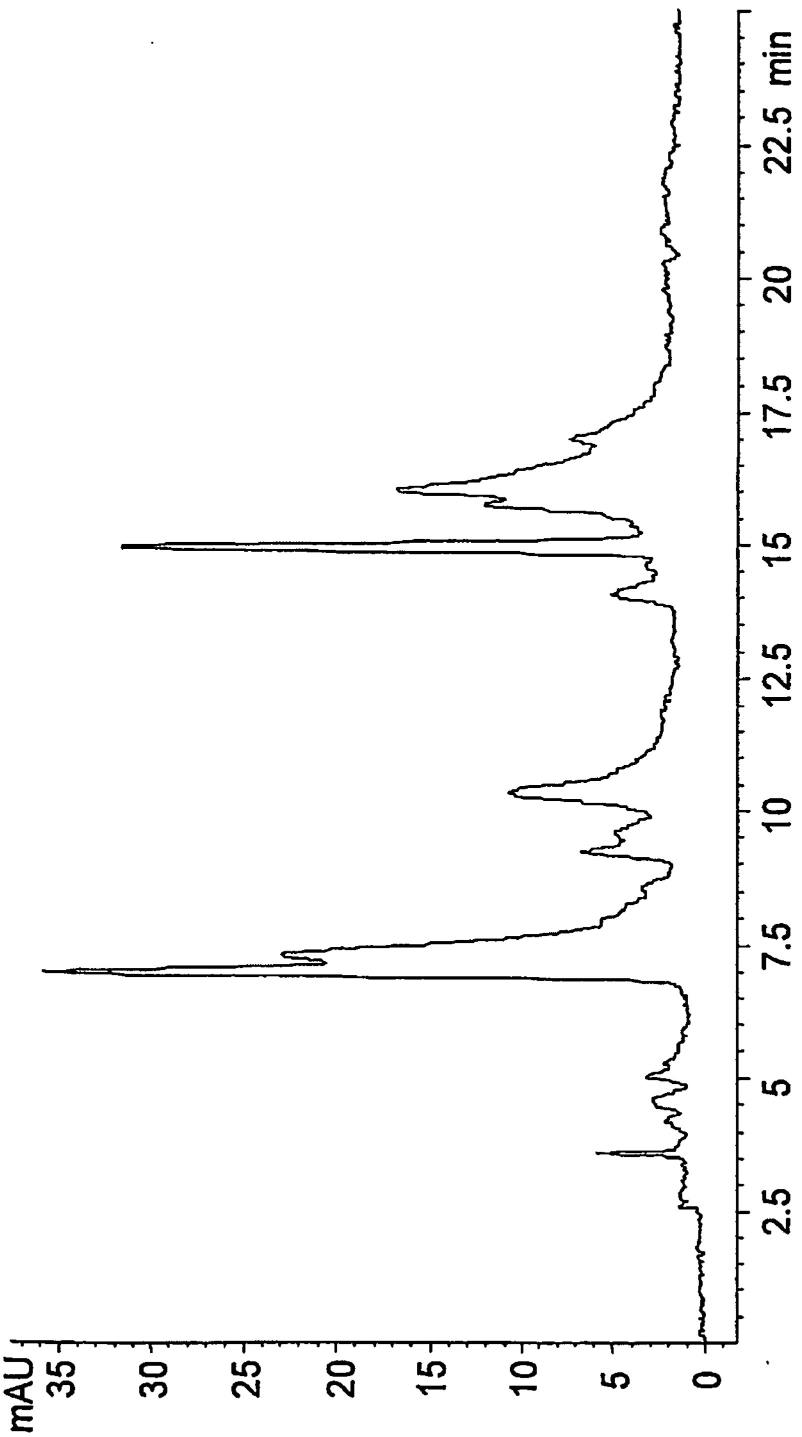


Figure 25A

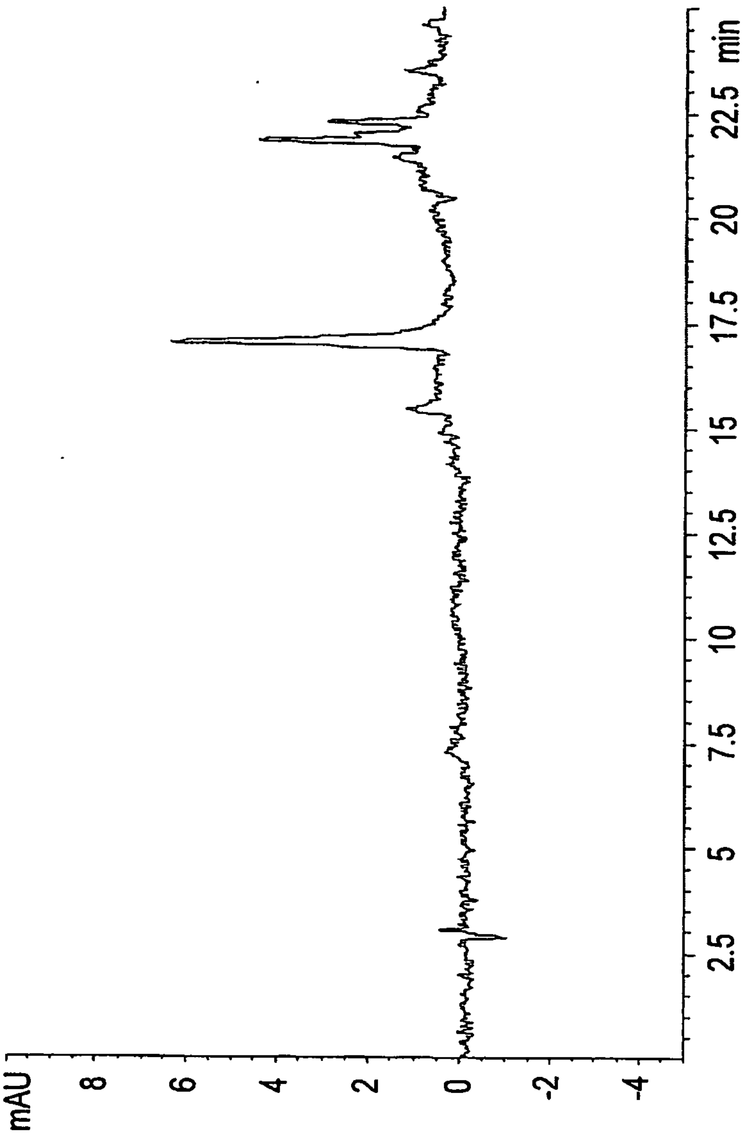


Figure 25B

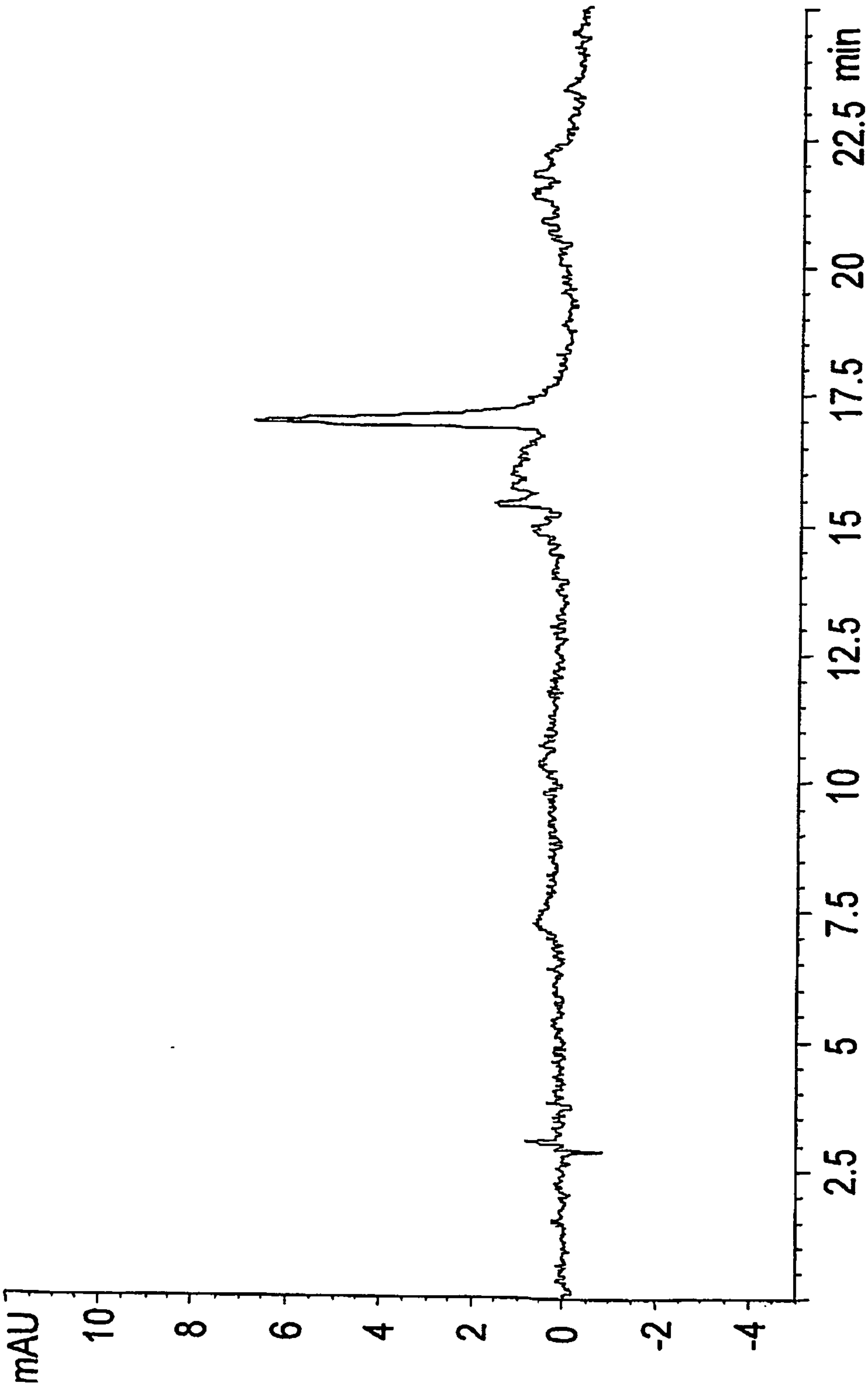


Figure 25C

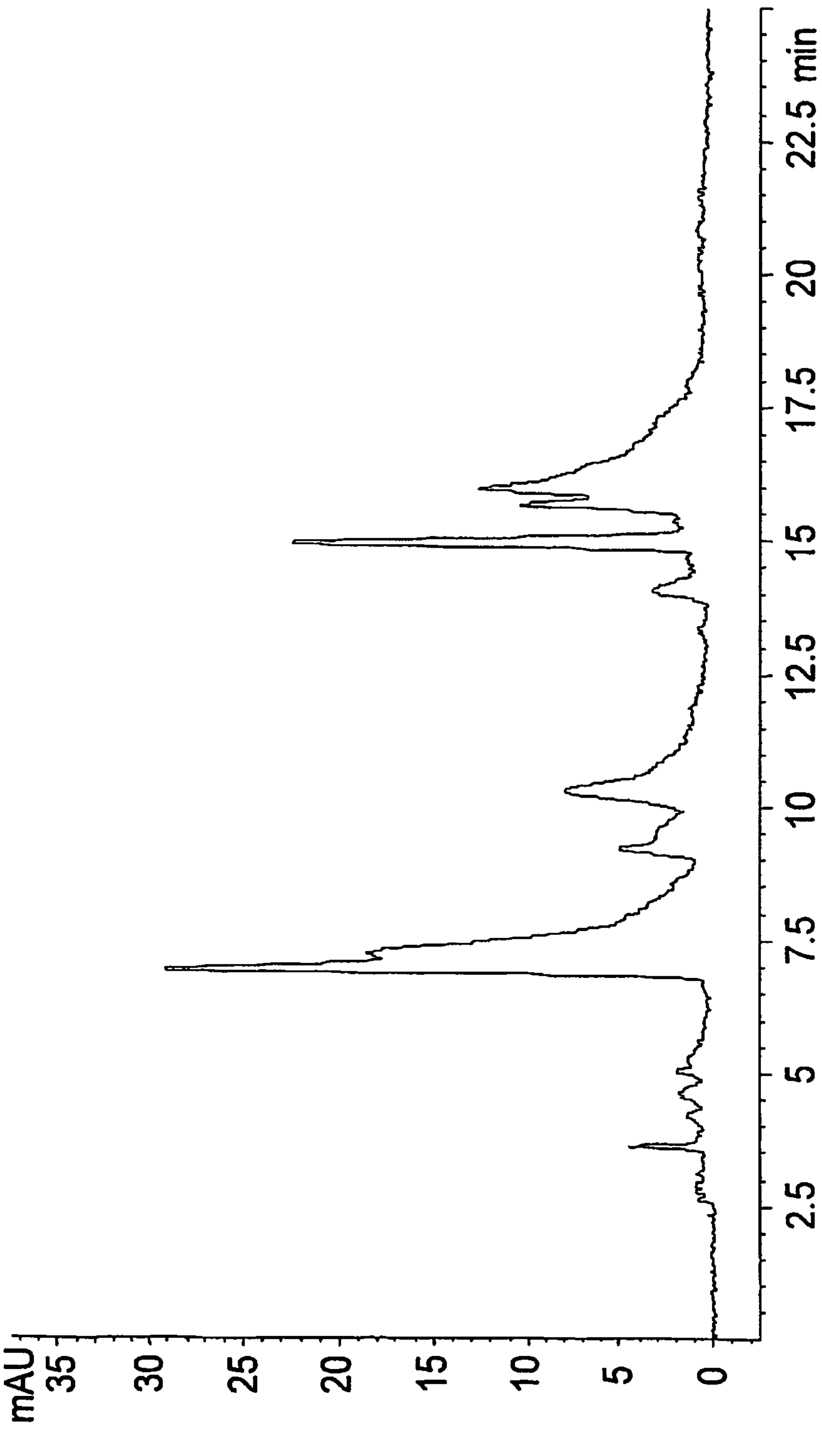


Figure 26A

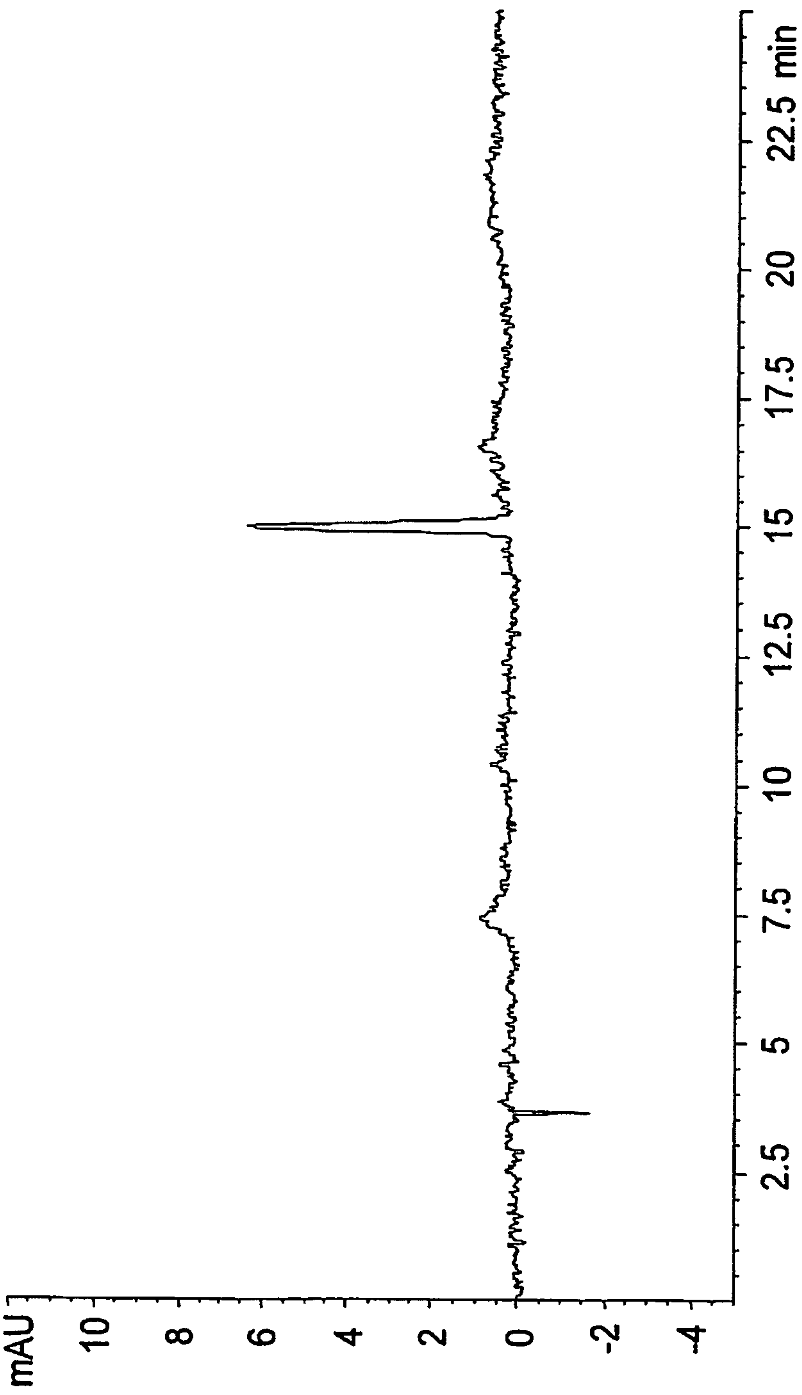


Figure 26B

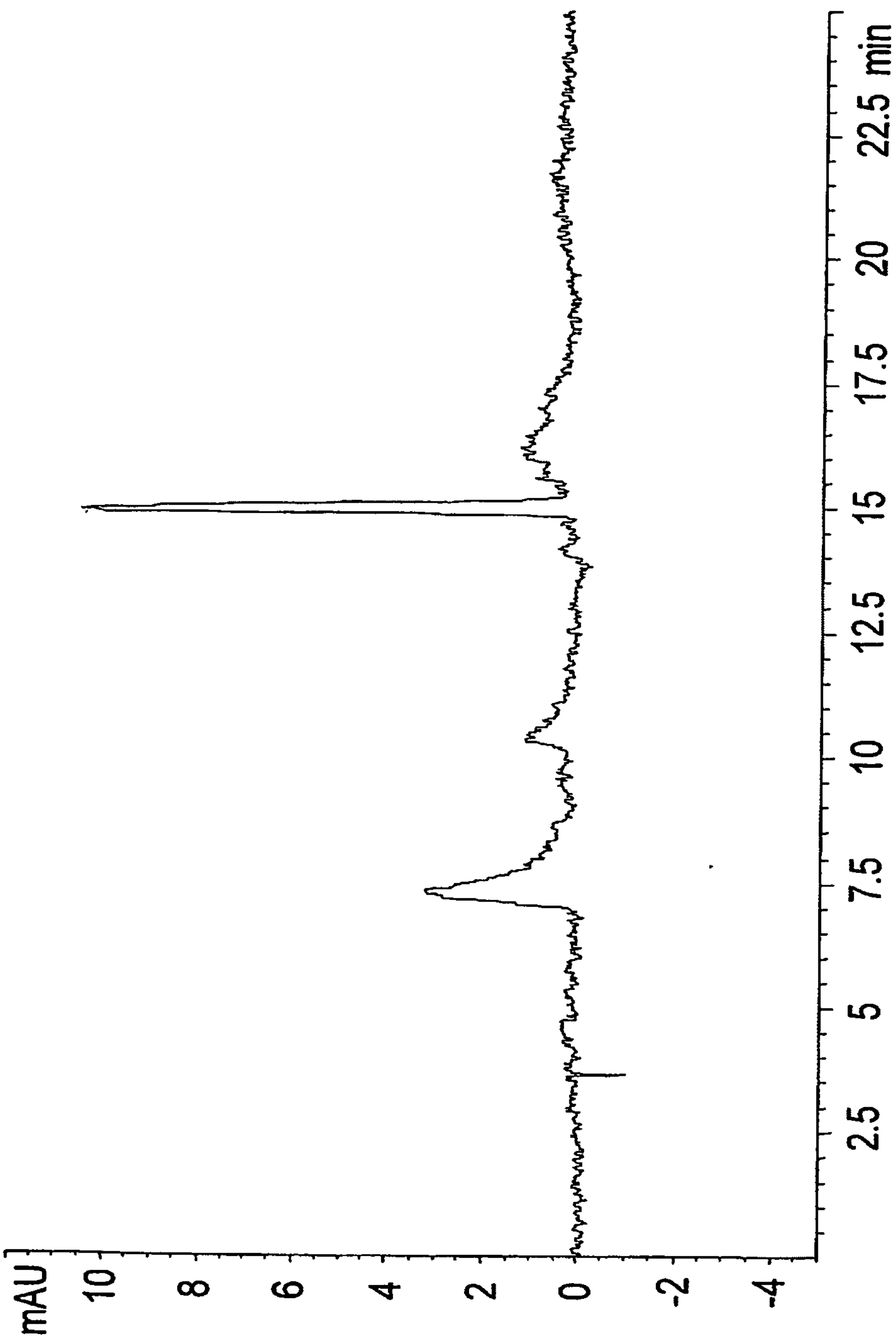


Figure 26C

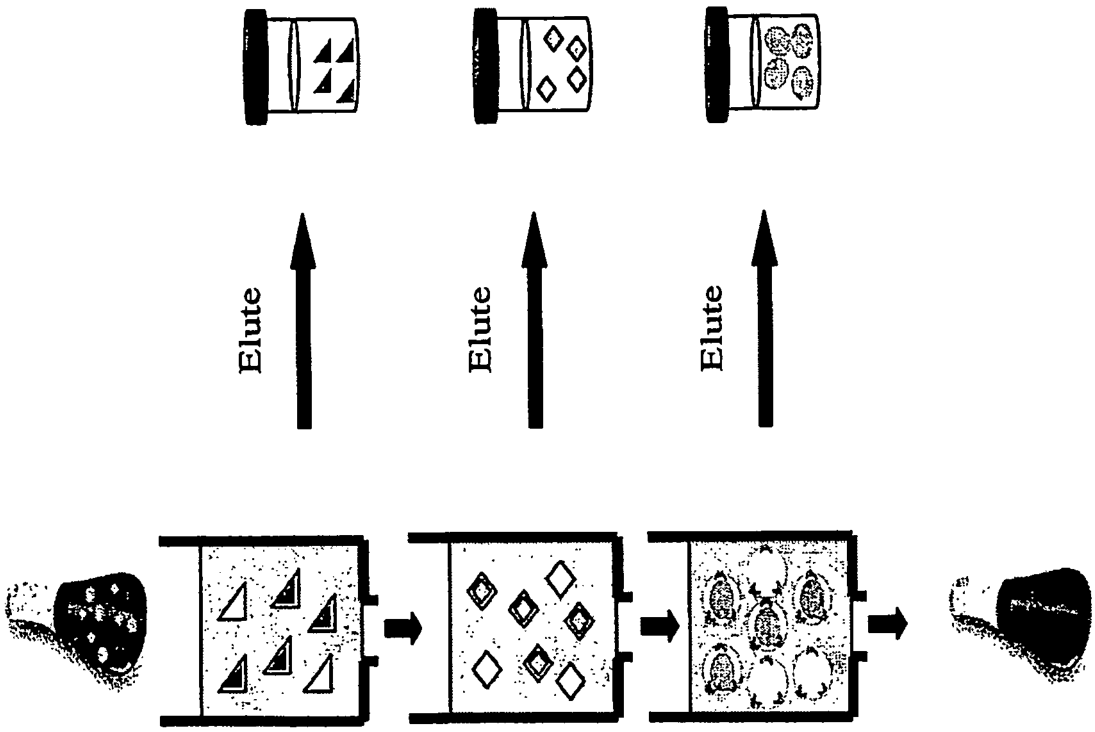


Figure 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2006108864 A2
- WO 2007021067 A1


### Non-patent literature cited in the description


- **BOTH MA ; ZHUANG et al.** *J. Chromatographic Sci.,* 2008, vol. 46 (8), 739-742
- **XIANG, H.-Y. ; ZHOU, C.-S. ; ZHONG, S.-A. ; LEI, Q.-F.** Synthesis of resveratrol imprinted polymer and its application in separation of active ingredient in Polygonum cuspidatum extracts. *Yingyong Huaxue,* 2005, vol. 22 (7), 739-743
- **MA, S. ; ZHUANG, X. ; WANG, H. ; LIU, H. ; LI, J. ; DONG, X.** Preparation and characterisation of trans-Resveratrol imprinted polymers. *Analytical Letters,* 2007, vol. 40, 321-333
- **CAO, H. ; XIAO, J. B. ; XU, M.** Evaluation of new selective molecularly imprinted polymers for the extraction of resveratrol from Polygonum cuspidatum. *Macromolecular Research,* 2006, vol. 14 (3), 324-330
- **KI, C. D. ; OH, C. ; OH, S.-G. ; CHANG, J. Y.** The use of a thermally reversible bond for molecular imprinting of silica spheres. *Journal of the American Chemical Society,* 2002, vol. 124 (50), 14838-14839
- **SPENCER, A.** Selective Preparation of Non-Symmetrically Substituted Divinylbenzenes by Palladium Catalysed Arylations of Alkenes with Bromobenzoic Acid Derivatives. *J. Organomet. Chem.,* 1984, vol. 265, 323-331
- **ROMERO-PERÉZ et al.** *J. Agric. Food Chem.,* 2001, vol. 49 (1), 210-215
- **D. A. LEARMONTH.** *Bioconjugate Chem.,* 2003, vol. 14, 262-267
- **ROBERTS et al.** *Eur. J. Med. Chem.,* 1994, vol. 29, 841-854
- **DOLLE, R.E. ; KRUSE, L.I.** *J. Org. Chem.,* 1985, vol. 51, 4047-4053
- **CONDO JR, A.M. ; BAKER, D.C. ; MOREAU, R.A. ; HICKS, K.B.** Improved Method for the Synthesis of trans-Feruloyl-β-sitostanol. *J. Agric. Food Chem,* 2001, vol. 49, 4961-4964